(19) Europäisches Patentamt
European Patent Office
Office européen des brevets

(11) **EP 1 896 124 B1**

(12) **EUROPÄISCHE PATENTSCHRIFT**

(45) Veröffentlichungstag und Bekanntmachung des Hinweises auf die Patenterteilung:
**16.08.2017   Patentblatt 2017/33**

(21) Anmeldenummer: **06743146.0**

(22) Anmeldetag: **09.06.2006**

(51) Int Cl.:
*A61N 1/36* *(2006.01)*          *G10L 15/02* *(2006.01)*
*A61B 5/04* *(2006.01)*          *A61B 5/12* *(2006.01)*
*A61B 5/00* *(2006.01)*          *A61N 1/08* *(2006.01)*
*G06F 19/00* *(2011.01)*          *G10L 15/24* *(2013.01)*
*G10L 15/00* *(2013.01)*

(86) Internationale Anmeldenummer:
**PCT/EP2006/005560**

(87) Internationale Veröffentlichungsnummer:
**WO 2007/000231 (04.01.2007 Gazette 2007/01)**

(54) **VORRICHTUNG, VERFAHREN UND COMPUTERPROGRAMM ZUR ANALYSE EINES AUDIOSIGNALS**

DEVICE, METHOD AND COMPUTER PROGRAM FOR ANALYSING AN AUDIO SIGNAL

PROCEDE, DISPOSITIF ET PROGRAMME INFORMATIQUE POUR ANALYSER UN SIGNAL AUDIO

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **29.06.2005   DE 102005030327**
**29.06.2005   US 172605**

(43) Veröffentlichungstag der Anmeldung:
**12.03.2008   Patentblatt 2008/11**

(73) Patentinhaber: **Fraunhofer-Gesellschaft zur Förderung der angewandten Forschung e.V.**
**80686 München (DE)**

(72) Erfinder: **KLEFENZ, Frank**
**68159 Mannheim (DE)**

(74) Vertreter: **Zimmermann, Tankred Klaus et al**
**Schoppe, Zimmermann, Stöckeler Zinkler, Schenk & Partner mbB Patentanwälte**
**Radlkoferstrasse 2**
**81373 München (DE)**

(56) Entgegenhaltungen:
**WO-A-01/99470          WO-A-02/084539**
**US-A- 4 536 844          US-A- 4 980 918**
**US-A- 5 381 512          US-A- 5 388 182**
**US-A- 6 064 913**

**Beschreibung**

Die vorliegende Erfindung bezieht sich im Allgemeinen auf eine Vorrichtung, ein Verfahren und ein Computerprogramm zur Analyse eines Audiosignals, um eine Analysedarstellung des Audiosignals zu erhalten, im Speziellen auf eine Vorrichtung, ein Verfahren und ein Computerprogramm zur Detektierung von Wanderwellen in der Cochlea unter Verwendung einer Parallelen Hough-Transformation.

Analyse und Modellierung des menschlichen Gehörsystems bilden schon seit geraumer Zeit einen Schwerpunkt sowohl bei der Erkennung und Klassifizierung von Audiosignalen als auch in der Medizintechnik. Dabei wurde insbesondere der Aufbau des menschlichen Ohrs bereits seit geraumer Zeit studiert. Um ein Verständnis der vorliegenden Erfindung zu erleichtern, werden im Folgenden einige wesentliche Erkenntnisse zu den Grundlagen der Hörwahrnehmung dargestellt.

Physiologie: Auditorische Peripherie und zentrales Gehör

Die physiologischen Gegebenheiten der menschlichen auditorischen Peripherie sind mittlerweile gut erforscht und können in einer Vielzahl wissenschaftlicher Abhandlungen nachgeschlagen werden. Daher sollen an dieser Stelle nur die wesentlichen und zum weiteren Verständnis späterer Ausführungen notwendigen Grundlagen dargestellt werden.

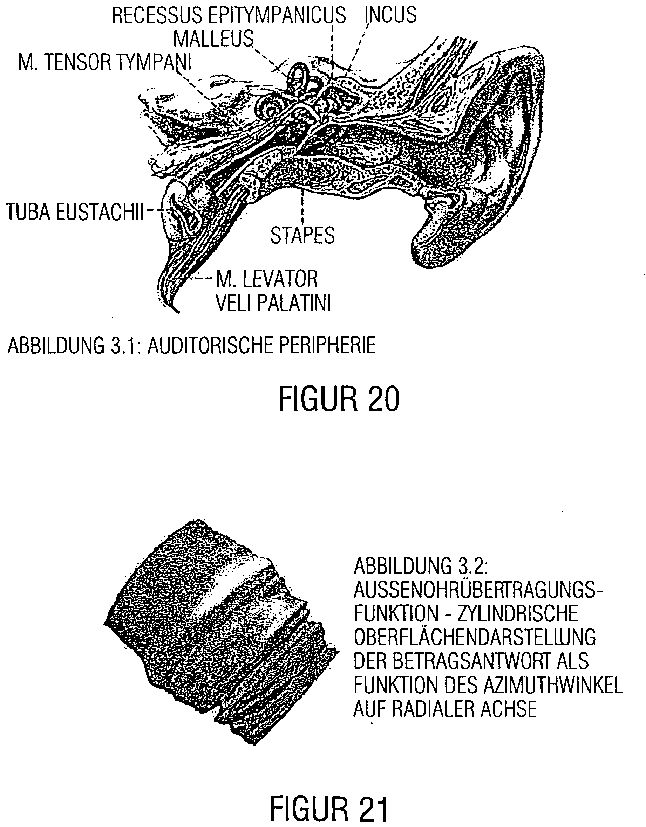

Der periphere Schallverarbeitungsapparat des Menschen (siehe Fig. 20 besteht aus der Gesamtheit von Außen-, Mittel- und Innenohr. Durch den äußeren Gehörgang gelangt Schall zum Trommelfell und wird im Mittelohr über die Gehörknöchelchen weitergeleitet. Anschließende Verarbeitung im Innenohr bewirkt eine frequenzabhängige Transduktion der mechanischen Schwingungen in neuronale Nervenaktionspotentiale und Weitergabe dieser an die angeschlossenen Hörnervenfasern.

Außenohr:

Das äußere Ohr bildet einen Trichter, der die einfallenden Schallwellen zum Trommelfell leitet. Ohrmuschel, Gehörgang, Schädelform und Schulter modifizieren das Schallsignal.

Da der Gehörgang (inkl. Ohrmuschel) an einem Ende geöffnet und am anderen geschlossen ist, wird er physikalisch näherungsweise als halboffenes Rohr aufgefasst. Somit kann im Resonanzfall, d.h. wenn ein Viertel der Schallwellenlänge der effektiven Gehörkanallänge entspricht, ein Schalldruckpegelgewinn beobachtet werden. Im Resonanzmaximum bei ungefähr 2500 Hz beträgt die Verstärkung bis zu 20 dB. Eine zweite Resonanz ("Cavum-Conchae-Resonanz") wird zwischen 2000 Hz und 2500 Hz allein durch die Ohrmuschel hervorgerufen.

In Abhängigkeit von der Schalleinfallsrichtung werden als Resultat der Außenohrform durch sogenannte "richtungsbestimmende Bänder" einzelne schmale Frequenzbereiche angehoben bzw. abgesenkt. Dadurch wird bis zu einem gewissen Maß die Lokalisation eintreffenden Schalls auch ohne binaurale Zeit- und Intensitätsunterschiede insbesondere in der vertikalen Ebene (Median-Sagittal-Ebene) möglich.

Zusammenfassen kann man die beschriebenen Phänomene in der Außenohr-Übertragungsfunktion ("Head related transfer function" HRTF), die in Fig. 21 dargestellt ist.

Mittelohr:

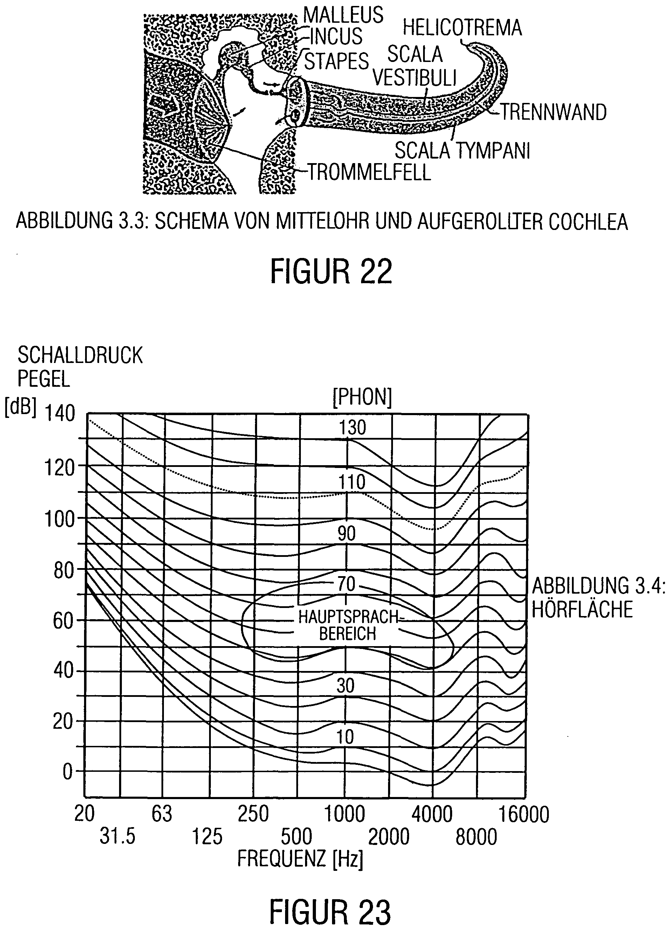

Die wesentliche Aufgabe des Mittelohres (MO) besteht in der Anpassung der Schallkennimpedanzen von Luft und den Flüssigkeiten im Innenohr. Bei Fehlen einer solchen Funktionalität würden wie im Fall der Schallleitungsschwerhörigkeit bis zu 98 % der einfallenden Schallenergie reflektiert. Bei gesundem Mittelohr können aber ungefähr 60 % der Signalintensität an das Innenohr weitergegeben werden. Die hierfür notwendige Schalldruckverstärkung wird möglich durch die aneinandergereihte Kopplung von Trommelfell, den drei Gehörknöchelchen (Hammer, Amboss und Steigbügel) sowie dem ovalen Fenster als Kontaktstelle zum Innenohr (siehe Fig. 22).

Drei unterschiedliche Mechanismen sind verantwortlich für diese Impedanztransformation:

1. Flächenverhältnis zwischen Trommelfell AT und Steigbügelfußplatte AS:

$$\frac{A_T}{A_S} \cong 17$$

2. Verhältnis der Hebelarme von Hammer $l_H$ und Amboss $l_A$:

$$\frac{l_H}{l_A} \cong 1,3$$

3. Hebelarm durch die Biegung des Trommelfells und die unsymmetrische Aufhängung des Hammers:

$$F_T \cong 1,4$$

Die Gesamtverstärkung errechnet sich zu:

$$\frac{p_{ges}}{p_T} = F_T \frac{A_T}{A_S} \frac{l_H}{l_A} \cong 30dB$$

(pT : Schalldruck am Trommelfell).

Bemerkenswert ist die Bedeutung der Transferfunktion des MO, die sich wie ein Bandpassfilter mit breitem Durchlassbereich verhält. Im Niederfrequenzbereich wird sie begrenzt durch die mechanischen Eigenschaften von Trommelfell und ovalem Fenster. Bei hohen Frequenzen limitieren die Trägheitsmomente und Reibungs- bzw. Biegungsverluste der Gehörknöchelchen die Übertragung. Vergleicht man den Verlauf der MO-Übertragungsfunktion mit dem der Hörschwelle (siehe Fig. 23), so sieht man, dass die Hörempfindlichkeitskurve weitestgehend durch die mechanischen Eigenschaften von mittlerem und äußerem Ohr bestimmt wird.

Eine zusätzliche Aufgabe erfüllen die Muskeln des MO (M. tensor tympanus und M. stapedius, siehe Fig. 20). Durch reflektorische Kontraktion kann die MO-Steifigkeit erhöht und so eine Dämpfung tiefer Frequenzen erreicht werden. Begrenzter Schutz gegenüber hohen Pegeln und Verringerung der Wahrnehmung selbstproduzierter Laute sind die Folge.

Innenohr:

Die Struktur des Innenohres setzt sich aus zwei Einheiten zusammen. Während das Vestibularorgan einen Bestandteil des Gleichgewichtssystems darstellt, bildet der Aufbau der Cochlea den abschließenden Teil der auditorischen Peripherie (siehe Fig. 22). Anatomisch gleicht die Cochlea einem Schneckenhaus mit zweieinhalb Windungen. Sie ist durch die cochleäre Trennwand (CT) in die zwei Perilymphflüssigkeit enthaltenden Kammern "Scala vestibuli" (SV) und "Scala tympani" (ST) geteilt (siehe Fig. 22).

Die Arbeitsweise der Cochlea lässt sich wiederum in zwei Abschnitten beschreiben. Der hydromechanische Teil wird bestimmt durch die makro- und mikromechanischen Eigenschaften des Schneckeninneren. Die eigentliche Funktionseinheit zur Umwandlung der Eingangssignale in neuronale Repräsentationen befindet sich innerhalb der cochleären Trennwand. Die Scala Vestibuli ist mit dem Mittelohr verbunden über das ovale Fenster (OF). Dieses schwingt mit der Steigbügelbewegung und zwingt so die inkompressible Lymphflüssigkeit zum Ausweichen. Die Ausweichbewegung wird an die cochleäre Trennwand weitergegeben und bildet eine Wanderwelle in Richtung des Helicotrema (HC), Cochlea-Spitze, aus. Aufgrund der längs ihrer Ausdehnung kontinuierlich veränderlichen mechanischen Eigenschaften (Massenbelag, Steifigkeit, Breite, etc.) bildet die Trennwand an bestimmten Stellen frequenzabhängige Resonanzen aus. Diese tonotopische Frequenzselektivität wird auch als Ortstheorie bezeichnet.

Den charakteristischen Frequenzen können auf der Trennwand Orte der maximalen Wellenamplituden zugeordnet werden, die kontinuierlich von hohen Frequenzen im Bereich des ovalen Fensters (Basis der Basilarmembran) bis zu tiefen Frequenzen am Helicotrema reichen (Ende bzw. Apex der Basilarmembran). Über diese Dispersionseigenschaft können Frequenzinhalte im einkommenden Audiosignal bis zu einem gewissen Grad aufgespalten werden.

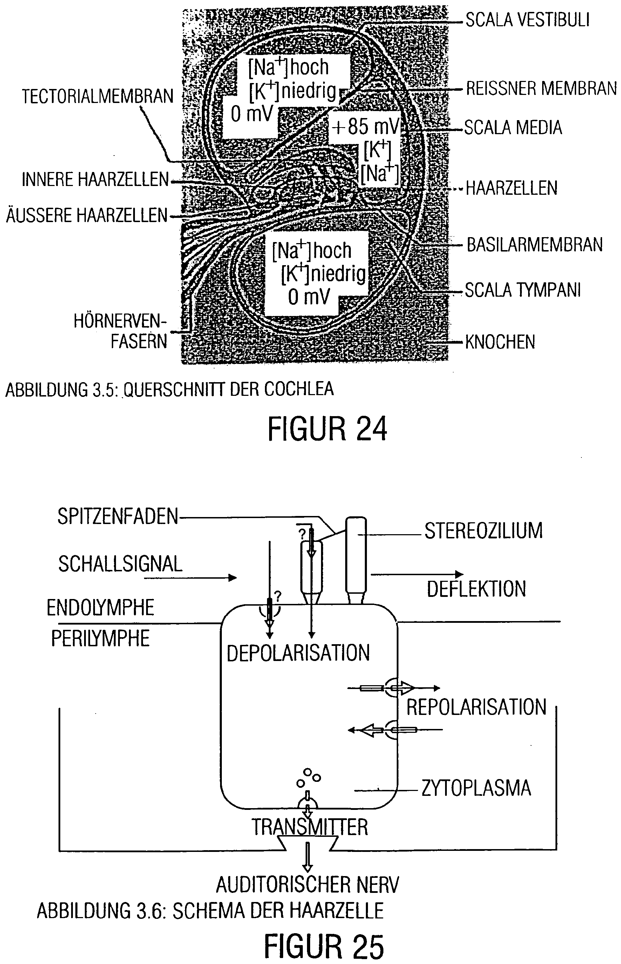

Unterstützt wird diese Funktionalität durch die Eigenschaften der cochleären Trennwand (s. Fig. 24). Diese wird zur Scala Vestibuli hin abgeschlossen durch die Reissnersche Membran (RM). Die Grenzfläche zur Scala Tympani besteht aus der Basilarmembran (BM) mit aufsitzendem Cortischen Organ (CO), auf dessen Oberseite sich in Längsrichtung drei Reihen äußerer Haarzellen und eine Reihe innerer Haarzellen befinden. Diese Haarzellen werden wiederum überspannt von der Tektorialmembran (TM).

Im Bereich dazwischen befindet sich die Endolymphflüssigkeit der Scala Media. Bei Bewegung der cochleären Trennwand geraten die Tektorialmembran und das Cortische Organ in Relativbewegung, was zu einer Auslenkung der auf den Haarzellen befindlichen Sinneshärchen führt. Dies geschieht teils durch direkten Kontakt, teils aber auch durch hydrodynamische Kopplung. Die äußeren Haarzellen besitzen nun die Fähigkeit, sich in Abhängigkeit der Trennwandschwingung sehr schnell zu verkürzen bzw. zu verlängern. Dies führt zu einer bis zu 1000-fachen Verstärkung der Wanderwellenamplituden und liefert spitze und ausgeprägte Schwingungsmaxima.

Ebenso wie die Sinneshärchen der äußeren Haarzellen werden diejenigen der inneren Haarzellen durch die Relativbewegung von Tektorialmembran und Cortischem Organ ausgelenkt. Die nachgemessene 3-dimensionale Bewegung der Cochlea ist kompliziert und wurde beispielsweise von Zenner und Gummert an der Universität Ulm bestimmt. In Folge dieser Bewegung werden biochemische Prozesse in Gang gesetzt, die eine Transduktion der mechanischen Bewegungen in neuronale Aktionspotentiale bewirken (siehe Fig. 25).

Im Ruhezustand besitzen die inneren Haarzellen ein Ruhemembranpotential von ungefähr -40 mV sowie eine niedrige Kalium-Konzentration. Die umgebende Flüssigkeit der Scala Media weist hingegen einen ungewöhnlich hohen Anteil an Kaliumionen auf und ist positiv geladen. Bei Deflektion der Sinneshärchen in eine Richtung öffnen sich sogenannte Transduktionsionenkanäle, durch die zwecks Potentialausgleich ein Einstrom positiv geladener Kaliumionen in die Haarzelle erfolgt. Auslenkung der Sinneshärchen in die entgegengesetzte Richtung verschließt diese Kanäle und durch Ionenverbindungen in die basolaterale Zellmembran kann das ursprüngliche Potential wiederhergestellt werden. Bei geöffneten Kanälen bewirkt das geänderte Sensorpotential eine vermehrte Freisetzung von afferenter Transmittersubstanz.

Diese diffundiert durch den synaptischen Spalt in Richtung Hörnerv. In Abhängigkeit von der Transmitterkonzentration im synaptischen Spalt steigt die Wahrscheinlichkeit der Auslösung eines Nervenaktionspotentials (NAP).

Bis zu einer Frequenz von knapp 5000 Hz folgt die Freisetzung von Transmittersubstanz hochgradig synchron der Deflektion der Sinneshärchen. Somit kann eine lineare Frequenzübertragung über zeitliche Kodierung erfolgen, was in der Literatur auch unter dem Begriff "Phase-Locking" zusammengefasst wird.

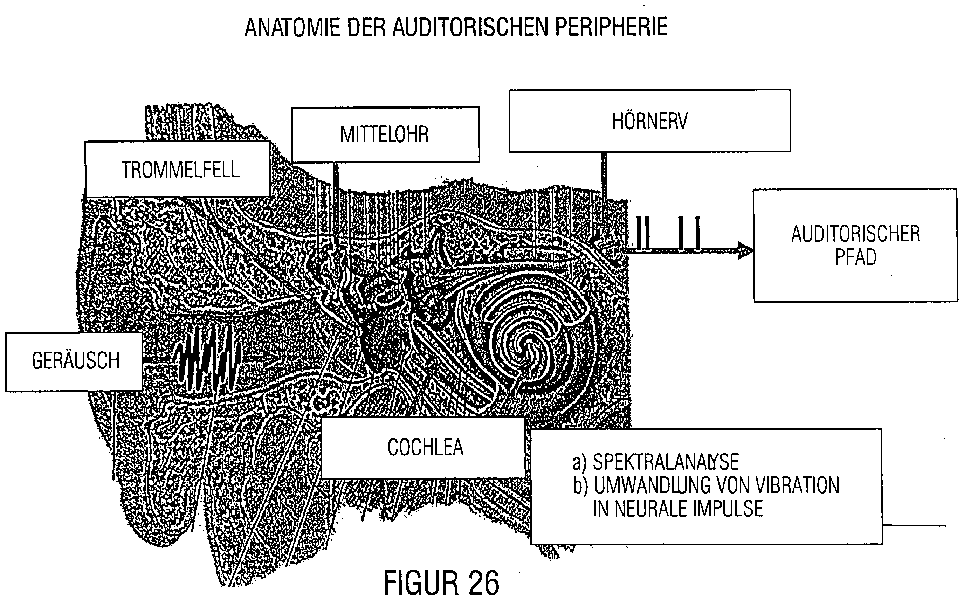

Weiterhin wird auch auf die Fig. 26 verwiesen, die noch einmal die Anatomie der auditorischen Peripherie zeigt. Die Fig. 26 zeigt hierbei die Umwandlung bzw. Weiterleitung eines Geräuschs über Trommelfell und Mittelohr zur Schnecke (Cochlea). Die Cochlea ermöglicht dabei eine Spektralanalyse des eintreffenden Geräuschs sowie eine Umwandlung von Vibrationen in neurale Impulse. Die Cochlea weist ferner Nervenzellen auf, die Nervenimpulse (Aktionspotenziale) erzeugen, welche über den Hörnerv an das Gehirn weitergeleitet werden.

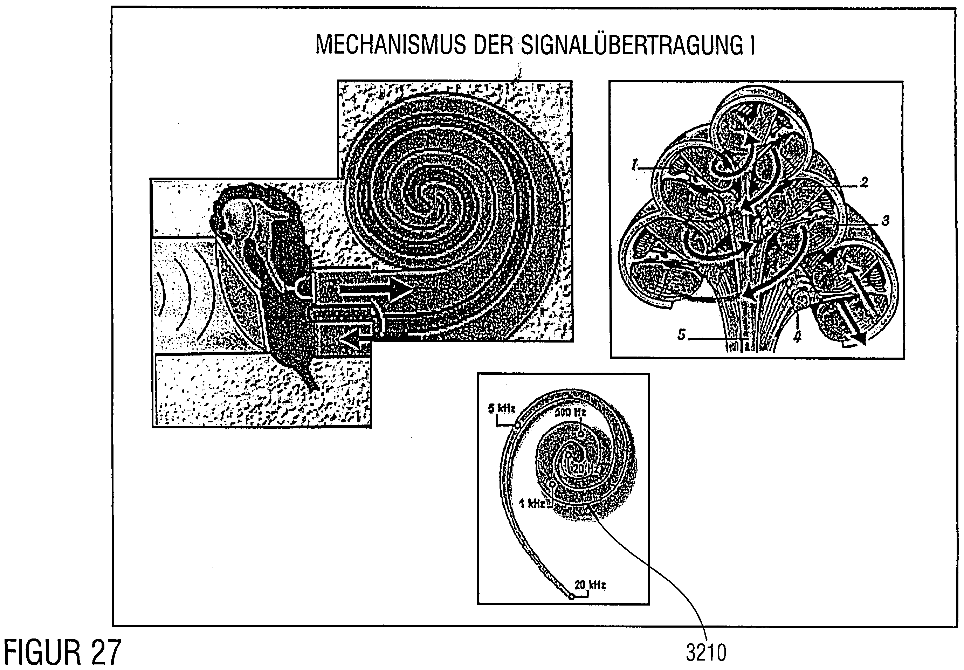

Fig. 27 zeigt noch einmal in schematischer Form den Mechanismus der Signalübertragung im menschlichen Ohr. Aus der Fig. 27 ist ersichtlich, dass die Cochlea 3210 unterschiedliche Frequenzen an unterschiedlichen Orten wahrnimmt (Ortstheorie). Es werden beispielsweise hohe Frequenzen (z.B. mit einer Frequenz von 20 KHz) an dem Anfang der Cochlea in Nervensignale umgewandelt, während niedrige Frequenzen (z.B. mit einer Frequenz von 20 Hz) an dem Ende der Cochlea in Nervensignale umgewandelt werden. Dadurch kann in der Chochlea gleichsam eine Spektralanalyse eines Geräuschs bzw. eines Audiosignals erfolgen, wobei für eine vorgegebene Frequenz jeweils die Nervenzellen am stärksten angeregt werden, die für eine Wahrnehmung der jeweiligen Frequenz optimal ausgelegt sind.

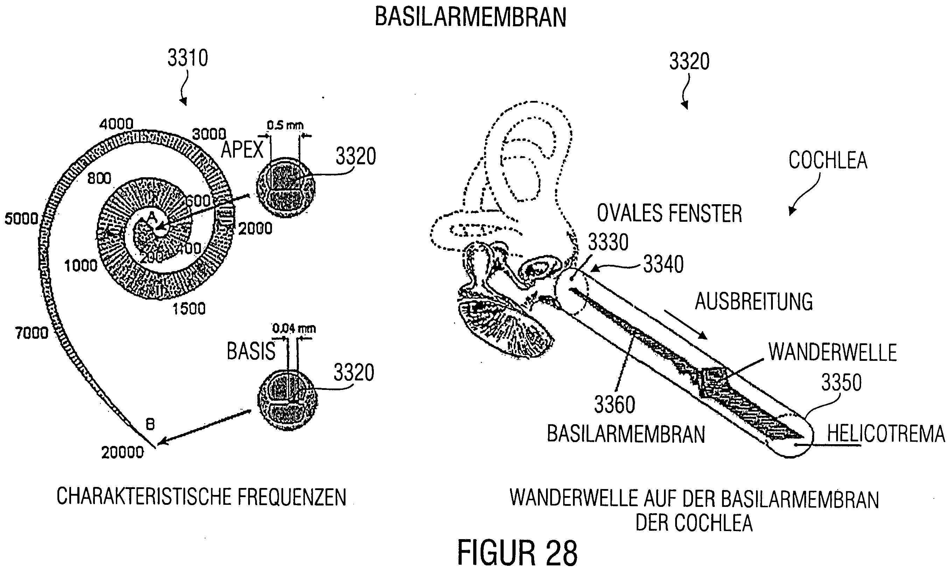

Die Fig. 28 zeigt den Aufbau der Schnecke, wobei auch auf die Geometrie der Basilarmembran eingegangen wird. Eine graphische Darstellung 3310 zeigt hierbei, dass die Breite der Basilarmembran 3320 von der Basis der Cochlea zu dem Ende (Apex) der Cochlea hin um den Faktor 10 zunimmt.

Eine graphische Darstellung 3320 zeigt ferner eine Einkopplung einer akustischen Welle in die Cochlea über ein ovales Fenster 3330. Die Einkopplung über das ovale Fenster 3330 erzeugt eine Wanderwelle in der Cochlea, die von der Basis 3340 der Cochlea zu dem Apex 3350 der Cochlea läuft und dabei die Basilarmembran 3360 der Cochlea auslenkt. Es ist hierbei zu beachten, dass Nervenzellen, die näher bei der Basis 3340 der Cochlea gelegen sind, früher angeregt werden als Nervenzellen, die weiter von der Basis 3340 der Cochlea entfernt sind. Mit anderen Worten, der Ort der Wanderwelle als Funktion der Zeit kann als Trajektorie der Wanderwelle angesehen werden. Die Trajektorie kann freilich auch auf diskrete Nervenzellen abgebildet werden, so dass eine Trajektorie ebenso beschreibt, in welcher zeitlichen Folge mehrere räumlich getrennte Nervenzellen durch eine Wanderwelle angeregt werden.

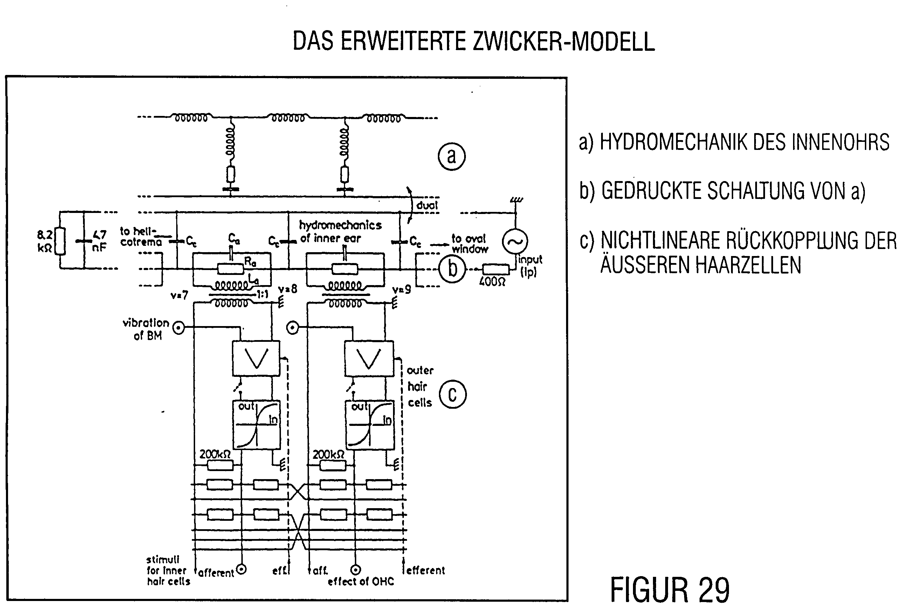

Fig. 29 zeigt ein beispielhaftes elektrisches Ersatzmodell, mit dessen Hilfe die Ausbreitung von Schallwellen durch die Cochlea bis zur Anregung der inneren Haarzellen modelliert werden kann. Das gezeigte Modell ist als "erweitertes Zwicker-Modell" bekannt. Das Modell beschreibt beispielsweise die Hydromechanik des Innenohrs und die nichtlineare Rückkopplung der äußeren Haarzellen. Es wird allerdings darauf hingewiesen, dass das gezeigte Modell nur eines von vielen möglichen Modellen zur Berechnung der Anregung der inneren Haarzellen ist.

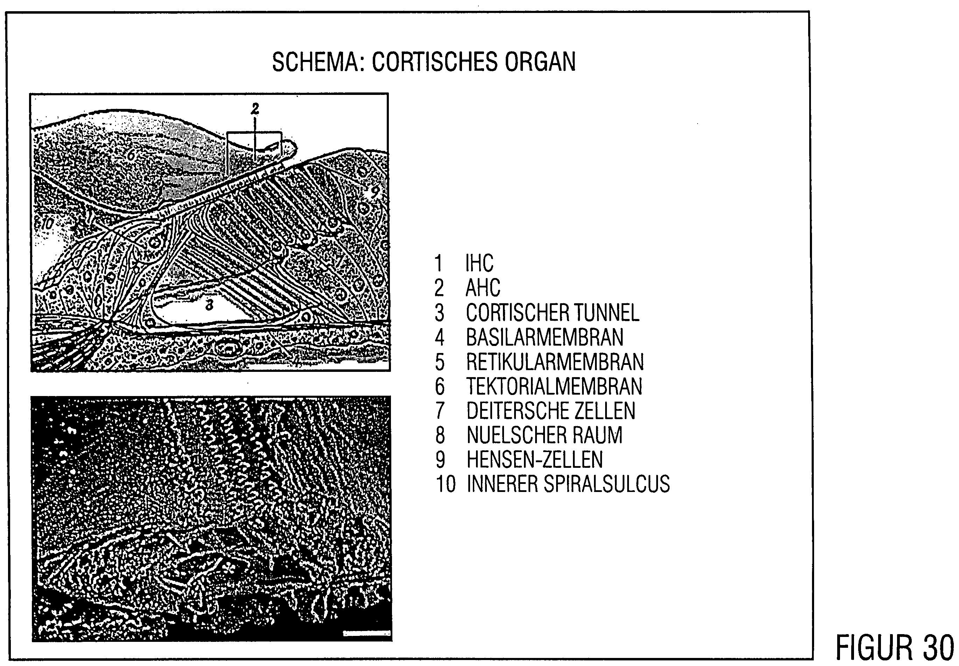

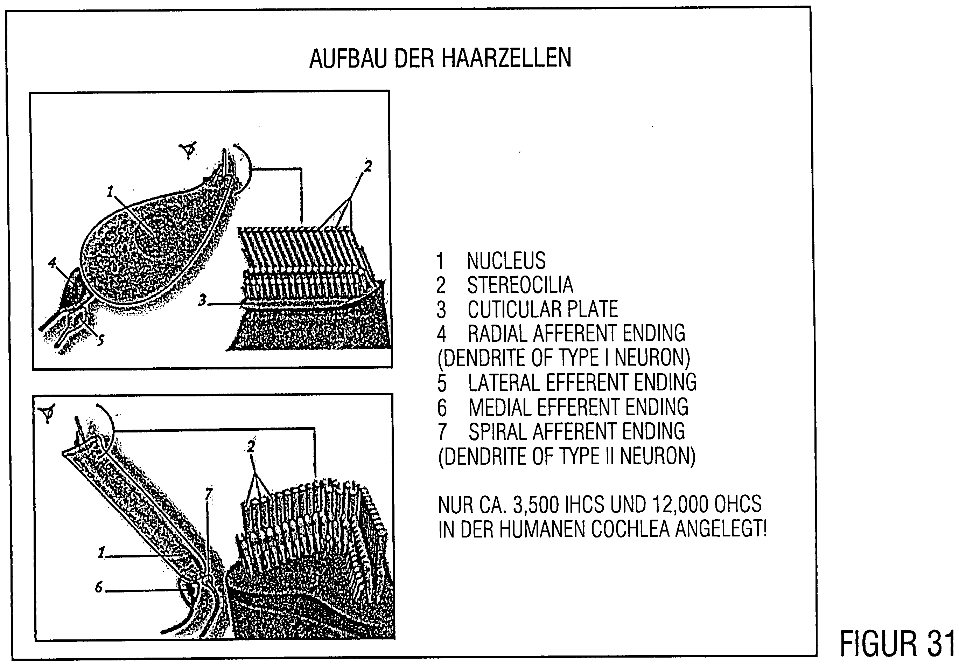

Fig. 30 beschreibt in einer schematischen Darstellung das Cortische Organ, und Fig. 31 beschreibt den Aufbau von zwei verschiedenen Sorten von Haarzellen.

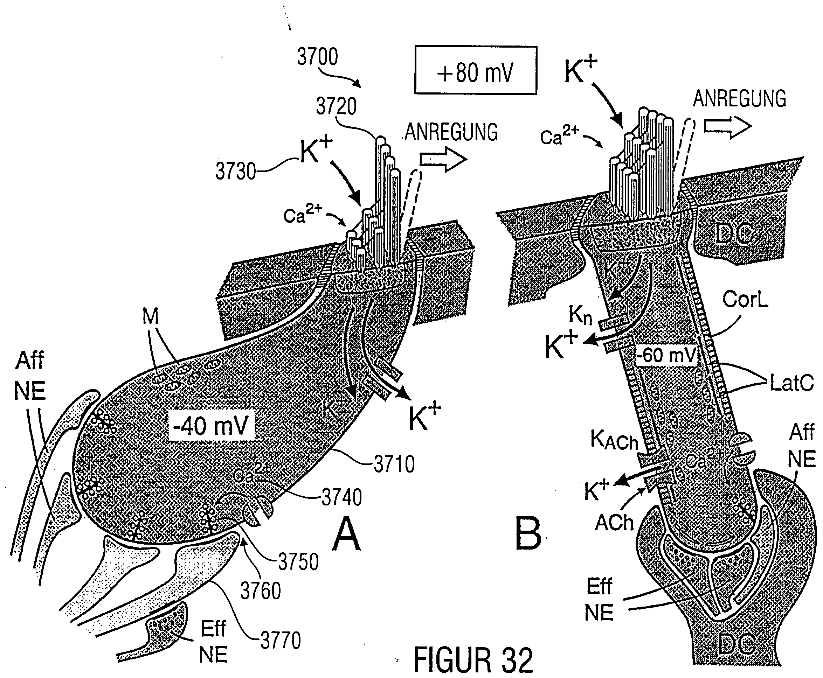

Fig. 32 zeigt eine detaillierte schematische Darstellung von zwei Haarzellen. Die schematische Darstellung der Fig. 32 ist in der Gesamtheit mit 3700 bezeichnet. Anhand der graphischen Darstellung 3700 werden hier zur Verbesserung des Verständnisses die chemischen Abläufe in einer inneren Haarzelle kurz skizziert.

Die Haarzelle 3710 weist eine Mehrzahl von Stereozilien 3720 auf, die die Form von feinen Härchen haben. Eine Anregung bzw. Auslenkung der Stereozilien bewirkt, dass sich die Durchlässigkeit bzw. Leitfähigkeit einer Zellmembran verändert, so dass positiv geladene Kalium-Ionen 3730 in die Haarzelle eintreten können. Dadurch verändert sich das intrazelluläre Potenzial der Haarzellen, das oft mit V(t) bezeichnet wird. In Abhängigkeit von dem intrazellulären Haarzellenpotenzial V(t) können positiv geladene Kalzium-Ionen 3740 in die Zelle eindringen, so dass sich damit die Konzentration an Kalzium-Ionen 3740 erhöht. Die Kalzium-Ionen wirken dann auf die Freigabe von Neurotransmitter-Molekülen 3750 in einen synaptischen Spalt 3760 zwischen der Haarzelle 3710 und einer Nervenfaser 3770. Die Freigabe der Neurotransmitter-Moleküle 3750 erfolgt typischerweise gequantelt in Vesikeln von mehreren tausend Molekülen.

Die Konzentration an Neurotransmittern in dem synaptischen Spalt 3760 verändert dann das Potenzial in dem synaptischen Spalt 3760. Übersteigt das Potenzial in dem synaptischen Spalt 3760 einen bestimmten Schwellwert, so wird schließlich ein Aktionspotenzial in der Nervenfaser 3770 erzeugt.

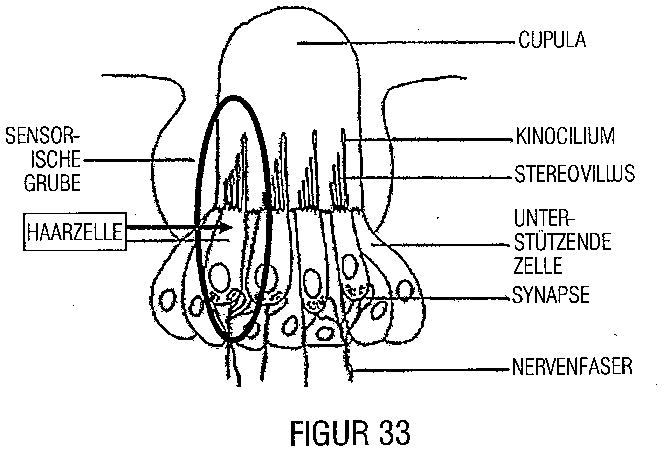

Fig. 33 zeigt schließlich zur Verdeutlichung die Anordnung einer Mehrzahl von Haarzellen in einer sensorischen Grube (sensory pit) einer menschlichen Cochlea. Aus der Darstellung der Fig. 33 geht hervor, dass eine einzige Haarzelle typischerweise eine Mehrzahl von Stereozilien (Härchen) aufweist und mit einer Mehrzahl von Nervenfasern gekoppelt ist.

Es existieren bereits einige Ansätze, um in Anlehnung an die Vorgänge in dem menschlichen Gehör Audiosignale zu verarbeiten bzw. zu identifizieren. Beispielsweise beschreiben Thorsten Heinz und Andreas Brückmann in dem Artikel "Using a physiological ear model for automatic melody transcription and sound source recognition" (Die Verwendung eines physiologischen Ohrmodells zur automatischen Melodieübersetzung und Geräuschquellenerkennung), der auf der 114. Versammlung der Audio-Ingenieur-Gemeinschaft (Audio Engineering Society) in Amsterdam, Niederlande im März 2003 vorgestellt wurde, eine Audiosignalanalyse und an der Wahrnehmung orientierte Modifikationen von konventionellen Signalverarbeitungsalgorithmen.

Der genannte Artikel beschreibt eine Nachbildung der Funktionalität des Innenohrs einschließlich der Umwandlung von mechanischen Vibrationen in Information über eine Konzentration einer Transmittersubstanz in den Spalten der inneren Haarzellen. Die Basilarmembran wird hierbei in 251 Regionen von gleichmäßiger Breite aufgeteilt, und jedes Segment wird an eine innere Haarzelle angeschlossen, wobei die innere Haarzelle durch Vibrationen des entsprechenden Abschnitts der Basilarmembran angeregt wird. Für eine Tonhöhenerkennung wird dann die Konzentration der Transmittersubstanz in den Spalten der 251 beschriebenen Haarzellen ausgewertet.

Dazu werden Tonhöhen-Trajektorien gebildet und segmentiert. Weiterhin beschreibt der genannte Artikel kurz die Erkennung einer Klangfarbe sowie eine Melodieerkennung.

Ferner beschreiben Toshio Irino und Roy D. Patterson in ihrem Artikel "Segregating Information about the size and shape of the vocal tract using a time domain auditory model: The Stabilized Wavelet-Mellin Transform" (Trennen von Informationen über die Größe und die Form des Vokaltrakts unter Verwendung eines Zeitdomänen-Gehörmodells: Die stabilisierte Wavelet-Mellin Transformation)(veröffentlicht in dem Elsevier-Journal for Speech Communication 36, 2002, Seiten 181-203) die Anwendung einer zweidimensionalen Mellin-Transformtion auf ein Gehörbild (Auditory Image). Gemäß dem genannten Artikel erzeugt die Mellin-Transformation aus dem Gehörbild (Auditory Image) ein Mellin-Abbild (Mellin Image), das invariant gegenüber der Größe eines Vokaltrakts eines Sprechers ist, auf dessen Sprachsignal das Gehörbild (Auditory Image) basiert.

Der genannte Artikel schlägt eine Spracherkennung anhand eines sogenannten Mellin-Abbilds vor, das durch eine räumliche Fourier-Transformation aus einem Größen-Form-Bild (Size-Shape-Image) entsteht. Das Größen-Form-Bild wird hingegen gemäß dem T. Irino und R.D. Patterson aus einem stabilisierten Gehörbild (Stabilized Auditory Image) durch eine Mehrzahl von Umwandlungsschritten gewonnen.

Ferner beschreiben A. Brückmann, F. Klefenz und A. Wünsche in dem Artikel "A neural net for 2D-slope and sinusoidal shape detection" (erschienen in dem CIST International Scientific Journal of Computing, ISSN 1727-6209) (Ein neuronales Netzwerk zur Erkennung von zweidimensionalen geraden und sinusförmigen Formen) ein neuronales Netzwerk zur Mustererkennung. Das beschriebene neuronale Netzwerk kann Geraden in verschiedener Steigung oder einem Satz von sinusförmigen Kurven verschiedener Frequenzen lernen und die entsprechenden Muster nach der Lernphase erkennen. Das entsprechende neuronale Netzwerk realisiert damit eine Hough-Transformation und ermöglicht also eine Erkennung von zweidimensionalen Mustern.

Die US 5,381,512 A beschreibt eine Verarbeitung einer Anregungs-Wellenform unter Verwendung eines Modells des menschlichen Gehörsystems, um eine Mehrzahl von Ausgangs-Wellenformen bereitzustellen. Jede Ausgangswellenform entspricht einer Anregung an unterschiedlichen Orten entlang einer Basilarmembran in der Cochlea und entspricht der schmalen Frequenz-Bandweite, der kurzen Zeitantwort und den Wellenausbreitungscharakteristika der menschlichen Cochlea. Eine primäre Merkmalsdetektion wird erreicht, indem Antwort-Wellenformen und deren räumliche und zeitliche Ableitung mit vorbestimmen Vergleichsmustern verglichen werden. Eine sekundäre Merkmalsdetektion wird erreicht, indem räumliche und zeitliche Muster von primären Merkmalen mit Mustern von typischen menschlichen Sprachelementen verglichen werden.

Es ist die Aufgabe der vorliegenden Erfindung, ein Konzept zum effizienten Erzeugen einer Analysedarstellung eines Audiosignals zu schaffen, wobei die Analysedarstellung ein geringes Datenaufkommen aufweist und sich dennoch gleichzeitig für eine Spracherkennung eignet.

Diese Aufgabe wird durch eine Vorrichtung gemäß Anspruch 1, ein Verfahren gemäß Anspruch 30, sowie ein Computerprogramm gemäß Anspruch 31 gelöst.

Die vorliegende Erfindung schafft eine Vorrichtung zum Analysieren eines Audiosignals, um eine Analysedarstellung des Audiosignals zu erhalten, mit einer Einrichtung zum Berechnen eines Nervenaktivitätsmusters über der Zeit an Nervenfasern eines Ohrmodells, das sich aufgrund des Audiosignals ergibt, und eine Einrichtung zum Verarbeiten des Nervenaktivitätsmusters, um als Analysedarstellung eine Folge von Zeitinformationen zu erhalten, die eine zeitliche Lage von aufeinanderfolgenden Trajektorien beschreiben, wobei eine Trajektorie Aktivitätsimpulse auf verschiedenen Nervenfasern aufgrund des gleichen Ereignisses in dem Audiosignal umfasst.

Es ist der Kerngedanke der vorliegenden Erfindung, dass Zeitinformationen, die eine zeitliche Lage von aufeinanderfolgenden Trajektorien eines Nervenaktivitätsmusters beschreiben, sich besonders gut für eine Analyse des Audiosignals eignen. Die Trajektorien beschreiben nämlich präzise das Auftreten von Ereignissen in dem Audiosignal und die Ausbreitung von Schallwellen auf einer Basilarmembran eines menschlichen Ohrs. Ferner ist festzuhalten, dass eine zeitliche Lage von aufeinanderfolgenden Trajektorien für verschiedene Laute (Vokale oder Konsonanten) oder Geräusche variiert. Damit eignen sich die Zeitinformationen hervorragend für eine Analyse eines Audiosignalinhalts des Audiosignals oder für eine Spracherkennung.

Es wurde ferner außerdem erkannt, dass eine Ermittlung der Trajektorien in dem Nervenaktivitätsmuster in einer besonders effizienten Weise erfolgen kann, und dass entsprechend Zeitinformationen, die eine zeitliche Lage von aufeinanderfolgenden Trajektorien beschreiben, mit geringem Verarbeitungsaufwand extrahiert werden können.

Schließlich beruht die vorliegende Erfindung auch auf der Erkenntnis, dass sich ein Nervenaktivitätsmuster besonders gut für eine Extraktion von Trajektorien eignet, da das Nervenaktivitätsmuster (zeitlich) wohldefinierte Aktivitätsimpulse (Aktionspotentiale) umfasst, die sich für eine Extraktion von Trajektorien besonders gut eignen.

Die vorliegende Erfindung bringt somit eine Reihe von wesentlichen Vorteilen gegenüber herkömmlichen Konzepten zum Analysieren eines Audiosignals mit sich. Aufgrund er Verwendung des Nervenaktivitätsmusters kann eine hohe Genauigkeit erzielt werden. Im Übrigen kann eine Analyse des Audiosignals in einer Weise erfolgen, die dem menschlichen Gehörempfinden nachvollzogen ist. Sämtliche in einem menschlichen Gehör auftretenden Phänomene bis hin zur Erzeugung von Aktionspotentialen in Nervenfasern des Gehörnervs könne in dem Nervenaktivitätsmuster berücksichtigt werden, so dass das Nervenaktivitätsmuster eine Information enthält, die auch ein menschliches Gehirn aufgrund eines Audiosignals empfangen würde.

Eine Extraktion von Trajektorien ist hierbei besonders vorteilhaft, da Trajektorien eine große Anzahl von Aktivitätsimpulsen auf die Nervenfasern zusammenfassen, wobei die Trajektorien durch einige wenige Parameter beschrieben werden. Von diesen Parametern ist die zeitliche Lage der wichtigste Parameter. Insbesondere bei der Auswertung von direkt aufeinanderfolgenden Trajektorien können relative Informationen über die zeitliche Lage der Trajektorien gewonnen werden, die für ein Audiosignal charakteristisch sind. Aufgrund der Zeitinformationen, die die zeitliche Lage von Trajektorien beschreiben, kann somit sowohl der Anfangszeitpunkt eines Lauts als auch eine Struktur des Nervenaktivitätsmusters innerhalb des Lauts durch eine geringe Anzahl von Parametern beschrieben werden.

Es hat sich ferner gezeigt, dass die erfindungsgemäße Analysedarstellung in Form von Zeitinformationen, die eine zeitliche Lage von direkt aufeinanderfolgenden Trajektorien beschreiben, eine besonders zuverlässige Analyse von Vokalen innerhalb eines Sprachsignals ermöglichen. Ferner hat sich auch gezeigt, dass die Trajektorien mit Hilfe von Methoden zur Mustererkennung in einer recheneffizienten Weise extrahiert werden können, wobei beispielsweise die Anwendung einer Hough-Transformation besonders vorteilhaft ist, da mit Hilfe einer Hough-Transformation auch gekrümmte Trajektorien ohne weiteres erkannt werden können.

Es sei im Übrigen darauf hingewiesen, dass ein Nervenaktivitätsmuster eine Aktivität bzw. Aktivitätsimpulse einer Gruppe von Nervenfasern des Ohrmodells beschreibt. Die Zeitinformationen kennzeichnen bevorzugter Weise einen Zeitpunkt eines Auftretens einer Trajektorie. Ferner wird darauf hingewiesen, dass eine Trajektorie bei einer erfindungsgemäßen Vorrichtung bevorzugterweise dann erkannt wird, wenn Aktivitätsimpulse, die durch das gleiche Ereignis in dem Audiosignal bedingt sind, auf einer Anzahl von Nervenfasern vorliegen, die größer als eine vorgegebene Mindestanzahl ist. In anderen Worten, es werden nur "ausreichend lange" Trajektorien erfasst, die auf mehr als einer vorgegeben Anzahl von Nervenfasern eintreffen. Dadurch kann in einer effizienten Weise verhindert werden, dass Störimpulse, die eben keine ausgedehnte Trajektorie bilden, fälschlicherweise als Trajektorie identifiziert werden.

Ein akustisches Ereignis kann beispielsweise ein Beginn eines Vokals, eines Konsonanten oder eines Lauts sein. Im Übrigen sei darauf hingewiesen, dass einem akustischen Ereignis auch mehrere Trajektorien zugeordnet sein können, wie beispielsweise aus bekannten Cochleogrammen ersichtlich ist. Beispielsweise kann ein Vokal eine Mehrzahl von Trajektorien umfassen, die in der erfindungsgemäßen Weise erkannt werden können, soweit sie eine ausreichende Länge und Intensität aufweisen.

Ferner sei darauf hingewiesen, dass eine Trajektorie in dem Nervenaktivitätsmuster typischerweise eine Wanderwelle auf einer Basilarmembran eines Ohrmodells beschreibt. In anderen Worten, eine Trajektorie beschreibt ein Nervenaktivitätsmuster auf einer Gruppe von benachbarten Nervenzellen, das einer Wanderwelle auf der Basilarmembran des Ohrmodells zugeordnet ist. In anderen Worten, eine Trajektorie beschreibt ein Auftreten von Aktivitätsimpulsen auf einer Gruppe von benachbarten Nervenfasern, wobei die Aktivitätsimpulse auf den benachbarten Nervenfasern zeitlich leicht versetzt durch eine Wanderwelle angeregt werden. Hierbei kann es freilich sein, dass einzelne Nervenfasern (beispielsweise aufgrund einer Störung) nicht angeregt werden, dass die Trajektorie also eine Unterbrechung aufweist, die allerdings nicht länger als 25 % ihrer Gesamtlänge ist. Es hat sich nämlich gezeigt, dass die erfindungsgemäßen Verfahren zum Bestimmen von Trajektorien eine Fehlertoleranz aufweisen, so dass auch nicht-ideale Trajektorien, die beispielsweise kurze Unterbrechungen aufweisen, erkannt und charakterisiert werden können.

Die Zeitinformation umfasst bevorzugterweise einen Anfangszeitpunkt der Trajektorie, wobei ein Anfangszeitpunkt einer vorgegebenen Trajektorie ein Zeitpunkt ist, zu dem ein erster zu der vorgegebenen Trajektorie gehöriger Aktivitätsimpuls aus einer Nervenfaser auftritt. Die Zeitinformation kann allerdings auch eine andere Information über die Trajektorie tragen, wie beispielsweise einen mittleren Zeitpunkt der Trajektorie oder einen Endzeitpunkt.

Ferner wird es bevorzugt, dass die Einrichtung zum Verarbeiten des Nervenaktivitätsmusters eine Einrichtung zur Mustererkennung umfasst, die ausgelegt ist, um in einer zweidimensionalen Darstellung, die das Nervenaktivitätsmuster über der Zeit beschreibt, ein gerades oder gekrümmtes linienförmiges Muster als eine Trajektorie zu erkennen, die zeitliche Lage der Trajektorie zu ermitteln und eine zu der Trajektorie gehörige Zeitinformation als Analysedarstellung des Audiosignals zu liefern. Es hat sich nämlich gezeigt, dass eine Analyse einer zweidimensionalen Darstellung des Nervenaktivitätsmusters eine besonders effiziente Erkennung von Trajektorien ermöglicht. Ferner sind zweidimensionale Analyseverfahren in der Lage, gerade oder gekrümmte linienförmige Strukturen in einer effizienten Weise zu erkennen und zu charakterisieren. Ferner hat sich gezeigt, dass Trajektorien, also linienförmige gerade oder gekrümmte Strukturen, mit einer der guten Genauigkeit erkannt werden können, da durch die räumliche Ausdehnung der Trajektorien bei einer Erkennung eine Mittlung über eine Mehrzahl von Nervenfasern (die durch das Nervenaktivitätsmuster beschrieben sind) erfolgen kann.

Ferner wird es bevorzugt, dass die Einrichtung zur Mustererkennung ausgelegt ist, um eine Information über eine Form der Trajektorien zu liefern. Die Form der Trajektorien liefert nämlich noch eine weitere Information, die in Kombination mit der Zeitinformation eine Weiterverarbeitung der Analysedarstellung des Audiosignals erleichtern bzw. eine aussagekräftigere Weiterverarbeitung der Analysedarstellung des Audiosignals ermöglichen kann. Die Information über die Form der Trajektorie umfasst bevorzugterweise auch eine Information über eine Krümmung der Trajektorie.

Da sich gezeigt hat, dass Trajektorien in dem Nervenaktivitätsmuster typischerweise hyperbelartig gebogen sind, wird es bevorzugt, dass die Einrichtung zur Mustererkennung darauf optimiert ist, gerade oder hyperbelförmig gekrümmte Trajektorien zu erkennen.

Ferner ist es vorteilhaft, wenn die Einrichtung zur Mustererkennung ausgelegt ist, um eine Information über eine Länge der Trajektorie zu liefern. Auch die Länge der Trajektorie kann nämlich bei einer Weiterverarbeitung der Analysedarstellung zur Verbesserung der Genauigkeit verwendet werden. Im Übrigen ist es auch vorteilhaft, wenn von der Einrichtung zur Mustererkennung eine Information geliefert wird, die eine Aussage darüber umfasst, über welchen Frequenzbereich (ausgedrückt beispielsweise durch Nummern der Nervenfasern, auf denen ein zu der Trajektorie gehöriges Aktionspotential vorliegt) sich eine Trajektorie erstreckt. Damit kann also nicht nur die Anwesenheit einer Trajektorie, sondern beispielsweise auch der angeregte Frequenzbereich ermittelt werden.

Ferner wird es bevorzugt, dass die Einrichtung zum Verarbeiten des Nervenaktivitätsmusters eine Einrichtung zum Mustervergleich umfasst, die ausgelegt ist, um eine zweidimensionale Darstellung, die das Nervenaktivitätsmuster über der Zeit beschreibt, mit mindestens einem Vergleichsmuster zu vergleichen, um eine Trajektorie zu erkennen, und um eine Zeitinformation zu erhalten, die eine zeitliche Lage der Trajektorie beschreibt. Ein Mustervergleich ist eine effiziente Möglichkeit, um in einer zweidimensionalen Darstellung ein Muster, wie beispielsweise eine Trajektorie, zu erkennen. Als Vergleichsmuster kann hierbei bevorzugterweise eine gerade oder eine hyperbelförmige Kurve dienen.

Es hat sich ferner herausgestellt, dass das Erkennen von geraden oder gekrümmten Trajektorien besonders vorteilhaft erreicht werden kann, indem die Einrichtung zum Verarbeiten des Nervenaktivitätsmusters ausgelegt ist, um eine zweidimensionale Darstellung des Nervenaktivitätsmusters schrittweise zu verzerren, um eine verzerrte Darstellung des zweidimensionalen Nervenaktivitätsmusters zu erhalten, und um zu erkennen, wenn in der verzerrten zweidimensionalen Darstellung des Nervenaktivitätsmusters eine näherungsweise gerade Linie enthalten ist. Die gerade Linie kann dann als Trajektorie erkannt werden, woraufhin eine zeitliche Lage der Trajektorie ermittelt und der erkannten Trajektorie eine zugehörige Zeitinformation zugeordnet werden kann. Eine derartige Einrichtung zum Verarbeiten des Nervenaktivitätsmusters ist besonders vorteilhaft, da das Vorhandensein einer geraden Linie einfach überprüft werden kann. Eine "gerade Linie" einer Trajektorie ist hierbei dadurch definiert, dass in einer Verarbeitungsstufe der Einrichtung zum Verarbeiten des Nervenaktivitätsmusters eine Mehrzahl von Aktivitätsimpulsen gleichzeitig bzw. zumindest zeitlich überlappend eintreffen. Ein gleichzeitiges bzw. zeitlich überlappendes Eintreffen von mehreren Aktivitätsimpulsen kann beispielsweise durch eine Summation der in einer Stufe eintreffenden Signale ermittelt werden, wobei ein gleichzeitiges Eintreffen von Aktivitätsimpulsen in einer Stufe eine ausgeprägte Spitze in einem Summationsergebnisses der Summation hervorruft.

Das schrittweise Verzerren der zweidimensionalen Darstellung des Nervenaktivitätsmusters kann beispielsweise durch eine Mehrzahl von Verzögerungseinrichtungen erfolgen, wobei Signale bzw. Nervensignale, die das Nervenaktivitätsmuster beschreiben, in Stufen der Einrichtung zum Verarbeiten des Nervenaktivitätsmusters verschieden stark verzögert werden.

In anderen Worten, das Verzerren der zweidimensionalen Darstellung des Nervenaktivitätsmusters erfolgt derart, dass eine gekrümmte Trajektorie durch das schrittweise Verzerren schrittweise gerade gebogen wird, wobei die Anzahl von Verzögerungsschritten, die für ein Geradebiegen der gekrümmten Trajektorie benötigt werden, eine Information über eine Krümmung der gekrümmten Trajektorie umfassen.

Ferner wird es bevorzugt, dass die Einrichtung zum Verarbeiten des Nervenaktivitätsmusters eine Kurvenerkennungseinrichtung umfasst, die ausgelegt ist, um das Nervenaktivitätsmuster über der Zeit in Form einer Mehrzahl von Signalen parallel zu empfangen, und um die Signale unterschiedlich schnell (bzw. mit unterschiedlichen Verzögerungen) parallel durch eine Mehrzahl von hintereinander geschalteten Stufen weiterzuleiten, wobei eine ausgewählte Stufe ausgelegt ist, um zu erkennen, wenn mindestens eine vorgegebene Anzahl von Signalen in der ausgewählten Stufe gleichzeitig aktiv sind.

Eine unterschiedlich schnelle Weiterleitung von mehreren Signalen, die das Nervenaktivitätsmuster bilden, ermöglicht ein Verbiegen von Trajektorien, die in dem ursprünglichen Nervenaktivitätsmuster vorhanden sind. Ist in einer Stufe eine Mehrzahl von Signalen aktiv, so deutet dies darauf hin, dass eine Trajektorie gerade gebogen oder näherungsweise gerade gebogen ist. Mit anderen Worten, laufen Aktivitätsimpulse gleichzeitig oder zumindest überlappend in eine Stufe ein, so kann die Stufe beispielsweise aufgrund einer Summation mit einer darauffolgenden Schwellwertentscheidung erkennen, dass eine (näherungsweise) geradegebogene Trajektorie in der Stufe anliegt. In anderen Worten, eine Stufe ist bevorzugt ausgelegt, um mehrere Signale bei einer Weiterleitung durch die Stufe unterschiedlich stark zu verzögern.

Weiterhin wird es bevorzugt, die Kurvenerkennungseinrichtung als ein neuronales Netz auszuführen. Ein solches neuronales Netz kann eine Mehrzahl von Trajektorien (bzw. Trajektorien-Formen) lernen und diese dann in einem laufenden Betrieb erkennen. Es ist ein weiterer Vorteil eines neuronalen Netzes, dass dieses auch nicht-ideale Trajektorien, die einem idealen Lernmuster sehr ähnlich sind, erkennen kann. Somit ist ein neuronales Netz eine sehr gute Ausführungsform zur Erkennung von Trajektorien in einem Nervenaktivitätsmuster, da auch hierbei nicht ideale Muster zu erkennen sind, die Schwankungen und Störungen unterworfen sein können.

Schließlich sei darauf hingewiesen, dass die Kurvenerkennungseinrichtung bevorzugt ausgelegt ist, um eine Trajektorie aufgrund einer Auswertung einer Hough-Transformation zu erkennen. Eine Hough-Transformation ist nämlich ein sehr effektives Verfahren, um analytisch beschreibbare Kurven, aber auch beliebige Kurven, in einer zweidimensionalen Darstellung zu erkennen. Die Ausführung der Hough-Transformation erfolgt bevorzugter Weise in einer parallelen Form, so dass eine sehr schnelle Berechnung erfolgen kann.

Bevorzugte Ausführungsbeispiele der vorliegenden Erfindung werden nachfolgend bezugnehmend auf die beiliegenden Zeichnungen näher erläutert. Es zeigen:

Fig. 1 ein Flussdiagramm eines erfindungsgemäßen Verfahrens zum Berechnen einer Analysedarstellung eines Audiosignals aufgrund des Audiosignals gemäß einem ersten Ausführungsbeispiels der vorliegenden Erfindung;

Fig. 2 ein Flussdiagramm eines erfindungsgemäßen Verfahrens zum Berechnen eines Neurotransmitter-Vesikel-Auftretens aufgrund des Audiosignals gemäß dem ersten Ausführungsbeispiel der vorliegenden Erfindung;

Fig. 3 ein Flussdiagramm eines erfindungsgemäßen Verfahrens zum Berechnen einer Basilarmembranbewegung aufgrund des Audiosignals gemäß dem ersten Ausführungsbeispiel der vorliegenden Erfindung;

Fig. 4 ein Flussdiagramm eines erfindungsgemäßen Verfahrens zum Berechnen einer Auslenkung eines Stereoziliums aufgrund der Basilarmembranbewegung gemäß dem ersten Ausführungsbeispiel der vorliegenden Erfindung;

Fig. 5 ein Flussdiagramm eines erfindungsgemäßen Verfahrens zum Berechnen des Neurotransmitter-Vesikel-Auftretens aufgrund der Auslenkung eines Stereoziliums gemäß dem ersten Ausführungsbeispiel der vorliegenden Erfindung;

Fig. 6 ein Blockschaltbild eines erfindungsgemäßen Verfahrens zum Berechnen eines Aktionspotenzials einer Nervenfaser aufgrund des Neurotransmitter-Vesikel-Austretens gemäß dem ersten Ausführungsbeispiel der vorliegenden Erfindung;

Fig. 7 ein Flussdiagramm eines erfindungsgemäßen Verfahrens zum Berechnen einer Analysedarstellung eines Audiosignals gemäß einem zweiten Ausführungsbeispiel der vorliegenden Erfindung;

Fig. 8 eine graphische Darstellung von beispielhaften Nervenaktivitätsmustern;

Fig. 9 eine graphische Darstellung einer Verzögerung bei einer Ausbreitung von Audiosignalen verschiedener Frequenz auf einer Basilarmembran;

Fig. 10 eine graphische Darstellung eines Cochleograms für einen Vokal "i";

Fig. 11 eine graphische Darstellung eines Cochleograms, einer Transmitter-Freisetzungs-Wahrscheinlichkeit und einer Transmitter-Vesikel-Freisetzung für einen Vokal "A";

Fig. 12 eine graphische Darstellung einer Verarbeitungskette für eine erfindungsgemäße Analyse eines Audiosignals gemäß dem zweiten Ausführungsbeispiels der vorliegenden Erfindung;

Fig. 13 ein Blockschaltbild einer Vorrichtung zum erfindungsgemäßen Verarbeiten des Nervenaktivitätsmusters gemäß dem zweiten Ausführungsbeispiel der vorliegenden Erfindung;

Fig. 14 eine graphische Darstellung von Signalen in einer Vorrichtung zum erfindungsgemäßen Verarbeiten des Nervenaktivitätsmusters gemäß dem zweiten Beispiel der vorliegenden Erfindung;

Fig. 15 ein Schaltbild eines Hubel-Wiesel-Netzes zur erfindungsgemäßen Berechnung einer Analysedarstellung eines Audiosignals gemäß dem zweiten Ausführungsbeispiel der vorliegenden Erfindung;

Fig. 16 ein Schaltbild eines Hubel-Wiesel-Netzes zur erfindungsgemäßen Berechnung einer Analysedarstellung eines Audiosignals gemäß dem zweiten Ausführungsbeispiel der vorliegenden Erfindung;

Fig. 17 eine graphische Darstellung von Trainingsmustern zum Trainieren eines Hubel-Wiesel-Netzes;

Fig. 18 eine schematische Darstellung einer Einrichtung zum Identifizieren eines Audiosignals:

Fig. 19 eine schematische Darstellung einer Einrichtung zur Extraktion eines Audiosignal-Inhaltes;

Fig. 20 eine graphische Darstellung der auditorischen Peripherie;

Fig. 21 eine graphische Darstellung der Außenohrübertragungsfunktion;

Fig. 22 eine graphische Darstellung von Mittelohr und aufgerollter Cochlea;

Fig. 23 eine graphische Darstellung einer Hörfläche;

Fig. 24 eine graphische Darstellung eines Querschnitts durch eine Cochlea;

Fig. 25 eine schematische Darstellung einer Haarzelle;

Fig. 26 eine graphische Darstellung einer Anatomie der auditorischen Peripherie;

Fig. 27 eine graphische Darstellung eines Mechanismus der Signalübertragung in einem menschlichen Ohr;

Fig. 28 eine graphische Darstellung der Geometrie der Basilarmembran eines menschlichen Ohrs und einer Reaktion der Basilarmembran auf eine Anregung;

Fig. 29 eine graphische Darstellung eines erweiterten Zwicker[b1]-Modells zur Beschreibung eines Innenohrs;

Fig. 30 eine graphische Darstellung eines Cortischen Organs;

Fig. 31 eine graphische Darstellung eines Aufbaus von Haarzellen;

Fig. 32 eine graphische Darstellung von chemischen Abläufen in einer Haarzelle; und

Fig. 33 eine graphische Darstellung einer sensorischen Grube.

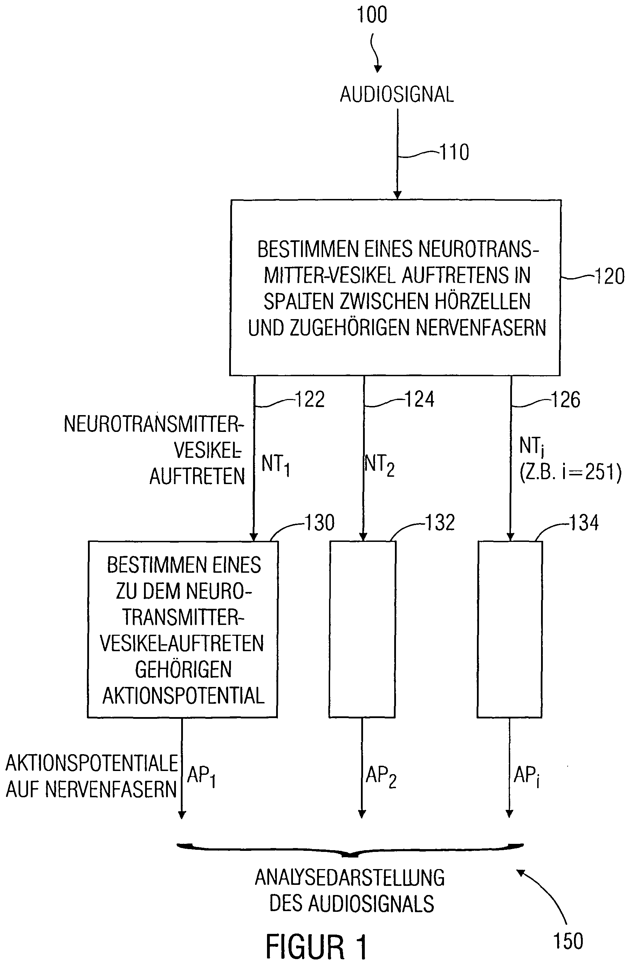

Fig. 1 zeigt ein Flussdiagramm eines erfindungsgemäßen Verfahrens zum Berechnen einer Analysedarstellung eines Audiosignals aufgrund des Audiosignals gemäß dem ersten Ausführungsbeispiel der vorliegenden Erfindung. Das Flussdiagramm der Fig. 1 ist in seiner Gesamtheit mit 100 bezeichnet.

Dabei wird für das Audiosignal 110 in einem ersten Schritt 120 ein Neurotransmitter-Vesikel-Auftreten in Spalten zwischen Hörzellen und zugehörigen Nervenfasern bestimmt. Das Auftreten von Neurotransmitter-Vesikeln, die typischerweise zwischen hundert und mehreren tausend Neurotransmitter-Molekülen umfassen, wird hierbei für eine Mehrzahl von Hörzellen bestimmt, wobei angenommen wird, dass die Hörzellen über ein Modell eines Ohrs räumlich verteilt sind. Es kann beispielsweise angenommen werden, dass die bei der entsprechenden Simulation des Neurotransmitter-Vesikel-Auftretens berücksichtigten Hörzellen äquidistant oder näherungsweise äquidistant über eine Cochlea eines Ohrmodells verteilt sind. Aufgrund der Anregung der Hörzellen durch das Audiosignal kann also für jede der betrachteten Hörzellen ein Auftreten von Neurotransmitter-Vesikeln bestimmt werden.

Basierend auf dem Neurotransmitter-Vesikel-Auftretens 122 kann dann in einem zweiten Schritt 130 in einer betrachteten Hörzelle ein Aktionspotenzial AP 1 auf einer Nervenfaser, die mit der betrachteten Hörzelle gekoppelt ist, bestimmt werden. Das gezeigte Vorgehen kann dann für alle Hörzellen wiederholt werden, die bei der Bestimmung des Neurotransmitter-Vesikel-Auftretens berücksichtigt wurden (Schritte 132 und 134). Es wird somit für alle bei der Bestimmung des Neurotransmitter-Vesikel-Auftretens beteiligten Hörzellen jeweils separat ein Aktionspotenzial AP1, AP2, AP3 auf einer der jeweiligen Hörzelle zugeordneten Nervenfaser aufgrund des der jeweiligen Hörzelle zugeordneten Neurotransmitter-Vesikel-Auftretens 122, 124, 126 berechnet.

Mit anderen Worten, werden i Hörzellen berücksichtigt, so wird für alle i Hörzellen ein zugehöriges Neurotransmitter-Vesikel-Auftreten 122, 124, 126 berechnet. Aufgrund des Neurotransmitter-Vesikel-Auftretens wird dann (für jede Nervenfaser separat) ein zugehöriges Aktionspotential AP1, AP2, AP3 berechnet. Somit liegen nach Abschluss der Berechnung i Aktionspotenziale auf Nervenfasern vor, die zusammen das Nervenaktivitätsmuster bilden.

Das Nervenaktivitätsmuster AP1, AP2, AP3 stellt somit eine Analysedarstellung des Audiosignals dar, die für eine weitere Verarbeitung, beispielsweise eine Audiosignalerkennung, verwendet werden kann.

Das erfindungsgemäße Verfahren bietet somit den Vorteil, dass eine besonders präzise und aussagekräftige Analysedarstellung des Audiosignals gebildet werden kann. Die Aktionspotenziale AP1, AP2, AP3 (die in ihrer Gesamtheit das Nervenaktivitätsmuster bilden) auf den Nervenfasern sind nämlich denjenigen Signalen sehr ähnlich, die von einem menschlichen Gehirn für eine Erkennung von akustischen Ereignissen verwendet werden.

Bei dem erfindungsgemäßen Verfahren, bei dem die Aktionspotenziale AP1, AP2, AP3 auf Nervenfasern von Neurotransmitter-Vesikel-Auftreten 122, 124, 126 abgeleitet werden, kann eine besonders hohe Genauigkeit bei einer nachfolgenden Analyse des Audiosignals erzielt werden. Die Aktionspotenziale auf den Nervenfasern tragen nämlich eine genaue zeitliche Information, da die Aktionspotenziale AP1, AP2, AP3 gequantelt auftreten. Ferner tritt bei der Bestimmung der Aktionspotenziale AP1, AP2, AP3 eine Totzeit (Refraktärzeit) auf, die bei bekannten Verfahren zur Bestimmung von Analysedarstellungen eines Audiosignals nicht berücksichtigt wird.

Im Übrigen ist festzuhalten, dass die Aktionspotenziale AP1, AP2, AP3 aufgrund ihrer Quantelung einfach dargestellt werden können, wobei nicht die Höhe eines Aktionspotenzials, sondern der Zeitpunkt des Auftretens eines Aktionspotenzials bzw. die Rate der nacheinander auftretenden Aktionspotenziale die Information trägt. Auch dadurch unterscheidet sich das erfindungsgemäße Verfahren wesentlich von bekannten Verfahren, bei denen beispielsweise eine Konzentration von Neurotransmittern in einem synaptischen Spalt ausgewertet wird, wobei die Konzentration eine kontinuierliche Kurve darstellt, die keine zeitlich scharf definierten Änderungen aufweist.

Im Übrigen ist festzuhalten, dass sich das Nervenaktivitätsmuster, das simulierte Aktionspotenziale AP1, AP2, AP3 auf einer Mehrzahl von Nervenfasern umfasst, auch verwendet werden kann, um Nervenfasern eines Hörnervs des menschlichen Patienten anzuregen, der beispielsweise einen Gehörschaden aufweist.

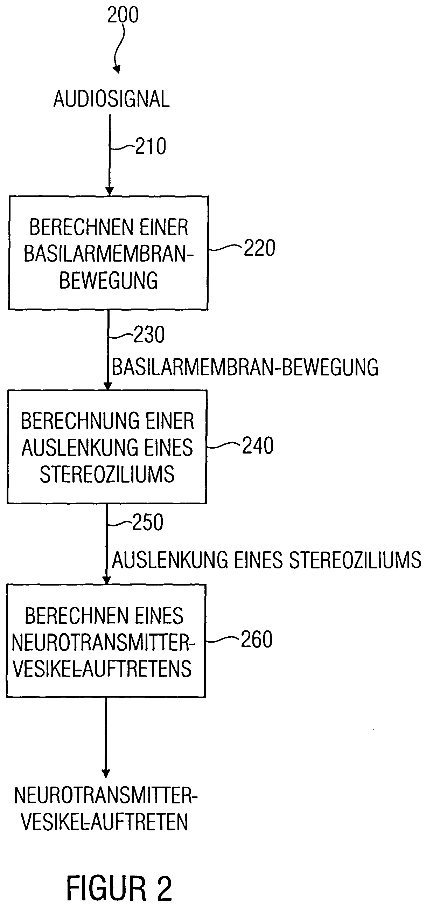

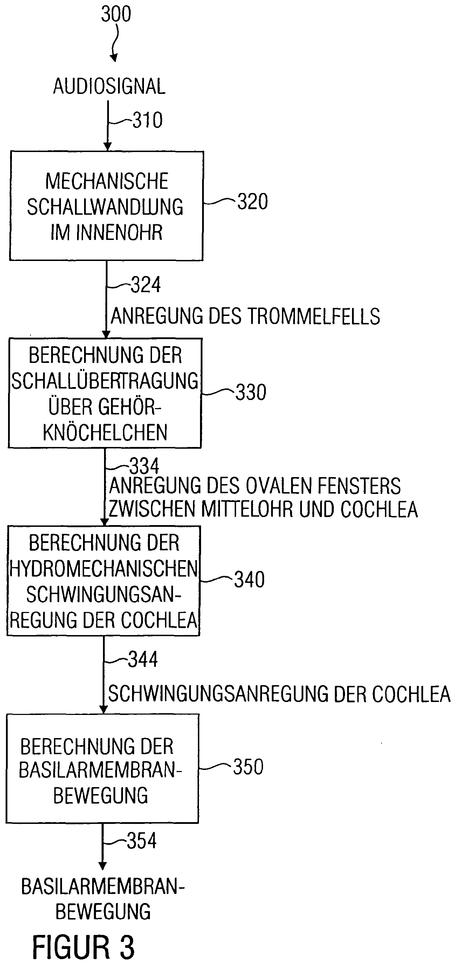

Fig. 2 zeigt ein Flussdiagramm eines erfindungsgemäßen Verfahrens zum Berechnen des Neurotransmitter-Vesikel-Auftretens aufgrund des Audiosignals gemäß dem ersten Ausführungsbeispiel der vorliegenden Erfindung. Das Flussdiagramm der Fig. 2 ist in seiner Gesamtheit mit 200 bezeichnet. Für ein Audiosignal 210 wird in einem ersten Schritt 220 eine zugehörige Basilarmembran-Bewegung 230 berechnet. Mit anderen Worten, basierend auf dem Audiosignal 210 wird eine Bewegung einer Basilarmembran unter Verwendung eines Ohrmodells berechnet. Die Bewegung kann beispielsweise durch Geschwindigkeit und/oder Auslenkung von verschiedenen Punkten der Basilarmembran beschrieben werden. Im Übrigen wird darauf hingewiesen, dass die Berechnung der Basilarmembran-Bewegung anhand von Fig. 3 näher erläutert wird.

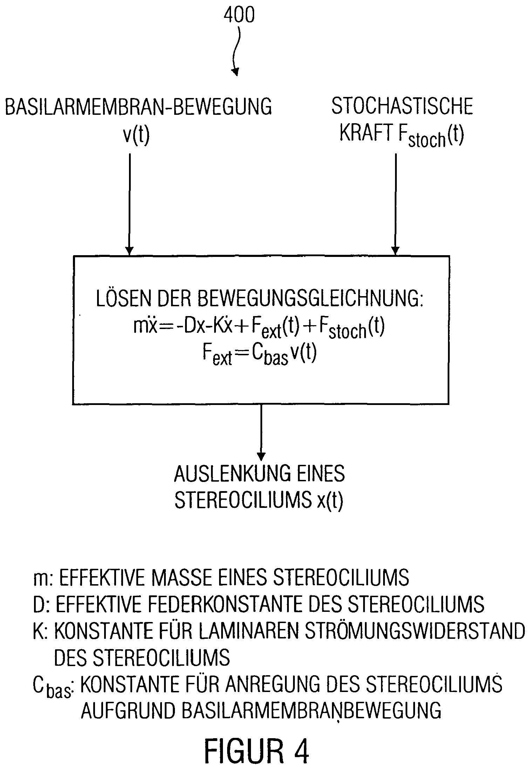

Basierend auf der Basilarmembran-Bewegung 230 wird dann in einem zweiten Schritt 240 eine Auslenkung 250 eines Stereoziliums, das mit der Basilarmembran gekoppelt ist, berechnet. Die Berechnung der Auslenkung des Stereoziliums wird im Übrigen anhand von Fig. 4 näher ausgeführt.

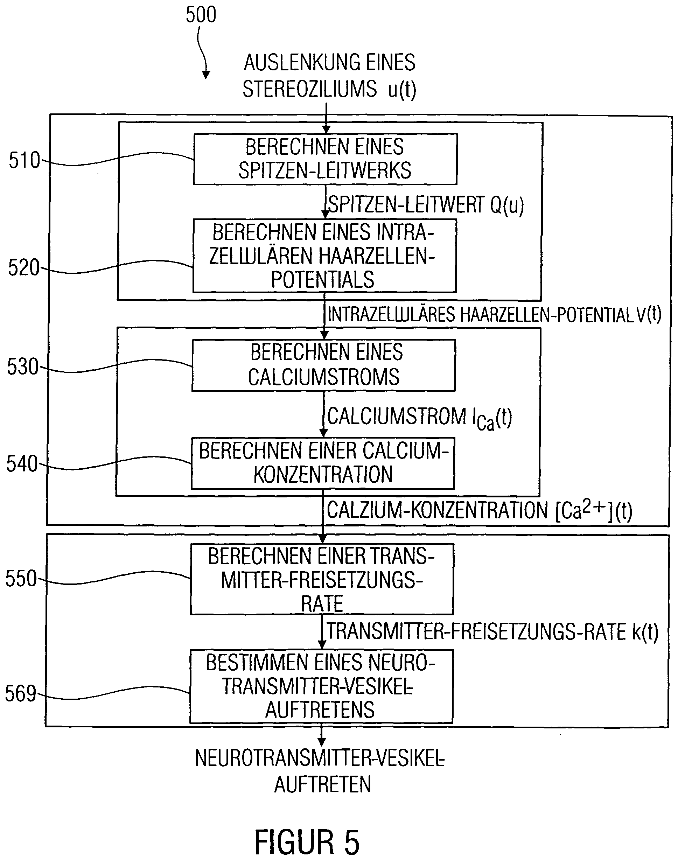

Aufgrund einer bekannten Auslenkung 250 des Stereoziliums kann in einem dritten Schritt 260 das Neurotransmitter-Vesikel-Auftreten berechnet bzw. bestimmt werden. Das Berechnen des Neurotransmitter-Vesikel-Auftretens wird im Übrigen anhand von Fig. 5 noch näher erläutert. Somit liefert das in Fig. 2 gezeigte Verfahren zu einem Audiosignal ein Neurotransmitter-Vesikel-Auftreten für eine oder mehrere Hörzellen. Es wird hierbei darauf hingewiesen, dass die Basilarmembran-Bewegung 230 bevorzugt an den Orten berechnet wird, an denen sich die betrachteten Hörzellen befinden. Es kann jedoch auch die gesamte Bewegung der Basilarmembran berechnet werden, soweit dies eine vorteilhafte Berechnung (beispielsweise unter Verwendung einer analytischen Lösung) ermöglicht.

Fig. 3 zeigt ein Flussdiagramm eines erfindungsgemäßen Verfahrens zum Berechnen einer Basilarmembran-Bewegung aufgrund eines Audiosignals gemäß dem ersten Ausführungsbeispiel der vorliegenden Erfindung. Die graphische Darstellung der Fig. 3 ist in ihrer Gesamtheit mit 300 bezeichnet.

Für ein Audiosignal 310 wird hierbei in einem ersten Schritt 320 eine mechanische Schallwandlung in einem Innenohr basierend auf einem Ohrmodell berechnet. Damit kann eine Anregung 324 des Trommelfells in dem Ohrmodells bestimmt werden. So kann beispielsweise von einem Audiosignal 310 ausgegangen werden, das auf dem Trommelfell eintrifft. Aufgrund mechanischer bzw. fluidischer Berechnungen kann damit die Anregung des Trommelfells ermittelt werden, woraufhin die Schwingung des Trommelfells bekannt ist.

In einem zweiten Schritt 330 kann dann auch die Schallübertragung über die Gehörknöchelchens des Mittelohrs in dem Ohrmodell berechnet werden. Hierbei kann eine detaillierte mechanische Analyse des Mittelohrs erfolgen. Es ist aber genauso gut möglich, lediglich Kraft- bzw.

Amplituden-Übersetzungsverhältnisse der Gehörknöchelchen zu berücksichtigen, wodurch sich eine sehr einfache Berechnung ergibt. Weiterhin ist es möglich, die Trägheit der Gehörknöchelchen und/oder fluidische Einflüsse wie Dämpfung mit zu berücksichtigen. Ferner kann schließlich für eine Berechnung der Schallübertragung über die Gehörknöchelchen auch noch berücksichtigt werden, dass die Übertragungscharakteristik des Mittelohrs in Abhängigkeit von der Schallintensität variieren kann. Unabhängig von der Komplexität des Modells, das zur Berechndung der Schallübertragung über die Gehörknöchelchen in einem zweiten Schritt 330 verwendet wird, kann als Ergebnis der beschriebenen Berechnung eine Anregung 334 des ovalen Fensters zwischen Mittelohr und Cochlea ermittelt werden.

Aufgrund einer Kenntnis der Anregung 334 des ovalen Fensters zwischen Mittelohr und Cochlea kann dann in einem dritten Schritt 340 die hydromechanische Schwingungsanregung der Cochlea berechnet werden. Dies kann durch eine geeignete hydromechanische Simulation oder anhand eines vereinfachten analytischen Modells erfolgen. Somit sind als Ergebnis einer Analyse der Cochlea fluidische Strömungen in der Cochlea bekannt bzw. können ermittelt werden.

Basierend auf einer Kenntnis der Schwingungsanregung 344 der Cochlea kann schließlich in einem vierten Schritt 350 eine Bewegung 354 der Basilarmembran berechnet werden. Hierbei können wiederum lineare oder nichtlineare mechanische Modelle zum Einsatz kommen. Es wird darauf hingewiesen, dass eine Vielzahl von Möglichkeiten besteht, um die Basilarmembran-Bewegung an ausgewählten Orten, an denen die betrachteten Nervenzellen angeordnet sind, zu berechnen.

Es wird ferner darauf hingewiesen, dass es nicht notwendig ist, alle gezeigten Zwischengrößen (also die Anregung 324 des Trommelfells, die Anregung 334 des ovalen Fensters oder die Schwingungsanregung 344 der Cochlea) explizit zu berechnen. Vielmehr existieren auch Verfahren, um näherungsweise direkt von dem Audiosignal 310 auf eine Bewegung 354 der Basilarmembran zu schließen. Hierbei kann beispielsweise ein lineares oder ein nicht-lineares Filter zum Einsatz kommen, um eine Bewegung eines vorgegebenen Punkts einer Basilarmembran (an dem sich bevorzugterweise eine Hörzelle befindet) zu ermitteln. Für die Berechnung der Basilarmembran-Bewegung 354 an mehreren Orten kann dann eine Mehrzahl von verschiedenartig ausgelegten Filtern verwendet werden. Ein Filter beschreibt dabei eine Antwort eines Ortes der Cochlea als Reaktion auf das akustische Signal.

Im Übrigen wird darauf hingewiesen, dass von F. Baumgarte ein besonders vorteilhaftes Gehörmodell für eine Modellierung der Cochlea vorgeschlagen wurde (F. Baumgarte: "Ein psychophysiologisches Gehörmodell zur Nachbildung von Wahrnehmungsschwellen für die Audiocodierung", Dissertation, Universität Hannover, 2000). Das Modell von Baumgarte erlaubt eine besonders vorteilhafte Modellierung der Cochlea eines Ohrmodells, wobei wesentliche Effekte wie etwa eine Signalreflexion an dem Ende der Cochlea mit berücksichtigt werden können.

Fig. 4 zeigt ein Flussdiagramm eines erfindungsgemäßen Verfahrens zum Berechnen einer Auslenkung eines Stereoziliums aufgrund der Basilarmembran-Bewegung. Das Flussdiagramm der Fig. 4 ist in seiner Gesamtheit mit 400 bezeichnet.

Es wird hierbei davon ausgegangen, dass die Bewegung der Basilarmembran nach einer der oben genannten Methoden (oder auch auf andere Weise) berechnet wurde, so dass eine Geschwindigkeit der Bewegung der Basilarmembran für diejenigen Orte, an denen sich zu betrachtende Nervenzellen befinden, bekannt ist. Eine Auslenkung $x(t)$ (zum Teil auch als $u(t)$ bezeichnet) eines Stereozilium kann durch Lösen einer Bewegungsgleichung in Abhängigkeit von einer Basilarmembran-Bewegung bestimmt werden. Die Basilarmembran-Bewegung ist hierbei freilich als eine relative Bewegung aufzufassen, die bevorzugt durch eine Relativgeschwindigkeit $v(t)$ beschrieben wird. Ferner wird es bevorzugt, als Anregung für das Stereozilium zusätzlich eine stochastische Kraft $F_{stoch}(t)$ zu berücksichtigen, die durch eine Stoßbewegung der Atome verursacht wird. Mit anderen Worten, es wird bevorzugt, bei der Anregung des Stereoziliums die Braun'sehe Bewegung mit zu berücksichtigen. Somit ergibt sich die Bewegungsgleichung eines Stereoziliums als inhomogene Bewegungsgleichung eines harmonischen Oszillators der Form

$$m\ddot{x} = -Dx - K\dot{x} + F_{ext}(t) + F_{stoch}(t)$$

Hierbei ist m die effektive Masse des betrachteten Stereoziliums, D eine effektive Federkonstante des Stereoziliums, und K eine Konstante für einen laminaren Strömungswiderstand des Stereoziliums, die die fluidische Dämpfung des Stereoziliums beschreibt. Die Anregung des Stereoziliums, die durch die externe Kraft $F_{ext}$ ausgedrückt wird, ist proportional zu der Relativgeschwindigkeit v(t) der Basilarmembran-Bewegung, so dass gilt:

$$F_{ext} = C_{Bas} v(t).$$

Dabei ist $C_{Bas}$ eine Konstante für eine Anregung des Stereoziliums aufgrund einer Bewegung der Basilarmembran. Wie schon erwähnt ergibt die Auswertung der gezeigten Bewegungsgleichung eine Auslenkung x(t) des Stereoziliums, die bisweilen auch mit u(t) bezeichnet wird.

Es wird im Übrigen darauf hingewiesen, dass auch andere Verfahren zur Berechnung einer Auslenkung der Stereozilien verwendet werden können. So ist es beispielsweise möglich, eine Bewegungsgleichung erster Ordnung zu verwenden, die ein Tiefpassverhalten des Stereoziliums modelliert. Mit anderen Worten, es kann bei einer alternativen Beschreibung davon ausgegangen werden, dass ein Stereozilium die Tiefpasssystem darstellt, das durch eine Bewegungsgleichung der Form eine Gleichung

$$\tau_c \dot{u}(t) + u(t) = \tau_c C_{cilia} v(t).$$

Im Übrigen sei darauf hingewiesen, dass die Berücksichtigung der durch die Braun'sche Bewegung hervorgerufenen Kraft $F_{stoch}(t)$ optional ist.

Fig. 5 zeigt ein Flussdiagramm eines erfindungsgemäßen Verfahrens zum Berechnen eines Neurotransmitter-Vesikel-Auftretens aufgrund einer Auslenkung eines Stereoziliums gemäß dem ersten Ausführungsbeispiel der vorliegenden Erfindung. Das in Fig. 5 gezeigte Flussdiagramm ist in seiner Gesamtheit mit 500 bezeichnet.

Aufgrund einer Auslenkung u(t) eines Stereozilium kann beispielsweise in einem ersten Schritt 510 ein Spitzen-Leitwert (apical conductance) G(u) berechnet werden. Eine Auslenkung eines Stereoziliums verändert nämlich eine Anzahl von offenen Ionen-Kanälen, wodurch sich ein Leitwert einer Membran einer Hörzelle ändert.

Aufgrund des aus dem ersten Schritts 510 bekannten Spitzen-Leitwerts G(u) kann somit in einem zweiten Schritt 520 ein intrazelluläres Haarzellen-Potenzial V(t) berechnet werden. Bei der Berechnung dieses Membranpotenzials des Zellkörpers kann beispielsweise ein Modell einer passiven elektrischen Schaltung verwendet werden, was die Berücksichtigung einer Zellkapazität ermöglicht. Weiterhin können die Leitfähigkeiten verschiedener Membranen der Hörzelle sowie die durch verschiedene Ionen bedingten Potenziale berücksichtigt werden.

Nach einer Berechnung des intrazellulären Haarzellen-Potenzials V(t) in dem zweiten Schritt 520 kann in einem dritten Schritt 530 ein Kalziumstrom $I_{Ca}(t)$ berechnet werden. Die Freisetzung von Neurotransmittern in den synaptischen Spalt wird nämlich durch Kalzium-Ionen vermittelt. Hierbei kann beispielsweise aufgrund des intrazellulären Haarzellen-Potenzials V(t) ein Anteil von offenen Kalziumkanälen bestimmt werden. Der Kalziumstrom an sich ist wiederum abhängig von der Anzahl der offenen Kalziumkanäle sowie einer Potenzialdifferenz zwischen dem intrazellulären Haarzellen-Potenzial V(t) und einem entgegengerichteten Potenzial für das Kalzium. Im Übrigen sei angemerkt, dass die Anzahl der offenen Kalziumkanäle einer Trägheit unterliegt, die ebenfalls berücksichtigt werden kann.

Aufgrund des Kalziumstroms $I_{Ca}(t)$ kann dann in einem vierten Schritt 540 eine Kalziumkonzentration $[Ca^{2+}](t)$ bestimmt werden. Hierbei kann wiederum beispielsweise ein Tiefpass-Charakter berücksichtigt werden, wobei angenommen werden kann, dass die Kalziumkonzentration in einem Gleichgewichtszustand einen konstanten Wert annimmt.

Aufgrund der Kalziumkonzentration $[Ca^{2+}](t)$ kann ein in einem fünften Schritt 550 eine Transmitter-Freisetzungs-Rate k(t) ermittelt werden. Hierbei kann bevorzugt die Speicherung der Transmitter-Substanzen in einem Reservoir, in dem synaptischen Spalt und in einem Verarbeitungsspeicher berücksichtigt werden. Aufgrund der Transmitter-Freisetzungs-Rate k(t) kann in einem damit gekoppelten sechsten Schritt 560 ein Auftreten bzw. eine Freisetzung von Neurotransmitter-Vesikeln bestimmt werden. Es wird hierbei bevorzugt, von einer gequantelten und stochastischen Freisetzung von Neurotransmitter-Vesikeln auszugehen, wobei ein stochastischer Transport durch eine Funktion N(n,p) beschrieben werden kann. Dabei kann beispielsweise davon ausgegangen werden, dass jedes von n Neurotransmittern-Quanten in einem Simulationsintervall eine gleiche Freisetzungswahrscheinlichkeit hat. Im Übrigen wird darauf hingewiesen, dass auch die Rückführung von Neurotransmittern bzw. Neurotransmitter-Vesikeln in einem Reservoir bei einer Modellierung mit eingeschlossen werden kann.

Es wird ferner darauf hingewiesen, dass ein vorteilhaft einsetzbares Modell einer inneren Haarzelle in dem Artikel "A revised model of the inner hair cell and auditory nerve complex" von C.J. Sumner, E.A. Lopez-Poveda, L.P. O'Mard und R. Meddis beschrieben ist (J. Acoust. Soc. Am., Vol. 111, No. 5, Pt. 1, May 2002) beschrieben ist.

Es wird allerdings ferner darauf hingewiesen, dass auch andere Modelle für eine Berechnung eines Neurotransmitter-Vesikel-Auftretens aufgrund einer Auslenkung u(t) eines Stereoziliums verwendet werden können. Allerdings hat es sich gezeigt, dass es besonders empfehlenswert ist, ein Modell zu verwenden, das eine stochastische Freisetzung von Neurotransmitter-Vesikeln berücksichtigt, da eine solche stochastische Freisetzung ein zu berechnendes Nervenaktivitätsmuster deutlich besser beschreibt als Modelle, die von einer kontinuierlichen bzw. nichtgequantelten Freisetzung von Neurotransmitter-Vesikeln ausgehen. Wie schon erwähnt kann im übrigen durch eine Auswertung der Neurotransmitter-Vesikel-Freisetzung eine genauere Information über ein Audiosignal erhalten werden als bei Verwendung kontinuierlicher Freisetzungs-Modelle, bei denen keine scharf definierten zeitlichen Übergänge stattfinden.

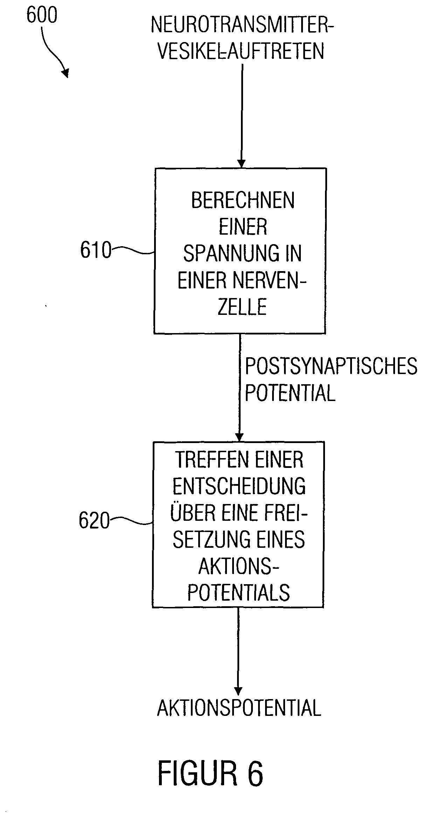

Fig. 6 zeigt ein Blockschaltbild eines erfindungsgemäßen Verfahrens zum Berechnen eines Aktionspotenzials einer Nervenfaser aufgrund eines Neurotransmitter-Vesikel-Auftretens gemäß dem ersten Ausführungsbeispiel der vorliegenden Erfindung. Das Flussdiagramm der Fig. 6 ist in seiner Gesamtheit mit 600 bezeichnet.

Bei dem gezeigten Verfahren zum Berechnen eines Aktionspotenzials aufgrund eines Neurotransmitter-Vesikel-Auftretens wird in einem ersten Schritt 610 eine Spannung in einer Nervenzelle aufgrund des Neurotransmitter-Vesikel-Auftretens berechnet. Bei dieser Spannung handelt es sich beispielsweise um ein post-synaptisches Potenzial bzw. eine Depolarisation der post-synaptischen Nervenzelle. Für eine Berechnung der Spannung in der Nervenzelle kann beispielsweise eine Diffusion von Neurotransmittern berücksichtigt werden. Mit anderen Worten, eine durchschnittliche Neurotransmitter-Konzentration kann durch Auswertung eines Faltungsintegrals bestimmt werden, wobei in dem Faltungsintegral ein Diffusions-Typ-Kern ausgewertet werden kann. Eine solche Berücksichtigung des Diffusionsverhaltens wird beispielsweise in dem Artikel "Estimating Transmitter Release Rates from Postsynaptic Current Fluctuations" von Erwin Neher und Takeshi Sakaba (The Journal of Neuroscience, December 15, 2001, 21(24): 9638-9654) beschrieben. Bei der Berechnung des post-synaptischen Potenzials können ferner eine Kapazität der Zellmembran sowie Membranpotenzial-abhängige Leitfähigkeiten von Membran-Ionenkanälen berücksichtigt werden. In ein entsprechendes Modell zur Berechnung einer Spannung in der Nervenzelle fließt somit ein Ionenaustausch durch eine Zellmembran sowie eine Anregung durch die Neurotransmitter ein.

Aufgrund des in dem ersten Schritt 610 berechneten post-synaptischen Potenzials kann dann in einem zweiten Schritt 620 eine Entscheidung über eine Freisetzung eines Aktionspotenzials getroffen werden. Hierbei wird davon ausgegangen, dass sich durch die Freisetzung der Neurotransmitter das post-synaptische Potenzial erhöht.

Zusammenfassend lässt sich also festhalten: Die inneren Haarzellen sind die Verbindung zwischen der Basilarmembran und den auditiven Nervenfasern (ANF), die Impulse in Richtung Gehirn senden. Bevor jedoch eine am postsynaptischen Ende befindliche ANF ein Aktionspotenzial in Richtung Gehirn senden kann muss sich Ihre Spannung ausreichend erhöhen und einen Schwellenwert überschreiten. Dazu ist eine Verkettung von Ereignissen in der presynaptischen IHC nötig, die die mechanischen Schwingungen in elektrische Signale umwandelt.

Am oberen Ende einer IHC befinden sich Haarbündel- drei Reihen von Stereozilien - die durch sogenannte Tip-Links miteinander verbunden sind. Sie folgen der schwingenden Bewegung der Basilarmembran - gedämpft durch die Flüssigkeit, in der sie sich befinden und pendeln durch ihre Steifigkeit wieder in die Ausgangslage zurück. Die Stereozilienbewegung lässt sich als ein System gekoppelter harmonischer Oszillatoren auffassen. Vereinfacht wird sie durch folgende Gleichung beschrieben

$$\tau_c \dot{x}(t) + x(t) = \tau_c C_{cilia} v(t) \, ,$$

wobei x(t) für die Auslenkung der Stereozilien und v(t) für die Geschwindigkeit der BM steht. $\tau$c ist eine Zeitkonstante, Ccilia ein Gain-Faktor. Bei niedrigen Frequenzen bewegen sich die Stereozilien phasengleich mit der BM-Geschwindigkeit, bei hohen Frequenzen phasenverschoben.

Im Inneren der IHC Zellmembran liegt ein erheblich niedrigeres Potenzial vor als außerhalb. Dieses ist im Wesentlichen beeinflusst durch die vorliegenden Konzentrationen an (positiv geladenen) Kalium-Ionen. Es herrscht ein stetiger Ionenfluß zwischen innen und außen. Im ruhenden Zustand stellen sich Gleichgewichtskonzentrationen von [K+] = 130mmol/l (innen) sowie [K+] = 155mmol/l außen ein. Es wird davon ausgegangen, dass auch im Ruhezustand etwa 15% der, an den Stereozilienenden befindlichen Ionenkanäle geöffnet sind. Bewegen sich die Stereozilien nun in medialer Richtung führt das zu einer Öffnung von zusätzlichen Kanälen. Die Leitfähigkeit zwischen Innerem und Äußerem der IHC wird erhöht. Dadurch fließen vermehrt postiv geladene Kaliumionen von außen nach innen und die Spannung im Inneren der IHC steigt an. Andersherum hemmt eine Auslenkung der Stereozilien in entgegengesetzter (lateraler) Richtung den Ionenflusses und führt damit zu einem Absinken des Membranpotenzials.

Die Stereozilien reagieren hochgradig sensibel auf Bewegung. Bereits sinusförmige Auslenkung um +/-0.003 Grad wird als hörbares Signal wahrgenommen. Aber auch ohne externe Anregung durch die schwingende Basilarmembran bewegen sich die Stereozilien. Sie unterliegen Brown'scher Bewegung, so dass auch ganz ohne eingehende Schallwelle - je nach Art der Nervenfaser - bis zu über 100 Aktionspotenziale je Sekunde emittiert werden.

Depolarisierung der Haarzelle öffnet spannungsabhängige Calciumkanäle in der Zellmembran, die in der Nähe sogenannter aktiver Zonen liegen. Das sind Regionen am afferenten Ende des synaptischen Spalts, in denen sich Neurotransmitter befinden. Von außen können so verstärkt Ca++-Ionen in die IHC eintreten. Dieser Zustrom von Calcium-Ionen führt zur Freisetzung von Neurotransmittern. Eine hohe Geschwindigkeit bei der Signalübertragung wird dadurch gewährleistet dass die Botenstoffe bereits in sogenannten Vesikeln etwa gleicher Anzahl von einigen 1000 Neurotransmittermolekülen "verpackt" und abrufbereit sind. Modelliert werden kann dies durch sogenannte Pools von verfügbaren Vesikeln.

Die Freisetzung der Transmittermoleküle in den synaptischen Spalt geschieht, in dem das Vesikel an dafür vorgesehenen Stellen der pre-synaptischen Membran, den Aktiven Zonen, diffundiert, sich mit der Membran verbindet, und seinen Inhalt - die Neurotransmitter - in den Spalt freigibt. Leert sich der Pool zur Verfügung stehender Vesikel, so wird er nach und nach wieder aufgefüllt. Ferner empfiehlt sich die Verwendung eines Reprocessing Pool, in dem Transmitter aus dem Spalt mit einer gewissen Rate wieder zu Vesikeln verpackt werden und von dort aus direkt wieder in den freien Pool gelangen. Ein Teil der Transmitter geht im synaptischen Spalt verloren.

Die Ausschüttung von Neurotransmittern aus dem Pool von Vesikeln wird im Allgemeinen als binomialverteilt beschrieben. Die Emissionswahrscheinlichkeit ist dabei von der Calciumkonzentration im inneren der IHC-Zellmembran in der Nähe der aktiven Zonen abhängig.

Die ursprüngliche Modellierung des Spannnungsverlaufs in Nervenzellen geht auf Hodgkin und Huxley aus dem Jahr 1952 zurück. Dabei sind im Wesentlichen der Austausch von Kalium- [K] und Natrium-Ionen [Na] sowie ein spezifisches "Leck" [L] (im Wesentlichen von CL⁻-Ionen) von Bedeutung, die sich durch unterschiedliche maximale Leitfähigkeiten (gNa, gK, gL) und den zeitlichen Verlauf der Membrandurchlässigkeit, in Abhängigkeit vom vorliegenden Potenzial (modelliert durch die von u(t) abhängigen Variablen m, h, n) auszeichnen. Die Membranspannung u(t) verhält sich gemäß

$$u'C = -\Sigma I_k(t) + I(t)$$

$$\Sigma Ik = (u-VNa)m^3hgNa+(u-VK)n^4gK +(u-VL)gL$$

wobei u' die Ableitung nach der Zeit bezeichnet. VNa, VK und VL sind Nernst-Gleichgewichtspotenziale, die die Richtung des Ionenaustauschs determinieren. Ik(t) stellt den Ionenaustausch durch die Zellmembran dar, I(t) dagegen ist ein externer Strom - hier z.B. durch die von den IHC emittierten Neurotransmitter - und C der Kondensator, den die Zellmembran bildet. Wenn ein externer Strom in die Zelle strömt, lädt sich der Kondensator und es entsteht Leck durch die Membran-Ionenkanäle. Das zeitliche Verhalten der Leitfähigkeiten in Abhängigkeit vom Membranpotenzial wird durch ein System dreier Differentialgleichungen beschrieben:

$$m' = \alpha m(u)(1 - m) - \beta m(u)m,$$

$$n' = \alpha n(u)(1 - n) - \beta m(u)n,$$

$$h' = \alpha h(u)(1 - h) - \beta h(u)h.$$

Die Funktionen $\alpha i(u)$ und $\beta i(u)$ in Abhängigkeit von der Spannung u sind von Hodgkin und Huxley für i = {m, n, h} angepasst worden. Es ergeben sich unterschiedlich schnelle Reaktionen der verschiedenen Ionenflüsse auf eine externe Spannungsänderung, die den Spannungsverlauf in der Membran charakterisieren. Diffundiert nun ein Vesikel aus der presynaptischen IHC in den Spalt, so bindet es an einem Rezeptorprotein der post-synaptischen Membran und setzt Ladung frei. Durch die Moleküle eines Vesikels erhöht sich das post-synaptische Potenzial um ein so bezeichnetes mEPP (miniature end-plate potenzial) von etwa 0.5- 1 mV.

Überschreitet die Depolarisation der post-synaptischen Nervenzelle, die durch das post-synaptische Potential beschrieben wird, einen bestimmten Schwellwert v, so kommt es beispielsweise zur Freisetzung eines Aktionspotentials.

Aktionspotenziale kennzeichnen sich durch ihren stets nahezu identischen zeitlichen Verlauf. Die Membranspannung depolarisiert zunächst für eine sehr kurze Dauer von weniger als einer Millisekunde extrem stark, danach hyperpolarisiert sie und ist für einen Zeitraum blockiert, in dem keine weiteren Aktionspotenziale auftreten können.

Das Verhalten einer auditiven Nervenfaser i lässt sich durch die Zeitpunkte gefeuerter Spikes

$$T = \{ \overset{k}{\underset{i}{}} \mid 1 \le k <= ni \} = \{t \mid u_i(t) = v\}$$ beschreiben, wobei $u_i(t)$ die Spannung der postsynaptischen Membran i und v der zur AP-Auslösung nötige Schwellenwert sind. Im allgemeinen wird $v = v(t)$ als dynamisch angenommen, da Neuronen unmittelbar nach Emission eines Spikes eine kurze Weile blockiert sind. Diese Spikeunterdrückung wird in der Regel modelliert mit Hilfe einer kurzen absoluten Refraktärzeit $\tau_{abs}$ von in etwa 0.8 ms gefolgt von einem anschließenden exponentiellen Abklingen mit einer Zeitkonstante $\tau$ von etwa 0.25 ms, so dass die Schwellwertspannung modelliert werden kann durch

$$v(t) = v_O + \Sigma \eta o e^{\left(\frac{-(t-t_i^{(k)}-\tau_{abs})}{\tau}\right)} * I_{[\tau_{abs}.\infty]}\left(t - t_i^{(k)}\right),$$

wenn das Neuron sich nicht im absolut refraktären Zustand befindet und $v(t) = \infty$, falls $t - t(k)_i^{(k)} \in [0, \tau_{abs}]$ für ein $k \in \{1,...,ni\}$, mit $I(.)$ als Indikatorfunktion. Aus Recheneffizenzgründen wird die Summe meist auf den Einfluss der letzten 1 oder 2 Spikes vereinfacht.

Mit anderen Worten, ein Aktionspotenzial wird ausgelöst, wenn das post-synaptische Potenzial den vorgegebenen Schwellenwert überschreitet. Dieser Schwellenwert kann zeitlich variieren. Im Übrigen wird darauf hingewiesen, dass bei einer Erzeugung eines Aktionspotenzials bevorzugterweise sowohl eine absolute Refraktärzeit, d.h. eine Totzeit, während der eine Auslösung eines weiteren Aktionspotenzials nicht mehr möglich ist, als auch eine relative Refraktärzeit, während der eine Schwelle für eine Auslösung eines Aktionspotenzials höher ist als in einem Ruhezustand, berücksichtigt werden können. Die absolute Refraktärzeit kann dabei beispielsweise durch einen unendlich hohen Schwellwert $v$ modelliert werden, aber auch anderweitig einbezogen werden. Die relative Refraktärzeit wird bevorzugt durch einen zeitlich variablen Schwellenwert $v$ für die Auslösung eines Aktionspotenzials beschrieben.

Somit kann gewährleistet werden, dass Totzeiten der Nervenfasern bei einer Erzeugung von Aktionspotenzialen bzw. einer Erzeugung des Nervenaktivitätsmusters, das Aktionspotenziale auf einer Mehrzahl von Nervenfasern beschreibt, berücksichtigt werden. Dadurch kann die Informationsmenge des Nervenaktivitätsmusters verringert werden, was eine weitere Verarbeitung des Nervenaktivitätsmusters und eine Speicherung des Nervenaktivitätsmusters erleichtert. Im Übringen kann durch eine beschriebene Anordnung auch eine spontane Aktivität der Nervenfasern mit berücksichtigt werden.

Weiterhin sei darauf hingewiesen, dass während der absoluten Refraktärzeit auch das zeitlich überlappte Auftreten einer Mehrzahl von Neurotransmitter-Vesikeln nicht zu einer Auslösung eines Aktionspotenzials führt. In der relativen Refraktärzeit hingegen löst zwar das Auftreten eines einzigen Neurotransmitter-Vesikels noch kein Aktionspotenzial aus, während hingegen ein überlappendes Auftreten mehrerer Neurotransmitter-Versikel in der relativen Refraktärzeit durchaus in einer Auslösung eines Aktionspotenzials resultieren kann.

Gemäß dem anhand der Fig. 1 bis 6 beschriebenen Verfahren kann also eine besonders aussagekräftige Analysedarstellung eines Audiosignals erzeugt werden, die ein Nervenaktivitätsmuster umfasst. In der erfindungsgemäßen Weise wird also das Nervenaktivitätsmuster basierend auf einem Neurotransmitter-Vesikel-Auftreten berechnet, wobei die stochastische Natur des Neurotransmitter-Vesikel-Auftretens berücksichtigt werden kann. Im Übrigen können auch bei der Bestimmung von Aktionspotenzialen auf den Nervenfasern alle relevanten Effekte (wie beispielsweise die Diffusion von Neurotransmittern und die Potenzialabhängigkeit bei der Auslösung eines Aktionspotenzials auf einer Nervenfaser) berücksichtigt werden. Dadurch weist das in der erfindungsgemäßen Weise berechnete Nervenaktivitätsmuster eine besonders hohe zeitliche Genauigkeit auf, die für eine aussagekräftige Analyse benötigt wird. Anhand eines erfindungsgemäß bestimmten Nervenaktivitätsmuster kann nämlich beispielsweise auch eine Phasenbeziehung zwischen den Aktionspotenzialen benachbarter Nervenzellen ausgewertet werden, die herkömmlicherweise aufgrund einer zu unpräzisen Modellierung nur wenig aussagekräftig ist.

Ferner wird darauf hingewiesen, dass es vorteilhaft ist, Neurotransmitter-Vesikel 1-zu-1 in elektrische Impulse einer Höhe von 1mV umzusetzen. Eine berechnete Freisetzung eines Neurotransmitter-Vesikels kann beispielsweise verwendet werden, um in einer Cochlea-Implantat-Ansteuervorrichtung einen Rechteck-Impuls zu erzeugen die entstehenden elektrischen Impulse'dann einem Cochlea-Implantat zuzuführen. Durch die Impulse können dann Hörnervenfasern getriggert werden. Hierbei werden beispielsweise 251 Nervensignale für 251 Hörnerven auf die in einem Cochlea-Implantat typischerweise verfügbaren 22 Kanäle skaliert.

Weiterhin wird es bevorzugt, das Nervenaktivitätsmuster weiter zu verarbeiten, um eine noch vorteilhaftere Analysedarstellung des Audiosignals zu erhalten. Die Berechnung des Nervenaktivitätsmusters kann hierbei vorteilhafter Weise nach dem oben beschriebenen Verfahren erfolgen, jedoch sind auch andere Verfahren, die ein Nervenaktivitätsmuster liefern, für die Gewinnung einer verbesserten Analysedarstellung verwendbar.

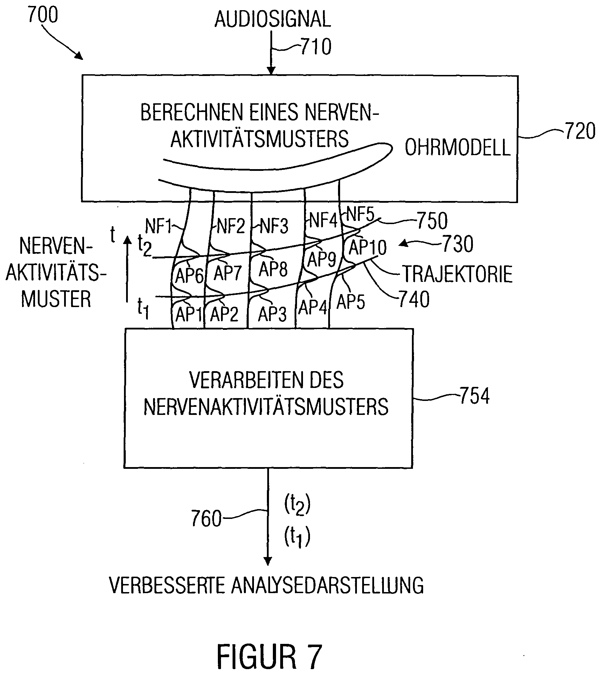

Fig. 7 zeigt ein Flussdiagramm eines erfindungsgemäßen Verfahrens zur Berechnung einer verbesserten Analysedarstellung eines Audiosignals gemäß einem weiteren Ausführungsbeispiel der vorliegenden Erfindung. Das Flussdiagramm der Fig. 7 ist in seiner Gesamtheit mit 700 bezeichnet. Dabei wird basierend auf einem Audiosignal 710 in einem ersten Schritt 720 ein Nervenaktivitätsmuster 730 berechnet. Bei der Ermittlung des Nervenaktivitätsmusters 730 in dem ersten Schritt 720 kann beispielsweise ein Ohrmodell verwendet werden, wie dies schon oben ausführlich erläutert wurde. Ergebnis der Berechnung des Nervenaktivitätsmusters 730 sind somit zeitliche Verläufe von Aktionspotenzialen auf einer Mehrzahl von Nervenfasern NF1, NF2, NF3, NF4, NF5.

Es wird hierbei darauf hingewiesen, dass das Nervenaktivitätsmuster 730 als eine Antwort auf das Audiosignal 710 charakteristische Trajektorien 740, 750 aufweist. Mit anderen Worten ausgedrückt: tritt ein akustisches Ereignis in dem Audiosignal 710 auf, so resultiert dieses akustische Ereignis in einer Serie von Aktionspotenzialen Ap1, AP2, AP3, AP4, AP5, die mit leichtem zeitlichem Versatz auf einer Mehrzahl von Nervenfasern NF1, NF2, NF3, NF4, NF5 auftreten. Eine Trajektorie 740, 750 umfasst somit eine Mehrzahl von Aktionspotenzialen AP1, AP2, AP3, AP4, AP5 bzw. Spitzen auf mehreren verschiedenen Nervenfasern NF1, NF2, NF3, NF4, NF5 ("pulse spiking trains").

Eine Trajektorie 740, 750 ist u.a. dadurch gekennzeichnet, dass sie in einer zweidimensionalen Darstellung des Nervenaktivitätsmusters 730 über der Zeit als eine Linie erkennbar ist. Die Trajektorie 740, 750 kann hierbei entweder gerade oder gekrümmt sein, wobei verschiedene Arten der Krümmung (z.B. parabolisch oder hyperbelförmig) auftreten können. Im Übrigen ist zu erwähnen, dass es in manchen Fällen auch möglich ist, eine Trajektorie 740, 750 durch einen Ausschnitt aus einer Sinusfunktion anzunähern. Ferner ist festzuhalten, dass eine Trajektorie 740, 750 definitionsgemäß eine Mindestlänge aufweist. Eine Trajektorie 740, 750 liegt also nur dann vor, wenn Aktionspotenziale AP1, AP2, AP3, AP4, AP5 auf einer Mindestanzahl von Nervenfasern auftreten. Dabei bilden dann die Aktionspotentiale in einer zweidimensionalen Darstellung des Nervenaktivitätsmusters als Funktion der Zeit ein Liniensegment.

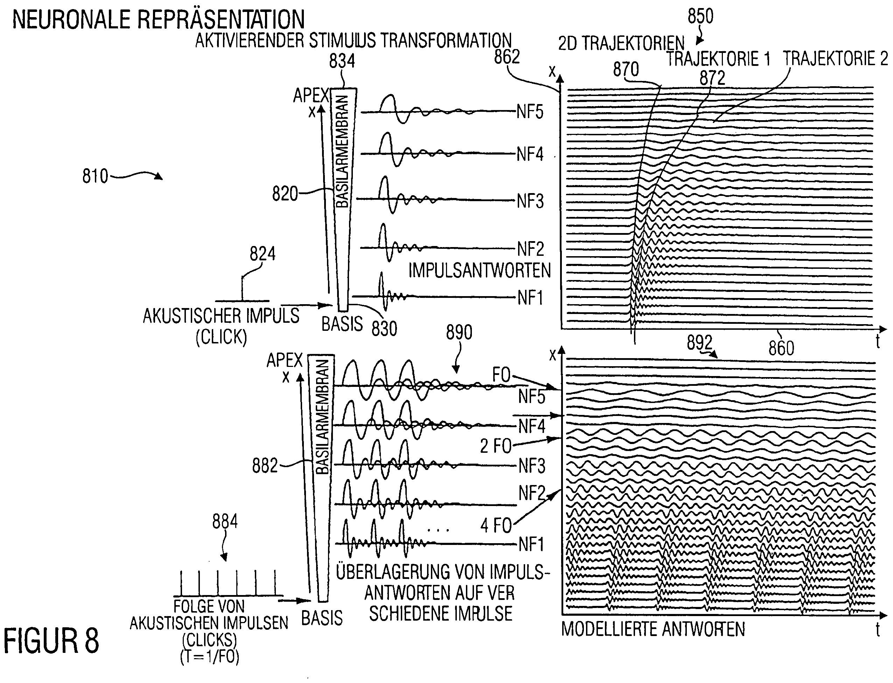

Im Übrigen ist festzuhalten, dass eine zweidimensionale Darstellung eines Nervenaktivitätsmusters 730 als Funktion der Zeit hier eine Darstellung beschreibt, in der die Aktionspotenziale auf mehreren Nervenfasern parallel als Funktion der Zeit angetragen sind, wobei die Zeit in Richtung einer Abszisse angetragen ist, und wobei Verläufe der Aktionspotenziale auf mehreren Nervenfasern durch Linienzüge, die im Wesentlichen parallel zu der Abszisse verlaufen, angetragen sind (vgl. auch Fig. 8). Eine Trajektorie 740, 750 liegt somit vor, wenn zusammengehörige Maxima oder Minima der Aktionspotenziale AP1, AP2, AP3, AP4, AP5 in der zweidimensionalen Darstellung des Nervenaktivitätsmusters 730 durch eine gerade oder gebogene Linie (die bevorzugterweise glatt bzw. knickfrei ist) verbunden werden können. Eine Trajektorie kann somit als eine Verbindungslinie von zusammengehörigen Maxima bzw. Minima von Aktionspotenzialen benachbarter Nervenfasern angesehen werden.

Das in dem Flussdiagramm 700 gezeigte erfindungsgemäße Verfahren umfasst in einem zweiten Schritt 754 das Verarbeiten des Nervenaktivitätsmusters, das in dem ersten Schritt 720 gebildet wird. Dabei wird das Nervenaktivitätsmuster 730 bevorzugt in einer zweidimensionalen Darstellung als Funktion der Zeit und als Funktion der Position der Nervenfasern betrachtet. In dem zweiten Schritt 754 wird somit das Nervenaktivitätsmuster 730 verarbeitet, um als Analysedarstellung eine Folge von Zeitinformationen t1, t2 zu erhalten, die die zeitliche Lage von aufeinander folgenden Trajektorien 740, 750 beschreiben. Dabei wird davon ausgegangen, dass eine Trajektorie 740, 750 Aktivitätsimpulse AP1, AP2, AP3, AP4, AP5 auf verschiedenen Nervenfasern NF1, NF2, NF3, NF4, NF5 aufgrund des gleichen Ereignisses in dem Audiosignal umfasst bzw. verbindet. Die ermittelten Zeitinformationen t1, t2 beschreiben hierbei den Zeitpunkt des Auftretens der Trajektorien 740, 750. Es wird ferner darauf hingewiesen, dass eine Trajektorie 740, 750 nur dann erkannt wird, wenn eine Mindestanzahl an Nervenfasern NF1, NF2, NF3, NF4, NF5 ein Aktionspotenzial AP1, AP2, AP3, AP4, AP5 aufweisen, wodurch verhindert wird, dass einzelne Aktivitätspotenziale in dem Nervenaktivitätsmuster fälschlich als Trajektorie identifiziert bzw. erfasst werden.

Ein akustisches Ereignis, das eine Trajektorie mit sich bringt, kann beispielsweise ein Beginn eines Lauts sein. Auch Vokale erzeugen mindestens eine charakteristische Trajektorie, die bei dem erfindungsgemäßen Verfahren erkannt werden kann, und der daraufhin eine Zeitinformation zugeordnet werden kann. Üblicherweise umfasst allerdings ein Laut sogar mehrere Trajektorien, wobei bevorzugterweise für jede der aufeinander folgenden Trajektorien eine zugehörige Zeitinformation erzeugt werden kann. Die Zeitinformation kann dabei beispielsweise einen Anfangszeitpunkt der Trajektorie kennzeichnen, also einen Zeitpunkt, an dem die erste zu der Trajektorie 740, 750 gehörige Nervenfaser NF1, NF2, NF3, NF4, NF5 ein Aktionspotenzial AP1, AP2, AP3, AP4, AP5 aufweist.

Die Verarbeitung des Nervenaktivitätsmusters 730 kann beispielsweise so ausgelegt werden, dass solche Muster in dem Nervenaktivitätsmuster als Trajektorie erkannt werden, die eine Wanderwelle auf einer Basilarmembran eines Ohrmodells zugeordnet sind. Mit anderen Worten, eine Trajektorie kann dann erkannt werden, wenn auf den einzelnen Nervenfasern ein Anregungsmuster anliegt, das auf einer Ausbreitung einer Wanderwelle basiert. Eine Wanderwelle löst dabei typischerweise nacheinander und mit einem geringen aber nicht verschwindenden zeitlichen Versatz Aktionspotenziale auf der Mehrzahl von Nervenfasern aus.

Weiterhin ist bei obigen Überlegungen zu berücksichtigen, dass üblicherweise Nervenfasern, die gemäß des Ohrmodells bevorzugt auf niedrige Frequenzen ansprechen, erst später aktiviert werden bzw. ein Aktionspotenzial aufweisen, als Nervenfasern, die bevorzugt hohe Frequenzen detektieren. Dies liegt darin begründet, dass Hörnerven, die auf hohe Frequenzen ansprechen, an dem Anfang der Cochlea bzw. der Basilarmembran angeordnet sind, während hingegen Hörnerven, die niedrige Frequenzen erfassen, an dem Ende der Cochlea angeordnet sind.

Mit der Ausbreitung eines Schallereignisses über die Cochlea bzw. über die Basilarmembran ergibt sich hierbei eine frequenzabhängige Verzögerung, sodass hohe Frequenzanteile früher als tiefe Frequenzanteile ein Aktivitätspotential auf den jeweils zugeordneten Nervenfasern erzeugen.

So zeigt auch die Darstellung der Figur 7 Aktionspotentiale AP1, AP2, AP3, AP4, AP5 auf eine Mehrzahl von Nervenfasern NF 1, NF 2, NF 3, NF 4, NF 5. Es wird hierbei davon ausgegangen, dass eine erste Nervenfaser NF 1 einer hohen Frequenz zugeordnet ist (z. B. 20 kHz), während eine fünfte Nervenfaser (NF 5) einer niedrigeren Frequenz (z. B. 20 Hz) zugeordnet ist. Eine dazwischenliegende zweite Nervenfaser NF 2, eine dritte Nervenfaser NF 3 und eine vierte Nervenfaser NF 4 sind dazwischenliegende Frequenzen zugeordnet.

Zum besseren Verständnis wird hier auch auf eine Zeitachse t hingewiesen. Es ist erkennbar, dass das Aktionspotential AP1 auf der ersten Nervenfaser NF 1 früher auftritt als Aktionspotentiale auf den übrigen Nervenfasern NF 2, NF 3, NF 4 und NF 5. Die zusammengehörigen Aktionspotentiale auf den fünf Nervenfasern bilden somit bei einer zeitlichen Betrachtung eine erste Trajektorie 740, der ein Anfangszeitpunkt t1 zugeordnet werden kann. Ein später folgendes zweites akustisches Ereignis erzeugt wiederum Aktionspotentiale AP6, AP7, AP8, AP9, AP10 auf den Nervenfasern NF1, NF2, NF3, NF4, NF5 , die die zweite Trajektorie 750 bilden. Der zweite Trajektorie 750 kann dann ein zweiter Zeitpunkt t2 zugeordnet werden.

Aufgabe der Verarbeitung des Nervenaktivitätsmusters in dem zweiten Schritt 730 ist es nunmehr, die ersten Trajektorie 740 und die zweite Trajektorie750 zu erkennen und entsprechende Zeitinformationen (t1), (t2), die den Trajektorien 740, 750 zugeordnet sind, zu liefern. Die Zeitinformationen (t1) und (t2) bilden somit eine verbesserte Analysedarstellung des Audiosignals 710, die eine noch vorteilhaftere Verarbeitung als das Nervenaktivitätsmuster selbst ermöglicht. Die verbesserte Analysedarstellung 760 kann insbesondere auch zur Zwecke einer Spracherkennung eingesetzt werden. Daneben eignet sich die verbesserte Analysedarstellung 760 auch sehr gut für eine Rhythmuserkennung eines Signals.

Fig. 8 zeigt zum besseren Verständnis eine Darstellung von beispielhaften Nervenaktivitätsmustern. Eine erste graphische Darstellung 810 zeigt, wie eine Basilarmembran 820 durch einen akustischen Impuls 824 (CLICK) angeregt wird. Der akustische Impuls 824 wird über das ovale Fenster der Cochlea eingekoppelt und erreicht somit zuerst eine Basis 830 der Basilarmembran 820. Der akustische Impuls breitet sich dann über die Basilarmembran 820 zu deren Apex 834 aus. Entlang der Basilarmembran sind beispielsweise fünf (Hör-)Nerven angeordnet, wobei eine grafische Darstellung 840 Pulsantworten der Basilarmembran 820 bei einer Anregung durch den akustischen Impuls 824 an verschiedenen Orten der Basilarmembran zeigt. Es ist hierbei ersichtlich, dass in der Nähe der Basis 830 der Basilarmembran 820 eine kurze Impulsantwort auftritt, die im wesentlichen hohe Frequenzanteile umfasst, während hingegen in der Nähe des Apex 834 der Basilarmembran 820 eine lange Impulsantwort auftritt, die im wesentlichen niedrige Frequenzen umfasst.

Eine weitere grafische Darstellung 850 zeigt Impulsantworten für eine Vielzahl von Orten entlang der Basilarmembran. Eine Abszisse 860 beschreibt hierbei die Zeit, während eine Ordinate 862 einen Ort entlang der Basilarmembran 820 kennzeichnet. Aus der grafischen Darstellung 850 ist ersichtlich, dass die Impulsantworten an verschieden Orten entlang der Basilarmembran 820 bei einer Anregung durch den akustischen Impuls 824 eine Mehrzahl von Trajektorien aufweisen, von denen zwei Trajektorien 870, 872 beispielhaft in der grafischen Darstellung 850 eingezeichnet sind. Trajektorien, die in der grafischen Darstellung 850 der Impulsantwort der Basilarmembran gezeigt sind, können im übrigen in ähnlicher weise auch in einem Nervenaktivitätsmuster identifiziert werden, das hier nicht explizit gezeigt ist.

Es wird im übrigen auch noch darauf hingewiesen, dass die in der graphischen Darstellung 850 gezeigten Trajektorien 870, 872 ein unterschiedliches Krümmungsverhalten aufweisen. Das unterschiedliche Krümmungsverhalten resultiert aus der Tatsache, dass an verschiedenen Orten der Basilarmembran 820 verschiedene Frequenzen dominant sind (beispielsweise an der Basis der Basilarmembran hohe Frequenzen und an dem Apex der Basilarmembran niedrige Frequenzen).

Eine weitere grafische Darstellung 880 zeigt analog zu der grafischen Darstellung 810 eine Anregung einer Basilarmembran 882 durch eine Folge von akustischen Impulsen (CLICKS) die mit 884 bezeichnet ist. Entsprechend zeigt eine grafische Darstellung 890 eine Überlagerung von Impulsantworten auf verschiedene akustische Impulse. Eine grafische Darstellung 892 zeigt wiederum die entsprechende Impulsantwort für eine große Anzahl von Orten x auf der Basilarmembran 881.

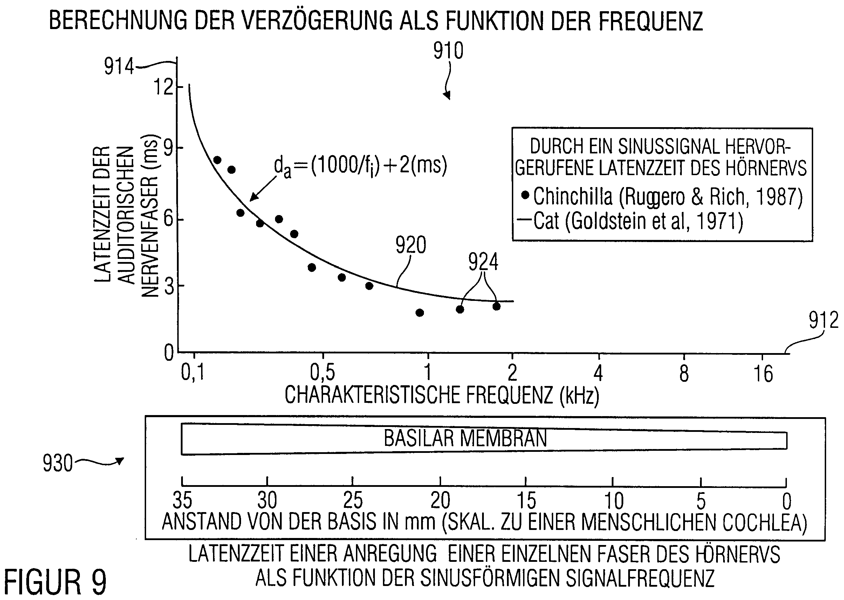

Zur weiteren Verdeutlichung zeigt die Figur 9 eine grafische Darstellung einer Verzögerung bei einer Ausbreitung von Signalen verschiedener Frequenz auf der Basilarmembran. Hierbei beschreibt eine erste grafische Darstellung 910 eine Latenzzeit von auditorischen Nervenfasern als Funktion einer charakteristischen Frequenz.

Eine Frequenz ist hierbei an einer Abszesse 912 in einem Bereich zwischen 0,1 kHz und 16 kHz angetragen. Eine Ordinate von 914 beschreibt eine Latenzzeit der auditorischen Nervenfaser in einem Bereich zwischen 0 und 12 Millisekunden. Eine Kurve 920 beschreibt einen Verlauf einer durch ein Sinussignal hervorgerufenen Latenzzeit des Hörnervs über der Frequenz für eine Katze. Messpunkte 924 beschreiben einen ähnlichen Verlauf für einen Chinchilla. Es wurde herausgefunden, dass die Latenzzeit auf auditorischen Nervenfasern als Funktion einer charakteristischen

Frequenz $f_i$ für die betreffende Nervenfaser durch die folgende Gleichung beschrieben werden kann:

$$d_a = (1000/f_i) + 2ms.$$

Der entsprechende Zusammenhang ist im Detail beispielsweise in dem Artikel "A Space-Time Theory of Pitch and Timbre Based on Cortical Expansion of the Cochelar Travelling Wave Delay" von S. Greenberg, D. Poeppel und T. Roberts, vorgestellt auf dem XIth International Symposium on Hearing, Grantham, ausführlich erläutert. Für Einzelheiten wird daher auf den entsprechenden Artikel verwiesen.

Es wird hierbei ferner darauf hingewiesen, dass eine zweite grafische Darstellung 930 einen Abstand von zu einer charakteristischen Frequenz gehörigen Hörzellen von einer Basis einer menschlichen Cochlea zeigt. Hieraus wird wiederum deutlich, dass zu hohen Frequenzen gehörige Hörzellen nahe bei der Basis der menschlichen Cochlea angeordnet sind, während zu niedrigen Frequenzen gehörige Hörzellen entfernt von der Basis der menschlichen Cochlea angeordnet sind.

Die vorliegende Darstellung bestehend aus der ersten grafischen Darstellung 910 und der zweiten grafischen Darstellung 930 zeigt allerdings auch, dass eine Latenzzeit des auditorischen Nervs für tiefe Frequenzen (unter ca. 0,5 kHz) stark ansteigt. Daraus kann gefolgert werden, dass eine Ausbreitungszeit in der Nähe des Endes der Basilarmembran (also entfernt von der Basis der Cochlea) deutlich abnimmt.

Die beschriebene Latenzzeit der auditorischen Nervenfasern resultiert, wie auch schon anhand von Figur 8 beschrieben, in einer Krümmung von Trajektorien in dem Nervenaktivitätsmuster, das Aktionspotenziale auf einer Mehrzahl von Nervenfasern, die mit Hörzellen gekoppelt sind, beschreibt.

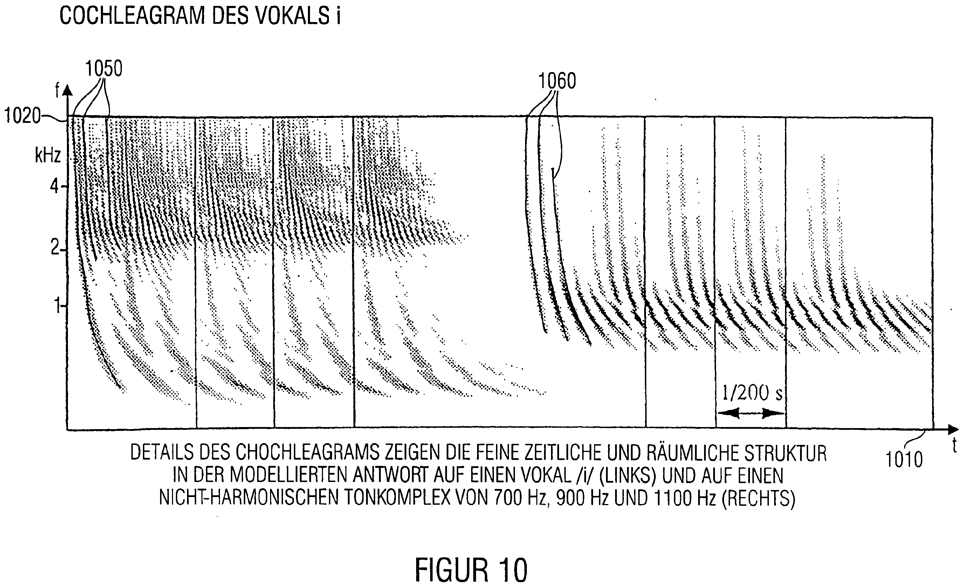

Zur weiteren Verdeutlichung zeigt die Figur 10 eine grafische Darstellung eines Cochleograms für einen Vokal "i" und für einen nicht-harmonischen Tonkomplex von 700 Hz, 900 Hz und 1100 Hz. Eine Abszisse 1010 beschreibt hierbei die Zeit, während eine Ordinate 1020 eine Frequenz beschreibt. In dem entsprechenden Cochleogram des Vokals "i" sind wiederum deutlich Trajektorien erkennbar, von denen einige ausgewählte Trajektorien gekennzeichnet und mit 1050 bezeichnet sind. In ähnlicher Weise zeigt auch das Cochleogram des nicht-harmionischen Tonkomplexes Trajektorien, die mit 1060 bezeichnet sind.

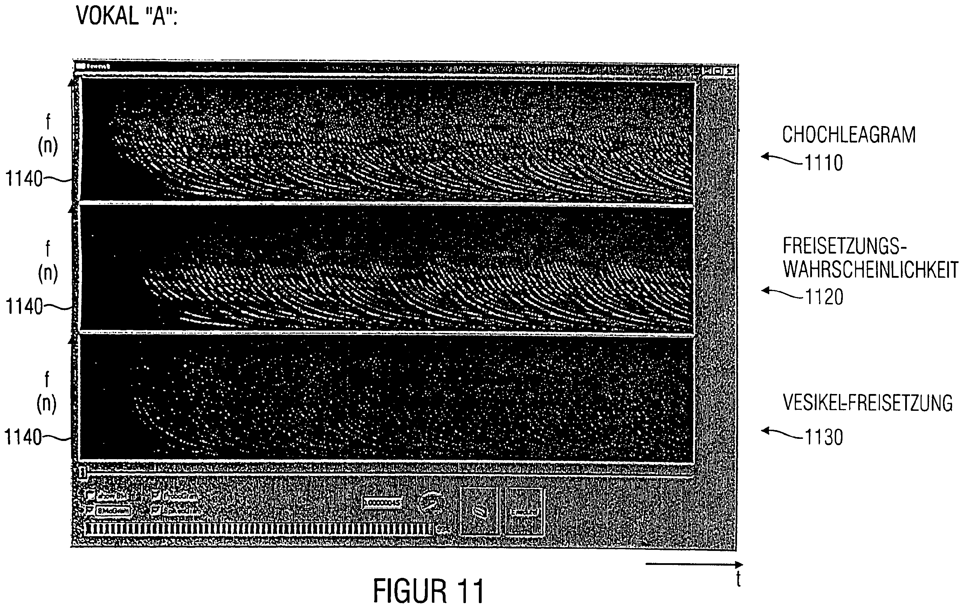

Figur 11 zeigt eine weitere grafische Darstellung eines Cochleograms sowie eine Transmitter-Freisetzungs-Wahrscheinlichkeit und eine Transmitter-Vesikel-Freisetzung für einen Vokal "A". Das Cochleogram ist hierbei in einer ersten grafischen Darstellung 1110 gezeigt. Eine zweite grafische Darstellung 1120 beschreibt eine auf dem Cochleogram basierende Transmitter-Freisetzungs-Wahrscheinlichkeit. Es wird hierbei darauf hingewiesen, dass das Cochleogram eine Anregung der Basilarmembran über der Zeit und Frequenz beschreibt. Basierend darauf, kann, wie schon oben beschrieben, eine Freisetzungs-Wahrscheinlichkeit für Neurotransmitter-Vesikel durch Analyse der mechanischen, chemischen und elektrischen Abläufe in einer Hörzelle berechnet werden. Die Freisetzungs-Wahrscheinlichkeit wurde beispielsweise als k(t) bezeichnet. Basierend auf der Freisetzungswahrscheinlichkeit k(t) kann dann eine Neurotransmitter-Vesikel-Freisetzung durch eine stochastische Auswertung berechnet werden. Ein Beispiel für eine entstehende Neurotransmitter-Vesikel-Freisetzung ist in der dritten grafischen Darstellung 1130 gezeigt. Es ist hierbei ersichtlich, dass auch die Vesikel-Freisetzung über Zeit und Frequenz charakteristische Trajektorien aufweist. Diese Trajektorien werden dann durch eine Modellierung des synaptischen Spalts auf Trajektorien von Aktionspotenzialen (also Trajektorien in dem Nervenaktivitätsmuster) abgebildet.

Es wird hierbei schließlich darauf hingewiesen, dass die an Ordinaten 1140 eingetragene Frequenz jeweils zugehörigen Hörzellen bzw. Nervenfasern (n) zugeordnet werden kann. Somit treten die gezeigten Trajektorien in sehr ähnlicher Form auch in dem Nervenaktivitätsmuster auf.

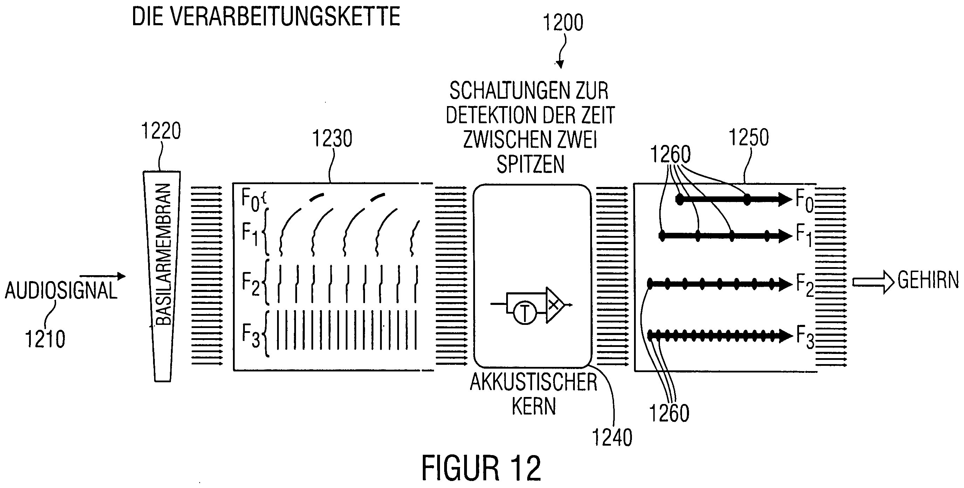

Figur 12 zeigt eine grafische Darstellung einer Verarbeitungskette für eine erfindungsgemäße Analyse eines Audiosignals gemäß dem zweiten Ausführungsbeispiel der vorliegenden Erfindung. Die grafische Darstellung der Figur 12 ist in der Gesamtheit mit 1200 bezeichnet.

Gemäß der Fig. 12 wird aufgrund eines Audiosignals 1210 die Bewegung einer Basilarmembran 1220 an einer Mehrzahl von Punkten (bzw. Orten oder Regionen) berechnet. Ein Anregungsmuster der Basilarmembran eine Abhängigkeit von einer Position entlang der Basilarmembran wird dann entweder direkt einer Einrichtung zur Erkennung von Trajektorien zugeführt, oder es wird basierend auf dem Anregungsmuster der Basilarmembran ein Nervenaktivitätsmuster erzeugt, dass für eine weitere Verarbeitung herangezogen wird. Das Anregungsmuster der Basilarmembran weist, ebenso wie ein daraus abgeleitetes Nervenaktivitätsmuster, bei einer Betrachtung über der Zeit eine Mehrzahl von Trajektorien auf, die charakteristisch für ein Audiosignal sind. Die Trajektorien sind dabei aufgrund der anhand der Figuren 8 und 9 beschriebenen Laufzeitunterschiede zwischen hohen und niedrigen Frequenzen typischerweise gebogen. Im übrigen ist anzumerken, dass die Trajektorien beispielsweise auch unterbrochen sein können. Ferner können in einzelnen Frequenzbereichen bzw. räumlichen Bereichen der Basilarmembran unterschiedliche Anregungsmuster und Trajektorien auftritt. Unterbrochene Trajektorien bzw. Teil-Trajektorien, die nur in einem bestimmten Frequenzbereich angeregt werden, sind beispielsweise für Sprachsignale charakteristisch.

Das gezeigte Nervenaktivitätsmuster 1230 bzw. ein Basilarmembran-Anregungsmuster, das dem Nervenaktivitätsmuster ähnlich ist, wird daraufhin einem akustischen Kern 1240 zugeführt. Der akustische Kern ist ausgelegt, um eine Zeit zwischen zwei Spitzen zu detektieren. Im übrigen wird darauf hingewiesen, dass es die Aufgabe des akustischen Kerns ist, Trajektorien in dem Nervenaktivitätsmuster (bzw. Basilarmembran-Anregungsmuster) zu identifizieren. Der akustische Kern kann ausgelegt sein, um den gesamten Frequenzbereich, also alle Nervenfasern, die das Nervenaktivitätsmuster bilden, oder nur einen ausgewählten Teilbereich zu analysieren. Ferner ist es auch möglich, verschiedene Kombinationen von Teilbereichen sowie den gesamten Frequenzbereich parallel zu analysieren.

Der akustische Kern 1240 ist ausgelegt, um eine Trajektorie zu erkennen, wenn eine Mindestanzahl von betrachteten Nervenfasern ein Aktivitätsmuster 1230 aufweist, was in einer Zeit-Nervenfaser-Nummer-Darstellung einem linienförmigen Verlauf in Form einer typischerweise gekrümmten Linie entspricht. Der akustische Kern 1240 liefert dann als Ausgangssignal eine Folge von Zeitinformationen, die eine zeitliche Lage der Trajektorien beschreiben. Dies kann beispielsweise der Anfangszeitpunkt einer Trajektorie oder auch ein mittlerer Zeitpunkt innerhalb einer Trajektorie sein. Im übrigen wird darauf hingewiesen, dass es bevorzugt wird, eine parallele Auswertung für eine Mehrzahl von Frequenzbändern bzw. für eine Mehrzahl von Gruppen von Nervenfasern durchzuführen, um die Teilinformationen zu einer Mehrzahl von Frequenzbändern für eine weitere Verarbeitung, z. B. eine Spracherkennung, zur Verfügung zu stellen. Ein beispielhaftes Ausgangssignals eines akustischen Kerns 1240 ist in der grafischen Darstellung 1250 gezeigt. Das Ausgangssignal des akustischen Kerns 1240 stellt hierbei ein verbessertes Analysesignal dar, in dem die Datenmenge gegenüber dem Nervenaktivitätsmuster 1230 deutlich verringert ist. Das Ausgangssignal 1250 des akustischen Kerns beschreibt nämlich nur diskrete Zeitpunkte für ein oder mehrere Frequenzbänder, die Trajektorien zugeordnet sind. Die diskreten Zeitpunkte sind hier mit 1260 bezeichnet. Die nach Frequenzbändern aufgeteilten Informationen, die die zeitliche Lage von Trajektorien beschreiben, eignen sich im übrigen besonders gut für eine Spracherkennung.

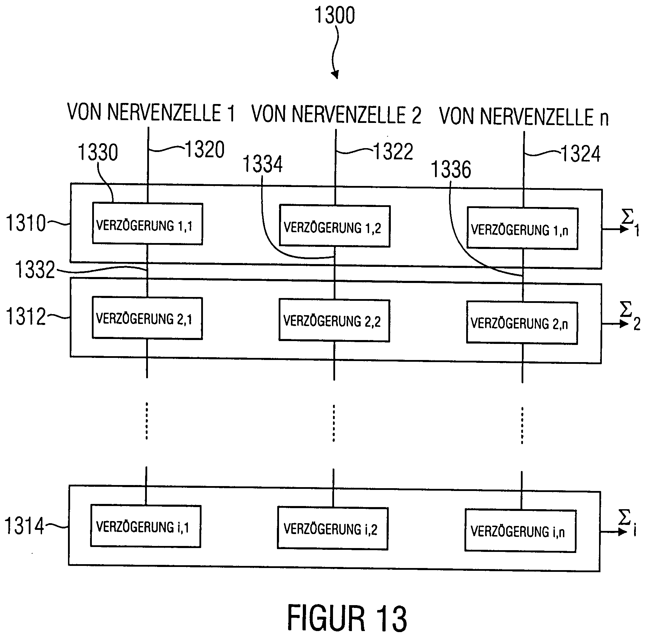

Figur 13 zeigt ein Blockschaltbild einer Vorrichtung zum erfindungsgemäßen Verarbeiten des Nervenaktivitätsmusters. Die in der Figur 13 gezeigten Vorrichtung ist in ihrer Gesamtheit mit 1300 bezeichnet. Die gezeigte Vorrichtung 1300 weist eine Mehrzahl von Stufen 1310, 1312, 1314 auf, wobei die erste Stufe 1310 parallel Signal 1320, 1322, 1324 von Nervenzellen empfängt. Die Signale 1320, 1322, 1324 beschreiben bevorzugt Aktionspotentiale auf Nervenfasern, die mit den entsprechenden Nervenzellen gekoppelt sind, und beschreiben somit das Nervenaktivitätsmuster.

In einer ersten Stufe 1310 wird dann beispielsweise das erste Nervensignal 1320 in einer ersten Verzögerungseinrichtung 1330 einer Verzögerung unterworfen und dann als verzögertes Nervensignal 1332 an eine zweite Stufe 1312 weitergeleitet. In ähnlicher weise wird auch das zweite Nervensignal 1322 in der ersten Stufe 1310 verzögert und als verzögertes Nervensignal an die zweite Stufe 1312 weitergeleitet. In der gleichen Weise werden auch die übrigen Nervensignale in der ersten Stufe 1310 verarbeitet (also beispielsweise auch das n-te Nervensignal 1324).

Die zweite Stufe 1312 ist parallel zu der ersten Stufe 1310 ausgelegt, ermöglicht also wiederum die verzögerte Weiterleitung der verzögerten Nervensignale 1332, 1334, 1336, wodurch zwei Mal verzögerte Nervensignale entstehen. Eine Vorrichtung zum erfindungsgemäßen Verarbeitung des Nervenaktivitätsmusters umfasst nun eine Mehrzahl von hintereinander geschalteten Stufen, die gleich wie die erste Stufe 1310 bzw. die zweite Stufe 1312 aufgebaut sind. Die Nervensignale 1320, 1322, 1324 werden also parallel durch die Mehrzahl von Stufen 1310, 1312, 1314 weitergeleitet, wobei jede Stufe eine einstellbare Verzögerung zu den Nervensignalen hinzufügt.

Weiterhin ist jede der Stufen 1310, 1312, 1314 ausgelegt, um eine Summe der an ihr einlaufenden bzw. der aus ihr auslaufenden Nervensignale (bzw. m-mal verzögerten Nervensignale) zu bilden. Ferner sind die Stufen 1310, 1312, 1314 bevorzugt ausgelegt, um diese Summe mit einem einstellbaren Schwellwert zu vergleichen, um festzustellen, ob zu einem gegebenem Zeitpunkt mindestens eine vorgegebene Anzahl von Nervensignalen bzw. verzögerten Nervensignalen (also einlaufende Nervensignale oder auslaufende Nervensignale) aktiv sind (bzw. ein Aktionspotential aufweisen).

Es wird ferner bevorzugt, dass die Verzögerungen der in den Stufen 1310, 1312, 1314 vorhandenen Verzögerungseinrichtungen unterschiedlich eingestellt sind, sodass beispielsweise ein erstes Nervensignal 1320 bei einem Durchlaufen der Stufen 1310, 1312, 1314 einer anderen Verzögerung unterliegt als das zweite Nervensignal 1322. Verzögerungen können beispielsweise so eingestellt sein, dass sich für die Nervensignale 1320, 1322, 1324 unterschiedliche Gesamtverzögerungen beim Durchlaufen durch die Stufen 1310, 1312, 1314 ergeben (wobei es freilich zulässig ist, das beispielsweise zwei Nervensignale in der gleichen Weise verzögert werden). In anderen Worten, die Einrichtung 1300 ist bevorzugt so ausgelegt, das sich nicht für alle Nervensignale die gleichen Verzögerungen ergeben. Außerdem ist es vorteilhaft, dass bei Vorhandensein von j Stufen 1310, 1312, 1314 mindestens (j-1) Stufen 1310, 1312 so ausgelegt sind, dass die in einer Stufe enthaltenen Verzögerungseinrichtungen für die Mehrzahl von Nervensignalen nicht alle die gleiche Verzögerung aufweisen. Dadurch kann erreicht werden, dass ein in eine erfindungsgemäße Einrichtung 1300 einlaufendes Nervenaktivitätsmuster über der Zeit beim Durchlauf durch die beschriebene Einrichtung zeitlich verzerrt wird, dass also einzelne Nervensignale gegenüber anderen Nervenssignalen zeitlich verschoben werden. Durch die Verzerrung können in einer zeitlichen Darstellung gebogene linienartige Muster, also Trajektorien, in dem Nervenaktivitätsmuster gerade gebogen werden.

Ferner wird darauf hingewiesen, dass durch die Summenbildung innerhalb einer Stufe erkannt werden kann, wenn eine ursprünglich gebogene Trajektorie in dem Nervenaktivitätsmuster zu einer geraden Linie gebogen wurde (die dadurch beschrieben wird, dass eine vorgegebene Anzahl der verzögerten Nervensignale nahezu gleichzeitig bzw. zeitlich überlappend ein Aktionspotential aufweisen).

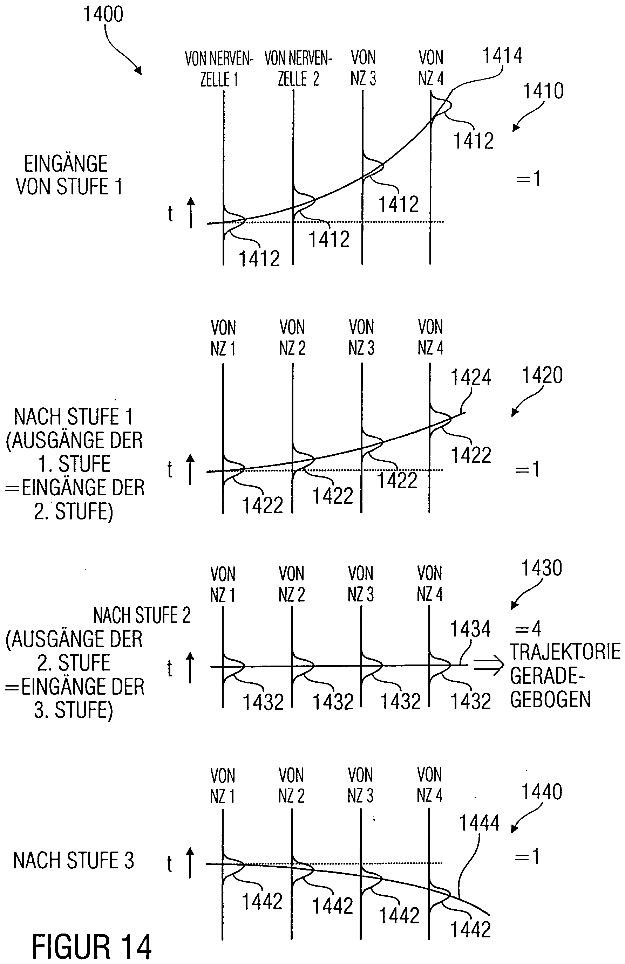

Die Funktionsweise der Einrichtung 1300 soll anhand der Figur 14 veranschaulicht werden. Figur 14 zeigt eine beispielhafte grafische Darstellung der Signale in einer Vorrichtung 1300 zum erfindungsgemäßen Verarbeiten des Nervenaktivitätsmusters. Die grafische Darstellung der Figur 14 ist in ihrer Gesamtheit mit 1400 bezeichnet.

Eine erste graphische Darstellung 1410 beschreibt hierbei ein beispielhaftes Nervenaktivitätsmuster an Eingängen der Vorrichtung 1300. Gezeigt sind hierbei beispielhaft die Signale von vier Nervenzellen (bzw. auf vier Nervenfasern) in einem zeitlichen Verlauf. Im übrigen wird darauf hingewiesen, dass die Aktionspotentiale 1412 eine Trajektorie 1314 bilden. Wie gezeigt, weist die Trajektorie 1414 in der zeitlichen Darstellung eine starke Krümmung auf, da die Aktionspotentiale 1412 von den verschiedenen Nervenfasern an den Eingängen der ersten Stufe 1310 einen deutlichen zeitlichen Versatz aufweisen. Somit liegt in der ersten Stufe 1310 zu einem festen Zeitpunkt nur jeweils ein Aktionspotential vor, sodass ein Schwellwert für eine Summe der an der ersten Stufe anliegenden Aktionspotentiale, der beispielsweise zu zwei gesetzt ist, nicht überschritten wird. Folglich liefert die erste Stufe kein Ausgangssignal an einem Schwellwertausgang.

Eine zweite grafische Darstellung 1420 beschreibt die Verhältnisse an einem Ausgang der ersten Stufe 1310. Hierbei wird davon ausgegangen, dass in der ersten Stufe 1310 das von der erste Nervenzelle NZ 1 gelieferte Nervensignal stärker verzögert wird als das von den anderen Stufen gelieferte Nervensignal. Im übrigen wird davon ausgegangen, dass bei dem gegebenem Beispiel das von der vierten Nervenzelle NZ4 gelieferte Nervensignal am wenigsten verzögert wird, während das Nervensignal von der dritten Nervenzelle NZ3 etwas mehr verzögert wird, und wobei die Verzögerung für Nervensignalen von den Nervensignalen NZ2 und NZ1 immer stärker ansteigen. Allgemein gesprochen werden Signale, die zu Nervenzellen gehören, die auf eine niedrigere Frequenz ansprechen, weniger stark verzögert, als Nervensignale von Nervenzellen, die höhere Frequenzen detektieren.

Die zweite grafische Darstellung zeigt somit wiederum Aktionspotentiale 1424 als Funktion der Zeit, wobei die Aktionspotentiale 1422 eine Trajektorie 1424 bilden. Wie aus der zweiten grafischen Darstellung 1420 ersichtlich ist, ist die Krümmung der Trajektorie 1424 an den Ausgängen der ersten Stufe geringer als eine (zeitlich-örtliche bzw. zeitlich-frequenzmäßige) Krümmung der Trajektorie 1414 an den Eingängen der ersten Stufe. Dies resultiert aus der unterschiedlichen Verzögerung der zu verschiedenen Nervenzellen gehörigen Nervensignale in den Verzögerungseinrichtungen (z.B. 1330) der ersten Stufe. Dadurch wird also eine gekrümmte Trajektorie gleichsam geradegebogen. Wie aus der zweiten grafischen Darstellung 1420 ersichtlich, weist die zweite Trajektorie 1424 allerdings noch eine Restkrümmung auf, so dass die von verschiedenen Nervenzellen bzw. Nervenfasern stammenden Aktionspotentiale 1422 nicht alle gleichzeitig an den Ausgängen der ersten Stufe 1310 bzw. Eingängen der zweiten Stufe 1312 anliegen.

Auch die zweite Stufe 1312 bewirkt eine weitere Verzögerung, wobei wiederum Signale von Nervenzellen, die für niedrige Frequenzen empfindlich sind, weniger verzögert werden als Signale von Nervenzellen, die für höhere Frequenzen empfindlich sind. Eine dritte grafische Darstellung 1430 zeigt die in der zweiten Stufe 1312 nochmals verzögerten Nervensignale an Ausgängen der zweiten Stufe. Es ist aus der dritten graphischen Darstellung 1430 ersichtlich, dass bei dem vorliegenden Beispiel die Nervensignale an den Ausgängen der zweiten Stufe jeweils so verzögert sind, dass Aktionspotentiale 1432 von mehreren Nervenzellen an den Ausgängen der zweiten Stufe gleichzeitig anliegen. In anderen Worten, eine Trajektorie 1434, die durch die Aktionspotentiale 1432 beschrieben wird, ist zumindest näherungsweise gerade gebogen. Die Aktionspotentiale 1432 treten also gleichzeitig bzw. näherungsweise gleichzeitig (zumindest aber zeitlich überlappend) auf, sodass das gleichzeitige Auftreten durch eine Summation der an den Ausgängen der zweiten Stufe (bzw. Eingängen der dritten Stufe) anliegenden Signale einen deutlichen Peak aufweist, der groß genug ist, um einen vorgegebenen Schwellenwert (z. B. zwei oder drei) zu überschreiten.

In anderen Worten, es kann durch eine geeignete Summiereinrichtung (oder eine andere geeignete Einrichtung) erkannt werden, wann eine gekrümmte Trajektorie gerade gebogen wurde. Die entsprechende Information ermöglicht einen Rückschluss sowohl auf den Anfangszeitpunkt der Trajektorie als auch auf die Form der Trajektorie. Es kann nämlich festgestellt werden, nach Durchlaufen von wie vielen Stufe eine Trajektorie gerade gebogen wurde. Dadurch kann bei Kenntnis der Verzögerungen für die einzelnen Nervensignale in den Stufen der Einrichtung 1300 auch auf eine ursprüngliche Form der Trajektorie zurückgeschlossen werden. Ferner ist die Durchlaufzeit für die Stufen bevorzugterweise bekannt, sodass auch der Zeitpunkt, zudem eine Trajektorie in die Einrichtung 1300 eingelaufen ist, bestimmt werden kann. Somit kann die Analysedarstellung sowohl charakteristische Zeitinformationen der Trajektorien als auch Informationen über Form bzw. Krümmung der Trajektorien umfassen.

Die zusätzliche Information über die Form der Trajektorien kann dann bei einer Weiterverarbeitung der Analysedarstellung vorteilhaft ausgenutzt werden, um z. B. eine Spracherkennung zu erleichtern oder eine Erkennungsqualität bei der Spracherkennung zu verbessern.

Im übrigen wird noch darauf hingewiesen, dass eine vierte grafische Darstellung 1440 zur Verbesserung des Verständnisses noch Ausgangssignale an Ausgängen einer dritten Stufe zeigt. Aktionspotentiale 1442 beschreiben eine Trajektorie 1444, die allerdings durch ein weiteres Verbiegen der Trajektorie wieder gekrümmt ist.

Es wird darauf hingewiesen, dass die Verzögerungen in den Stufen 1310, 1312, 1314 auf verschiedenem Weg erzielt werden können. Die Verzögerungseinrichtungen (z.B. 1330) können beispielsweise getaktet sein, und/oder es kann sich um kontinuierlich oder diskret einstellbare Verzögerungseinrichtungen handeln. Im übrigen ist es auch möglich, dass eine oder mehrere Verzögerungseinrichtungen in einer vorgegebenen Stufe für eine oder mehrere Nervensignale deaktiviert sind, sodass einige Nervensignale durch eine Stufe mit geringst möglicher Verzögerung weitergeleitet werden. Im übrigen ist festzuhalten, dass die Einrichtung 1300 insgesamt als eine analoge oder digitale Schaltung implementiert sein kann.

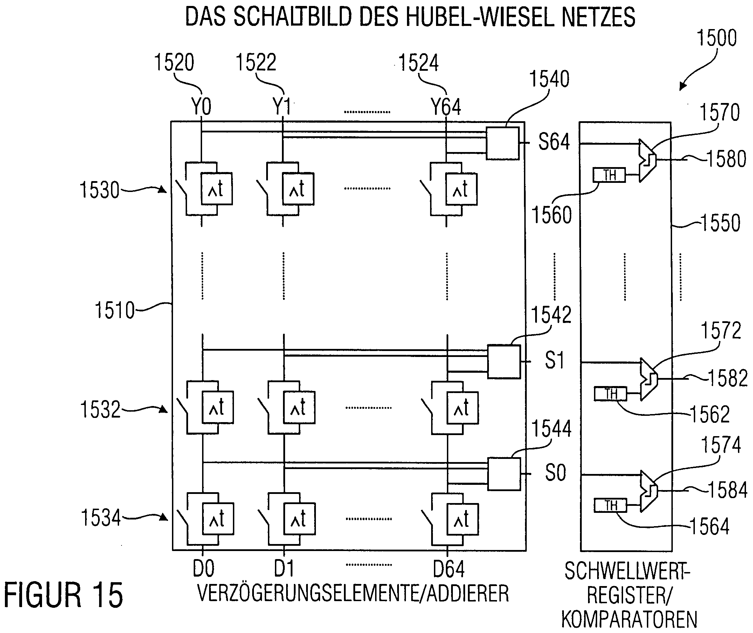

Figur 15 zeigt ein Schaltbild eines beispielhaften Hubel-Wiesel-Netzes zur erfindungsgemäßen Berechnung einer Analysedarstellung eines Audiosignals gemäß dem zweiten Ausführungsbeispiel der vorliegenden Erfindung. Das Schaltbild der Figur 15 ist in seiner Gesamtheit mit 1500 bezeichnet. Erster Schaltungsblock 1510 empfängt Eingangssignale 1520, 1522, 1524, die beispielsweise ein Nervenaktivitätsmuster oder ein Anregungsmuster einer Basilarmembran repräsentieren können. Die Eingangssignale 1520, 1522, 1524 werden dann durch eine Mehrzahl von Stufen 1530, 1532, 1534 geleitet. Ein Eingangssignal 1520 durchläuft somit eine Mehrzahl von Stufen 1530, 1532, 1534, wobei ein Eingangssignal 1520 in einer Stufe 1530, 1532, 1534 entweder eine Verzögerungseinrichtung durchläuft oder direkt zu einer darauffolgenden Stufe weitergeleitet wird. In anderen Worten, die Verzögerungseinrichtungen können auch überbrückt werden.

In anderen Worten, jede Stufe umfasst für jedes Signal eine schaltbare Verzögerungseinrichtung, wobei die Verzögerungseinrichtung in einen Signalpfad, der von einem Eingangssignal durchlaufen wird, eingeschaltet werden kann oder überbrückt werden kann. Signale an den Eingängen jeder Stufe werden abgegriffen und Summierern 1540, 1542, 1544 zugeführt, wobei jeweils die an den Eingängen einer Stufe anliegenden Signale aufsummiert werden. Der erste Schaltungsblock 1510 bildet somit ein Gitter von Verzögerungselementen und Addieren, die in der gezeigten Weise verschaltet sind.

Das Hubel-Wiesel-Netz 1500 weist ferner eine Schwellwerteinrichtung 1550 auf, wobei jeweils ein Wert aus einem Schwellwertregister 1560, 1562, 1564 sowie ein Ausgang eines Summierers 1540, 1543, 1544 einem Komparator 1570, 1572, 1572 zugeführt wird. Ausgangsignale 1580, 1582, 1584 der Komparatoren 1570, 1572, 1574 liefern dabei eine Aussage darüber, ob an den Eingängen einer vorgegebenen Stufe 1530, 1532, 1534 eine Anzahl von Signalen gleichzeitig aktiv sind, wobei eine Mindestanzahl, bei der ein aktives Ausgangssignal 1580, 1582, 1584 ausgegeben wird, durch die Schwellwertregister 1560, 1562, 1564 festgelegt ist. In anderen Worten, durch die Komparatoren 1570, 1572, 1574 kann in Verbindung mit den Summierern 1540, 1542, 1544 und den Schwellwertregistern 1560, 1562, 1564 festgestellt werden, wenn (bzw. nach Durchlaufen wie vieler der Stufen 1530, 1532, 1534) eine Trajektorie, die über die Eingänge 1520, 1522, 1524 des ersten Blocks 1510 eingelesen wurde, gerade gebogen ist.

Die Verzögerungen der einzelnen Stufen 1530, 1532, 1534 können dabei geeignet vorgegeben werden, um eine Erkennung einer möglichst großen Anzahl von Trajektorien (bzw. Trajektorien-Formen) zu ermöglichen.

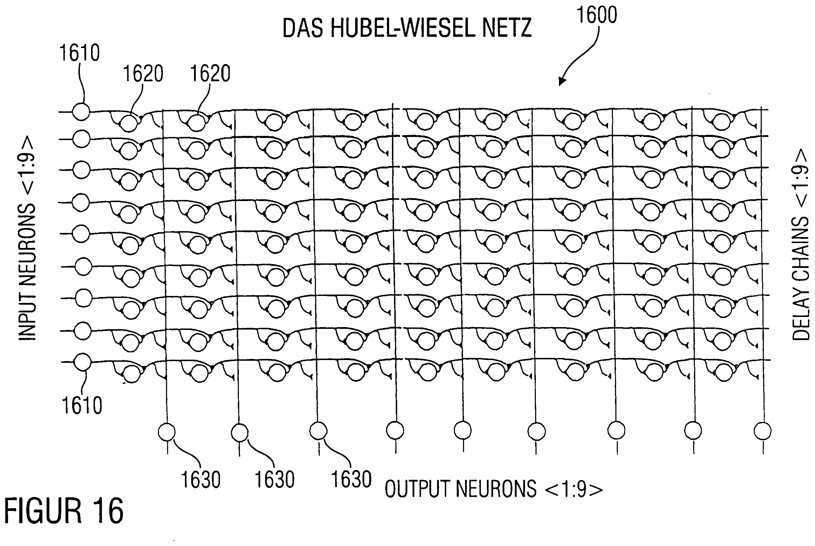

Figur 16 zeigt ein Blockschaltbild eines Hubel-Wiesel-Netzes zur erfindungsgemäßen Berechung einer Analysedarstellung eines Audiosignals gemäß dem zweiten Ausführungsbeispiel der vorliegenden Erfindung. Das gezeigte Hubel-Wiesel-Netzwerk ist in seiner Gesamtheit mit 1600 bezeichnet.

Eingangsneuronen 1610 sind ausgelegt, um ein Nervenaktivitätsmuster oder ein Anregungsmuster einer Basilarmembran eines Ohrmodells in Form von parallel bereitstehenden Zeitsignalen zu empfangen. Das Nervenaktivitätsmuster (bzw. das Anregungsmuster der Basilarmembran) wird dabei unter optionaler Einbeziehung von Verzögerungsneuronen durch mehrere Stufen des neuronalen Netzes weitergeleitet. Hierbei ist anzumerken, dass die Verzögerungsneuronen 1620 auch überbrückt werden können, sodass in einer Stufe keine Verzögerung eines von einem Eingangsneuron 1610 gelieferten Signals stattfindet. Das neuronale Netz weist ferner Ausgangsneuronen 1630 auf. Die Verschaltung des neuronalen Netzes 1600 kann dabei der Figur 16 entnommen werden. Es wird darauf hingewiesen, dass das gezeigte neuronale Netz in der Lage ist, eine gekrümmte Kurve bzw. Trajektorie in einem Nervenaktivitätsmuster (bzw. Basilarmembran-Anregungsmuster), das über die Eingangsneuronen 1610 das neuronale Netz 1600 eingegeben wird, zu erkennen. Das neuronale Netz ist dabei (nach einem Training) in der Lage, sowohl Zeitpunkt als auch Form einer Trajektorie in einem über die Eingangsneuronen 1610 eingegebenen Nervenaktivitätsmuster zu bestimmen, wobei ein aktives Ausgangsneuron diese Informationen beschreibt. Die Informationen über die Form und Zeit einer Trajektorie sind hierbei dadurch codiert, welches Ausgangsneuron wann aktiviert wird.

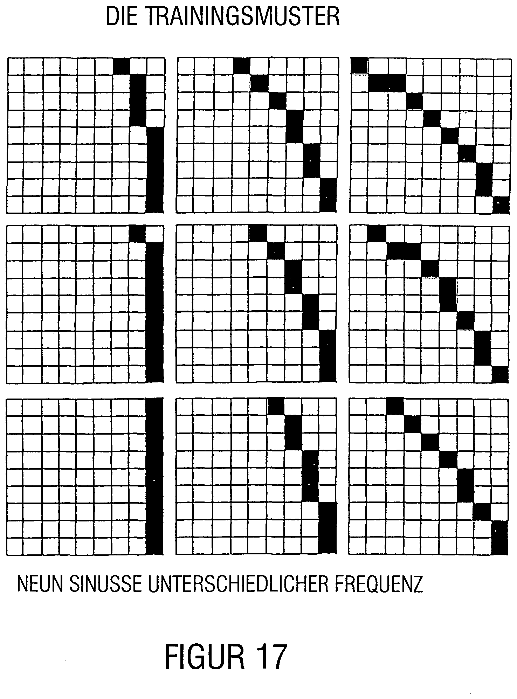

Figur 17 zeigt schließlich eine grafische Darstellung von beispielhaften Trennungsmustern, die zum Trainieren eines neuronalen Netzwerks 1600 verwendet werden können. Nach einem Training ist das neuronale Netzwerk 1600 dann in der Lage, entsprechende gerade oder gekrümmte Verläufe als Trajektorien zu identifizieren.

Somit ist festzuhalten, dass ein Hubel-Wiesel-Netz 1600 sich sehr gut dazu eignet, um Trajektorien in einem Nervenaktivitätsmuster zu erkennen. Hierzu ist lediglich das Nervenaktivitätsmuster an Eingänge 1520, 1522, 1524 des Hubel-Wiesel-Netzes anzulegen. An Ausgängen 1580, 1582, 1584 von Komparatoren 1570, 1572, 1574 stehen dann Signale an, die eine Aussage über Form und zeitliche Lage einer Trajektorie umfassen. Die Ausgangssignale 1580, 1582, 1584 können freilich bei Bedarf noch in eine leichter interpretierbare Form gebracht werden, aus der beispielsweise direkt eine Zeitinformation über eine Trajektorie hervorgeht. Die Zeitinformation bildet dann die vorteilhafte Analysedarstellung.

Es wird im übrigen darauf hingewiesen, dass das in Figur 17 bezeichnete neuronale Netz 1700, das sich sehr gut für eine Erkennung von Trajektorien in einem Nervenaktivitätsmuster eignet, im Detail in dem Artikel "A neural net for 2D-slope and sinusoidal shape detection" von A. Brückmann, S. Klefenz und A. Wünsche (veröffentlicht in dem CIST International Scientific Journal of Computing, ISSN 1727-6209) im Detail beschrieben wurde.

Ferner wird darauf hingewiesen, dass das Verarbeiten des Nervenaktivitätsmusters bevorzugt durch Anwendung einer sogenannten Hough-Transformation erfolgt (Vergleiche US Patent US 3,069,654). Eine Hough-Transformation ist nämlich in der Lage, in effektiver Weise aufeinanderfolgende Trajektorien in eine räumlich-zeitlichen Muster zu erkennen. Damit eignet sich die Hough-Transformation hervorragend zum Extrahieren einer Analysedarstellung aus einem Audiosignal, da im Rahmen der vorliegenden Erfindung erkannt wurde, dass eben Trajektorien in einem Nervenaktivitätsmuster eines Ohrmodells eine charakteristische Information des Audiosignals darstellen, die für eine weitere Analyse vorteilhaft eingesetzt werden kann.

Weiterhin wird darauf hingewiesen, dass auch andere bekannte Verfahren zur Mustererkennung eingesetzt werden können, um Trajektorien in den Nervenaktivitätsmuster zu erkennen. Hierbei können besonders vorteilhaft solche Verfahren eingesetzt werden, die eine Erkennung von gebogenen Linien ermöglichen, da erkannt wurde, dass Trajektorien in den Nervenaktivitätsmuster typischerweise eine hyperbolische Form aufweisen. Hierbei ist zu berücksichtigen, dass das Nervenaktivitätsmuster für eine Mehrzahl von Nerven über der Zeit ein zweidimensionales Muster ergibt, wobei entlang einer ersten Richtung Signale auf mehreren Nervenfasern beispielsweise durch eine Intensitätsverteilung bzw. durch numerische Werte dargestellt werden können, während hingegen in einer zweiten Richtung die zeitliche Entwicklung angetragen ist. Eine typische Darstellungsform für einen zeitlichen Verlauf eines Nervenaktivitätsmusters kann somit beispielsweise ähnlich zu den gezeigten Cochlea-Diagrammen (Cochleogrammen) sein.

Somit ist es möglich, einen beliebigen Mustererkennungsalgorithmus einzusetzen, der in der Lage ist, gekrümmte Linien zu erkennen. Allerdings hat sich gezeigt, dass die Anwendung einer Hough-Transformation besonders vorteilhaft ist, weil eben die Hough-Transformation sich besonders gut für eine Erkennung von gekrümmten Linien eignet. Im übrigen wird darauf hingewiesen, dass bei Durchführen einer Hough-Transformation in einer Anordnung 1600 bzw. 1700 auch bei Vorliegen mehrerer eng benachbarten Trajektorien ein gutes Erkennungsergebnis erzielt werden kann, wenn nur die Schwellenwerte bzw. Ansprechempfindlichkeiten der Ausgangsneuronen (bzw. der Komparatoren) passend eingestellt sind.

Ferner ist es möglich, im Rahmen einer Hough-Transformation (oder einer anderen Mustererkennungsoperation) auch eine Länge einer Trajektorie zu erkennen, sodass neben den Informationen über Zeitpunkt und Form der Trajektorie noch eine dritte Information für eine anschließende Analyse zur Verfügung steht.

Im übrigen wird darauf hingewiesen, dass auch ein zweidimensionaler Mustervergleich über der zeitlichen Darstellung des Nervenaktivitätsmuster durchgeführt werden kann, um eine Folge von Zeitinformationen, die eine zeitliche Lage von aufeinanderfolgenden Trajektorien beschreiben, zu gewinnen.

Ferner wird darauf hingewiesen, dass es vorteilhaft ist, aufgrund einer Analysedarstellung eines Audiosignals eine Mustererkennung durchzuführen. Gemäß der vorliegenden Erfindung existieren zwei besonders vorteilhafte Analysedarstellungen. Es hat sich nämlich gezeigt, dass ein Nervenaktivitätsmuster besonders vorteilhaft für eine Analyse eines Audiosignals eingesetzt werden kann. Ferner eignet sich auch eine Darstellung, die Zeitinformationen über in dem Nervenaktivitätsmuster enthaltene Trajektorien umfasst, besonders gut für eine Analyse des Audiosignals. Sowohl das Nervenaktivitätsmuster als auch die zeitliche Darstellung mit Informationen über in dem Nervenaktivitätsmuster enthaltene Trajektorien werden im folgenden als Audiosignal-Repräsentanten bezeichnet.

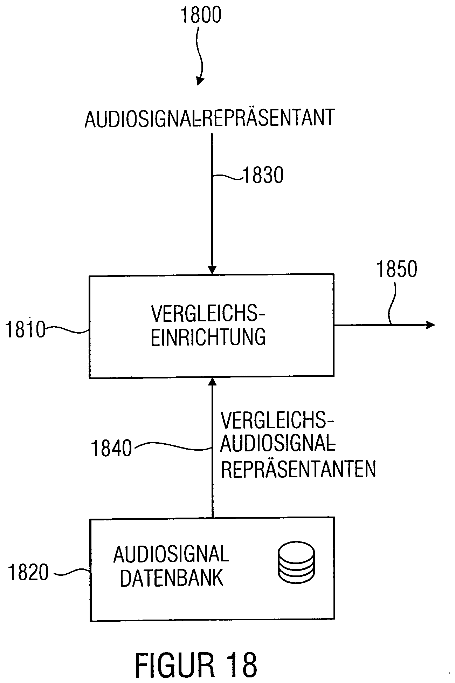

Figur 18 zeigt eine schematische Darstellung einer Einrichtung zur Identifizierung eines Audiosignals. Die in der Figur 18 gezeigte Einrichtung ist in Ihrer Gesamtheit mit 1800 bezeichnet. Die Einrichtung umfasst eine Vergleichseinrichtung 1810, die mit einer Audiosignal-Datenbank 1820 gekoppelt ist. Der Vergleichseinrichtung 1810 wird ferner ein Audiosignal-Repräsentant 1830 zugeführt. Basierend auf dem Audiosignal-Repräsentant 1830 und in der Audiosignal-Datenbank enthaltenen Vergleichs-Audiosignal-Repräsentanten erzeugt die Vergleichseinrichtung 1810 ein Vergleichsergebnis 1850, das eine Aussage darüber umfasst, ob der Audiosignal-Repräsentant 1830 eine Ähnlichkeit zu mindestens einem in der Audiosignal-Datenbank gespeicherten Vergleichs-Audiosignal-Repräsentanten 1840 aufweist. Das Vergleichsergebnis 1850 kann freilich auch eine Aussage darüber umfassen, zu welchen Vergleichs-Audiosignal-Repräsentanten 1840 der Audiosignal-Repräsentant 1830 die größte Ähnlichkeit aufweist.

Die Vergleichseinrichtung 1810 kann eine beliebige Vorrichtung zum Vergleich zweier Audiosignal-Repräsentanten umfassen. Beispielsweise ist es möglich, eine Einrichtung zu verwenden, die basierend auf mathematischen Methoden einen mathematischen Abstand zwischen dem Audiosignal-Repräsentant und dem Vergleichs-Audiosignal-Repräsentant ermitteln kann. Ferner kann auch ein neuronales Netz verwendet werden, um den Audiosignal-Repräsentanten 1830 mit den Vergleichs-Audiosignal-Repräsentanten 1840 zu vergleichen. Ein neuronales Netz kann jeweils beispielsweise mit einer Mehrzahl von Audiosignal-Repräsentanten trainiert werden.

Weiterhin wird darauf hingewiesen, dass die Audiosignal-Datenbank 1820 beispielsweise eine Mehrzahl von Vergleichs-Audiosignal-Repräsentanten umfassen kann, die Musikstücke beschreiben. Weiterhin ist es genau so gut möglich, dass die Audiosignal-Datenbank Vergleichs-Audiosignal-Repräsentanten umfasst, die lediglich einzelne Laute, beispielsweise einen Vokal oder einen Konsonanten beschreiben. Somit kann die gezeigte Einrichtung 1800 zum Identifizieren eines Audiosignals auch für eine Spracherkennung effektiv eingesetzt werden, wobei die gewählten Audiosignal-Repräsentanten sich für eine solche Anwendung besonders gut eignen.

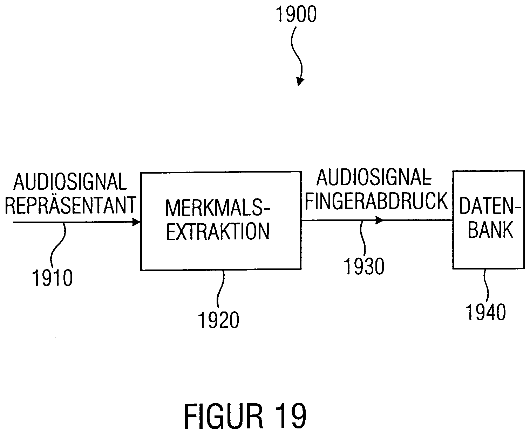

Figur 19 zeigt eine schematische Darstellung einer Einrichtung zum Extrahieren eines Audiosignal-Fingerabdrucks aus dem Audiosignal-Repräsentanten. Die in Figur 19 gezeigte Einrichtung zum Extrahieren des Audiosignal-Fingerabdrucks ist in ihrer Gesamtheit mit 1900 bezeichnet. Hierbei wird ein Audiosignal-Repräsentant 1910 einer Einrichtung 1920 zur Merkmalsextraktion zugeführt. Die Einrichtung 1920 zur Merkmalsextraktion erzeugt basierend auf dem Audiosignal-Repräsentant 1910 einen Audiosignal-Fingerabdruck 1930. Der Audiosignal-Fingerabdruck 1930 wird dann bevorzugt einer Audiosignal-Datenbank 1940 zugeführt.

Die Einrichtung 1920 zur Merkmalsextraktion kann beispielsweise ausgelegt sein, um eine Tonhöhe und/oder ein Rhythmus aus dem Audiosignal-Repräsentant 1910 zu extrahieren. Ist der Audiosignal-Repräsentant ein Nervenaktivitätsmuster, so kann beispielsweise eine Tonhöhe extrahiert werden, indem diejenigen Nervenfasern identifiziert werden, die eine maximale Aktivität in dem Nervenaktivitätsmuster aufweisen. Die Nervenfaser mit höchsten Aktivität in den Nervenaktivitätsmuster ist nämlich ein gutes Maß für eine Tonhöhe, da die Nervenfasern typischerweise eine bevorzugte Frequenz aufweisen. Ferner können Veränderungen (bzw. die entsprechenden Zeitpunkte der Veränderungen) in den Nervenaktivitätsmuster verwendet werden, um einen Rhythmus des Audiosignals zu bestimmen. Eine Erkennung von Veränderungen in den Nervenaktivitätsmuster ist vergleichsweise einfach möglich, indem zwischen aufeinanderfolgenden Momentanwerten des Nervenaktivitätsmusters ein Abstandsmaß, z. B. eine mathematische Norm, gebildet wird. Signifikante Veränderungen können dann erkannt werden, wenn das Abstandsmaß einen vorgegebenen Wert überschreitet.

Auch Verfahren zur Mustererkennung können auf den Audiosignal-Repräsentanten 1910 angewendet werden, um einen Audiosignal-Fingerabdruck zu erzeugen. Der Audiosignal-Fingerabdruck kann so somit beispielsweise eine kombinierte Information über eine Tonhöhe und/oder Rhythmus des Audiosignals umfassen.

Ist der Audiosignal-Repräsentant 1910 ferner eine Darstellung, die Zeitinformationen zu Trajektorien, die in den Nervenaktivitätsmuster enthalten sind, umfasst, so ist eine Merkmalsextraktion in einer besonders einfachen Weise möglich. Es können nämlich beispielsweise Zeitabstände zwischen den aufeinanderfolgenden Trajektorien berechnet werden, die dann für das Audiosignal charakteristisch sind.

Ferner hat sich auch gezeigt, dass eine Form der Trajektorien ein besonders wichtiges Merkmal ist. Somit ist es möglich, in einem Audiosignal-Fingerabdruck nur Informationen zu Trajektorien einer besonderen Form zu speichern, wodurch eine Datenmenge des Audiosignal-Fingerabdrucks 1930 verringert werden kann. Dies wiederum ermöglicht eine effiziente Ablage des Audiosignal-Fingerabdrucks in einer Datenbank.

Ferner hat sich gezeigt, dass einem Schallereignis typischerweise mehrere Trajektorien zugeordnet sind, die eine charakteristische Form und einen charakteristischen Abstand aufweisen. Somit kann beispielsweise bei der Merkmalsextraktion 1920 eine Gruppe von einer Mehrzahl von Trajektorien ausgewertet werden, woraufhin jeweils einer Gruppe von Trajektorien ein Symbol zugeordnet wird. Eine Folge von Symbolen, die zu aufeinanderfolgenden Gruppen von Trajektorien gehören, bilden dann den Audiosignal-Fingerabdruck und können in einer Datenbank abgelegt werden. Basierend auf einer Analyse einer Gruppe von Trajektorien kann ferner eine Spracherkennung realisiert werden, da Vokalen und Konsonanten jeweils charakteristische Trajektorien-Muster (im Hinblick auf Abstand und Form der Trajektorien) aufweisen, die erkannt werden können.

Zusammenfassend lässt sich also festhalten, dass die vorliegende Erfindung ein neurophysiologisch parametrisiertes Modell der ersten Stufen des Gehörsystems (first stages of the auditory system model) schafft. Das Modell setzt sich aus einer Cochlea-Modellierung, einem harmonischen Oszillatormodell der Stereozilienbewegungsgleichung sowie einem Modell für eine Neurotransmitter-Vesikel-Freisetzung der inneren Hörzellen (IHC Neurotransmitter Vesikel Release) zusammen. Ferner umfasst das erfindungsgemäße Modell eine Beschreibung für die Generierung von postsynaptischen Aktionspotentialen. Es wird hierbei darauf hingewiesen, dass bevorzugt ein Federkonstantenwert für die Beschreibung der Stereozilien verwendet wird, der mit Hilfe eines Rasterkraftelektronenmikroskops ermittelt ist. Das erfindungsgemäße Modell kann beispielsweise zur Optimierung einer Simulation von Cochlea-Implantaten dienen, da Cochlea-Implantate direkt den auditorischen Nerv anregen.

Ein wesentlicher Vorteil der vorliegenden Erfindung besteht darin, dass die Vorrichtung in einer neurophysiologischen Weise angepasst ist. Ferner wird in einer vorteilhaften Weise eine stochastische Ausschüttung von Neurotransmitter-Vesikeln berücksichtigt. Für eine Ankoppelung der Basilarmembrangeschwindigkeit an die Stereozilienbewegung wird ein erfindungsgemäßes Oszillatormodell verwendet, wobei die Bewegungsgleichungen für die Stereozilienbewegung optimiert ist. Eine Kräftegleichung bzw. Bewegungsgleichung für die Stereozilienbewegung lautet:

$$m\ddot{x} = -Dx - k\dot{x} + F_{ext} + F_{brown} \ .$$

Dabei beschreibt Dx die Federrückstellkraft, $k\dot{x}$ einen laminaren Strömungswiderstand, $F_{ext}$ (Proportionalitätskonstante x Basilarmembrangeschwindigkeit v) eine Anregung und $F_{brown}$ eine stochastische thermische Kraft durch Stoßbewegung der Atome.

Eine post-synaptische Generierung von Aktionspotentialen wird ebenso modelliert (vgl. E. Neher und T. Sakaba: "Quantal release parameters estimated from noise" J. Neurosience, December 15, 2001, 21 (24):9638-9654).

In anderen Worten, die vorliegende Erfindung zeigt eine Vorrichtung zur Analyse eines Audiosignals, wobei die ersten Stufen des Gehörsystems von der mechanischen Schallwandlung im Innenohr, der Übertragung durch die Gehörknöchelchen, der hydromechanischen Schwingungsanregung der Cochlea, der mechano-elektrischen Wandlung an den inneren Haarzellen bis hin der Erzeugung der Puls-Spitzen (Puls-Spike-Generierung) der Spiralganglienzellen des auditorischen Nervs modelliert sind.

Ein besonderer Vorteil der vorliegenden Erfindung liegt in der Verwendung des zur Basilaranregung verwendeten Modells. Ferner wird bei der vorliegenden Erfindung eine besonders vorteilhafte Modellierung der inneren Haarzellen verwendet. Eine Freisetzung von Neurotransmittern in einem synaptischen Spalt erfolgt gemäß der vorliegenden Erfindung in Vesikel verpackt. Die vorliegende Erfindung umfasst ferner ein besonders vorteilhaftes Modell einer Kopplung zwischen der Basilarmembran und einer Bewegung der Stereozilien, wobei ein harmonisches Oszillatormodell verwendet wurde. Ein weiterer Vorteil der vorliegenden Erfindung liegt auch in einer neurophysiologischen Parametrisierung. Für eine Federkonstante (spring stiffness konstante) wurde eine IHC-Tabellenwert für die Maus angenommen. Eine post-synaptische Spitzenerzeugung (Post-synaptische Spike-Generierung) erfolgt erfindungsgemäß unter Verwendung eines Diffusions-/Refraktärmodells). Die vorliegende Erfindung ist somit in der Lage, Gehörvorgänge sehr realistisch nachzubilden, also zu erzeugen und zu analysieren.

Im Übrigen sei darauf hingewiesen, dass die vorliegende Erfindung auch eine Vorrichtung zur Expansion von Wanderwellen auf einer Cochlea (Cochlea travelling wave expansion) schafft. Die entsprechende Vorrichtung basiert auf einer frequenzabhängigen Berechnungsformel für Verzögerungstrajektorien (Delay-Trajektorien) längs einer Basilarmembran. Diese Formel beruht auf den Arbeiten von Greenberg. Die Verzögerung der Trajektorien entsteht durch die Wellengruppengeschwindigkeit bei einer Ausbreitung eines Impulses auf der Basilarmembran eines Ohrmodells. Tiefe Frequenzen ergeben einen zeitverzögerten Ausschlag auf der Basilarmembran, da die tiefen Frequenzen an dem Ende der Basilarmembran registriert werden. Somit ist für die tiefen Frequenzen die Laufzeitverzögerung der Basilarmembran wirksam.

Gekrümmte Bahnen bzw. Trajektorien können mit einer Hough-Transformation vorteilhaft bestimmt werden. So eignet sich die Hough-Transformation sehr gut, um Kreise, Ellipsen oder Geraden zu erkennen, aber auch andere linienförmige Kurven können mit Hilfe einer Hough-Transformation erkannt werden. Im Übrigen wird darauf hingewiesen, dass ein Zeit-Weiterleitungs-Netzwerk (timing feed forward network) gemäß der Hubel-Wiesel-Theorie Muster wie Balken oder Sinusaudiosignale unterschiedlicher Frequenz selbst lernen kann. In anderen Worten, ein Hubel-Wiesel-Netzwerk kann selbststrukturierend und selbstorganisierend eine Hough-Transformation nach einer beschriebenen Verzögerungsleitungs-Methode lernen.

Eine besondere Erkenntnis der vorliegenden Erfindung liegt darin, dass die Hough-Transformation zur Informationsgewinnung aus den parallelen Puls-Spitzen-Zügen (Pulse spiking trains) direkt mit dem auditorischen Nerv eines Ohrmodells gekoppelt wird. In anderen Worten, die Hough-Transformation wird direkt an den auditorischen Nerv angeflanscht, um die parallelen "Pulse spiking trains" zu verarbeiten. Die Hough-Transformation biegt hierbei VerzögerungsTrajektorien gerade und detektiert sowohl die Signalform (Krümmung) als auch den Auftrittzeitpunkt der Trajektorien. Bei der Hough-Transformation durchlaufen die Daten kontinuierlich mehrere Stufen. Eine Zeitfensterung ist hierbei, anders als bei bekannten Analysemethoden, nicht erforderlich.

In anderen Worten, die vorliegende Erfindung modelliert die ersten Stufen des Gehörsystems, von der mechanischen Schallwandlung im Innenohr, der Übertragung durch die Gehörknöchelchen, der hydromechanischen Schwingungsanregung der Cochlea, der mechano-elektrischen Wandlung an den inneren Haarzellen bis hin zur Erzeugung von Pulsspitzen (Puls spike Generierung) in den Spiralganglienzellen des auditorischen Nervs. Jedes Audiosignal generiert ein zweidimensionales Basilarmembrangeschwindigkeitsprofil aufgetragen gegen die Zeit, wobei die Basilarmembran bevorzugt in n-Sektionen unterteilt ist. Ein Klick-Impuls erzeugt dabei eine Wanderwellenbewegung auf der Basilarmembran. Eine Verzögerungs-Trajektorie der Faserlatenzzeit des auditorischen Nervs (AN-fiber latency) eines sinusförmigen Anregungssignäls ist frequenzabhängig und berechnet sich nach der Formel von Greenberg zu $d_a = 1000/f_i + 2$ ms. Ein Audiosignal, das beispielsweise Vokale umfasst, ist durch ein Bündel von Verzögerungs-Trajektorien gegeben, wobei die Verzögerungs-Trajektorien eine jeweils frequenzabhängige Form aufweisen.

Die vorliegende Erfindung ermöglicht es hierbei, die Verzögerungs-Trajektorien zu detektieren. Hierbei kann bevorzugterweise ein erfindungsgemäßer Hubel-Wiesel-Neurosimulator eingesetzt werden, der die Detektion von sinusförmigen Mustern oder Geraden selbst erlernen kann. Ein entsprechender Hubel-Wiesel-Neurosimulator kann bevorzugt eine parallele Hough-Transformation lernen, die vorteilhaft in einer erfindungsgemäßen Vorrichtung zur Erzeugung eines Analysesignals aufgrund eines Audiosignals eingesetzt werden kann.

Die Verzögerungs-Trajektorien werden erfindungsgemäßer Weise durch eine parallele Hough-Transformation bestimmt, wobei vermerkt wird, zu welchem Zeitpunkt welche Verzögerungs-Trajektorie gefunden wird, um dadurch die vorliegende Signalform zu identifizieren. Bei einem erfindungsgemäßen Einsatz einer Hough-Transformation kann aus einem Nervenaktivitätsmuster (neural activity pattern) direkt ein Größen-Form-Abbild (size-shape-image) in einem Schritt erzeugt werden. Eine weitere Analyse des Audiosignals kann erfindungsgemäßer Weise in einem Hough-Raum (houghspace) erfolgen.

Es wird ferner darauf hingewiesen, dass die erfindungsgemäße Vorrichtung auch ein erfindungsgemäßes Verfahren definiert. Das Verfahren kann in beliebiger Weise durchgeführt werden, wobei sich eine elektronische Recheneinrichtung besonders gut für die Durchführung des erfindungsgemäßen Verfahrens eignet.

In anderen Worten, die erfindungsgemäße Vorrichtung und das erfindungsgemäße Verfahren kann in Hardware oder in Software implementiert werden. Die Implementation kann auf einem digitalen Speichermedium, beispielsweise einer Diskette, einer CD, einer DVD oder einem FLASH-Speicher mit elektronisch auslesbaren Steuersignalen erfolgen, die so mit einem programmierbaren Computersystem zusammenwirken können, dass das entsprechende Verfahren ausgeführt wird. Allgemein besteht die vorliegende Erfindung somit auch in einem Computer-Programm-Produkt mit auf einem maschinenlesbaren Träger gespeicherten Programmcode zur Durchführung des erfindungsgemäßen Verfahrens, wenn das Computerprogramm-Produkt auf einem Rechner abläuft. In anderen Worten ausgedrückt, kann die Erfindung somit als ein Computer-Programm mit einem Programmcode zur Durchführung des Verfahrens realisiert werden, wenn das Computer-Programm auf einem Computer abläuft.

Die vorliegende Erfindung zeigt also die Bearbeitung eines Basilarmembran-Schwingungsmusters mit Hilfe der Hough-Transformation.

## Patentansprüche

1. Vorrichtung (700) zum Analysieren eines Audiosignals, um eine Analysedarstellung (760) des Audiosignals zu erhalten, mit folgenden Merkmalen:

   eine Einrichtung (720) zum Berechnen eines Nervenaktivitätsmusters über der Zeit an Nervenfasern eines Ohrmodells, das sich aufgrund des Audiosignals (710) ergibt; und

   eine Einrichtung (230) zum Verarbeiten des Nervenaktivitätsmusters, um als Analysedarstellung (760) eine Folge von Zeitinformationen (t1,t2) zu erhalten, die eine zeitliche Lage von aufeinanderfolgenden Trajektorien (740,750) beschreiben, wobei eine Trajektorie (740,750) Aktivitätsimpulse auf verschiedene Nervenfasern (NF1, NF2, NF3, NF4, NF5) aufgrund des gleichen Ereignisses in dem Audiosignal 710 umfasst; **dadurch gekennzeichnet, dass** die Einrichtung (730) zum Verarbeiten des Nervenaktivitätsmusters ausgelegt ist, um eine zweidimensionale Darstellung des Nervenaktivitätsmusters über der Zeit schrittweise zu verzerren, um eine verzerrte zweidimensionale Darstellung des Nervenaktivitätsmusters über der Zeit zu erhalten, und um zu erkennen, wenn in der verzerrten zweidimensionalen Darstellung des Nervenaktivitätsmusters über der Zeit eine näherungsweise gerade Linie enthalten ist, um die näherungsweise gerade Linie als Trajektorie zu erkennen, um die zeitliche Lage der Trajektorie zu ermitteln und um die zu der Trajektorie gehörige Zeitinformation als Analysedarstellung des Audiosignals zu liefern.

2. Vorrichtung gemäß Anspruch 1, wobei ein Nervenaktivitätsmuster (730) eine Aktivität einer Gruppe von Nervenfasern des Ohrmodells beschreibt.

3. Vorrichtung gemäß Anspruch 1 oder 2, wobei eine Zeitinformation, (t1, t2), einen Zeitpunkt eines Auftretens einer Trajektorie (740,750) beschreibt.

4. Vorrichtung (700) gemäß einem der Ansprüche 1 bis 3, die ausgelegt ist, um eine Trajektorie (740,750) zu erkennen, wenn Aktivitätsimpulse, die durch gleiche Ereignisse in dem Audiosignal bedingt sind, auf eine Anzahl von Nervenfasern vorliegen, die größer ist als eine vorgegebene Mindestanzahl.

5. Vorrichtung gemäß einem der Ansprüche 1 bis 4, die ausgelegt ist, um einen Beginn eines Vokals, eines Konsonanten oder eines Lauts in dem Audiosignal als ein akustisches Ereignis zu erkennen.

6. Vorrichtung gemäß einem der Ansprüche 1 bis 5, die ausgelegt ist, um eine Trajektorie in dem Nervenaktivitätsmuster zu erkennen, die eine Wanderwelle auf einer Basilarmembran des Ohrmodells beschreibt.

7. Vorrichtung gemäß einem der Ansprüche 1 bis 6, wobei eine Trajektorie als ein Nervenaktivitätsmuster auf einer Gruppe benachbarter Nervenfasern, das einer Wanderwelle auf der Basilarmembran des Ohrmodells zugeordnet ist, definiert ist.

8. Vorrichtung gemäß einem der Ansprüche 1 bis 7, wobei eine Trajektorie durch ein Auftreten von Aktivitätsimpulsen auf einer Gruppe von benachbarten Nervenfasern definiert ist, wobei die Aktivitätsimpulse eine Anregung von benachbarten Nervenfasern durch eine Wanderwelle beschreiben.

9. Vorrichtung gemäß einem der Ansprüche 1 bis 8, bei der die Zeitinformationen Anfangszeitpunkte der Trajektorien beschreiben.

10. Vorrichtung gemäß Anspruch 9, wobei der Anfangszeitpunkt einer vorgegebenen Trajektorie ein Zeitpunkt ist, an dem ein erster zu der vorgegebenen Trajektorie gehöriger Aktivitätsimpuls auf einer Nervenfaser auftritt.

11. Vorrichtung gemäß einem der Ansprüche 1 bis 10, bei der die Einrichtung (730) zum Verarbeiten des Nervenaktivitätsmusters (730) eine Einrichtung zur Mustererkennung umfasst, die ausgelegt ist, um in einer zweidimensionalen Darstellung, die durch das Nervenaktivitätsmuster über der Zeit gebildet wird, ein gerades oder gekrümmtes linienförmiges Muster als eine Trajektorie (740, 750) zu erkennen, die zeitige Lage der Trajektorie (740, 750) zu ermitteln und eine zu der Trajektorie (740, 750) gehörige Zeitinformation (t1,t2) als Analysedarstellung des Audiosignals zu liefern.

12. Vorrichtung gemäß Anspruch 11, bei der die Einrichtung zur Mustererkennung ferner ausgelegt ist, um eine Information über eine Form der Trajektorien als Teil der Analysedarstellung zu liefern.

13. Vorrichtung gemäß Anspruch 12, bei der die Information über die Form der Trajektorie eine Information über eine Krümmung der Trajektorie umfasst.

14. Vorrichtung gemäß einem der Ansprüche 11 bis 13, bei der die Einrichtung zur Mustererkennung festgelegt ist, um eine gerade oder eine hyperbelartig gekrümmte Trajektorie zu erkennen.

15. Vorrichtung gemäß einem der Ansprüche 11 bis 14, bei der die Einrichtung zur Mustererkennung ferner ausgelegt ist, um eine Information über eine Länge der Trajektorie als Teil der Analysedarstellung zu liefern.

16. Vorrichtung gemäß einem der Ansprüche 1 bis 15, bei der die Einrichtung (730) zum Bearbeiten des Nervenaktivitätsmusters eine Einrichtung zum Mustervergleich umfasst, die ausgelegt ist, um eine zweidimensionale Darstellung, die das Nervenaktivitätsmuster über der Zeit beschreibt, mit mindestens einem Vergleichsmuster zu vergleichen, um eine Trajektorie zu erkennen und eine Zeitinformation zu erhalten, die eine zeitliche Lage der Trajektorie beschreibt.

17. Vorrichtung gemäß Anspruch 16, bei der das Vergleichsmuster eine gerade oder eine hyperbelförmige Kurve ist.

18. Vorrichtung gemäß einem der Ansprüche 1 bis 17, bei der die Einrichtung (730) zum Verarbeiten des Nervenaktivitätsmusters ausgelegt ist, um die zweidimensionale Darstellung des Nervenaktivitätsmusters über der Zeit schrittweise derart zu verzerren, so dass eine gekrümmte Trajektorie in dem Nervenaktivitätsmuster durch das schrittweise Verzerren schrittweise geradegebogen wird, wobei eine Anzahl von Verzerrungsschritten, die für ein Geradebiegen der gekrümmten Trajektorie benötigt werden, von einer Krümmung der gekrümmten Trajektorie abhängig sind, und wobei die Anzahl der Verzerrungsschritte, die für das Geradebiegen der gekrümmten Trajektorie benötigt werden, eine Aussage über eine ursprüngliche Form der Trajektorie umfasst.

19. Vorrichtung gemäß einem der Ansprüche 1 bis 18, bei der die Einrichtung (730) zum Verarbeiten des Nervenaktivitätsmusters eine Kurvenerkennungseinrichtung (1300,1500,1600) umfasst, die ausgelegt ist, um das Nervenaktivitätsmuster in Form einer Mehrzahl von Signalen (1320,1322,1324;1520,1522,1524) parallel zu empfangen, und um die Signale (1320,1322,1324;1520,1522,1524) unterschiedlich schnell parallel durch eine Mehrzahl von hintereinandergeschalteten Stufen (1310,1312,1314;1530,1532,1524) weiterzuleiten, wobei mindestens eine vorbestimmte Stufe (1310,1312,1314;1530,1532,1534) eine Schwellwerterkennungseinrichtung (1560,1570,1562,1572,1564,1574) aufweist, die ausgelegt ist, um zu erkennen, wenn mindestens eine vorgegebene Anzahl an Signalen in der vorbestimmten Stufe gleichzeitig aktiv sind.

20. Vorrichtung gemäß Anspruch 19, wobei mindestens eine Stufe (1310,1312,1314;1530,1532,1534) ausgelegt ist, um mehrere Signale bei einer Weiterleitung durch die Stufe unterschiedlich stark zu verzögern.

21. Vorrichtung gemäß Anspruch 19 oder 20, wobei die Kurvenerkennungseinrichtung (1300;1500;1600) ein neuronales Netz ist.

22. Vorrichtung gemäß einem der Ansprüche 1 bis 21, bei der die Einrichtung zum Verarbeiten des Nervenaktivitätsmusters ausgelegt ist, um eine Trajektorie aufgrund einer Auswertung einer Hough-Transformation zu erkennen.

23. Vorrichtung gemäß einem der Ansprüche 1 bis 22, bei der die Einrichtung zum Verarbeiten des Nervenaktivitätsmusters eine Einrichtung zur Durchführung einer parallelen Hough-Transformation umfasst.

24. Vorrichtung gemäß einem der Ansprüche 1 bis 23, die ferner eine Einrichtung (1800) zum Analysieren eines Audiosignalinhalts aufgrund der Zeitinformation (t1, t2) umfasst.

25. Vorrichtung gemäß Anspruch 24, bei der die Einrichtung zum Analysieren des Audiosignalinhalts eine Vergleichseinrichtung (1810) umfasst, die ausgelegt ist, um die Zeitinformation (t1, t2) mit in einer Datenbank (1820) vorhandenen Referenz-Zeitinformationen (1840) zu vergleichen, um als Vergleichsergebnis eine Information (1850) über den Audiosignalinhalt zu liefern.

26. Vorrichtung gemäß Anspruch 24 oder 25, bei der die Einrichtung zum Analysieren des Audiosignalinhalts ausgelegt ist, um einen Vokal oder Konsonanten aufgrund der zu mehreren aufeinanderfolgenden Trajektorien gehörigen Zeitinformationen zu erkennen.

27. Vorrichtung gemäß einem der Ansprüche 24 bis 26, bei der die Einrichtung zum Analysieren des Audiosignals ferner ausgelegt ist, um eine Information über eine Form und/oder Krümmung der Trajektorie zu verwenden.

28. Vorrichtung gemäß einem der Ansprüche 24 bis 27, bei der die Einrichtung zum Analysieren des Audiosignals ausgelegt ist, um basierend auf den Zeitinformationen eine Information über einen Rhythmus des Audiosignals zu ermitteln, wobei die Information über den Rhythmus des Audiosignals eine Beschreibung für den Inhalt des Audiosignals darstellt.

29. Vorrichtung (700) zum Analysieren eines Audiosignals, um eine Analysedarstellung (760) des Audiosignals zu erhalten, mit folgenden Merkmalen:

eine Einrichtung (720) zum Berechnen eines Nervenaktivitätsmusters über der Zeit an Nervenfasern eines Ohrmodells, das sich aufgrund des Audiosignals (710) ergibt; und

eine Einrichtung (230) zum Verarbeiten des Nervenaktivitätsmusters, um als Analysedarstellung (760) eine Folge von Zeitinformationen (t1,t2) zu erhalten, die eine zeitliche Lage von aufeinanderfolgenden Trajektorien (740,750) beschreiben, wobei eine Trajektorie (740,750) Aktivitätsimpulse auf verschiedene Nervenfasern (NF1, NF2, NF3, NF4, NF5) aufgrund des gleichen Ereignisses in dem Audiosignal 710 umfasst; **dadurch gekennzeichnet, dass** die Einrichtung (730) zum Verarbeiten des Nervenaktivitätsmusters eine Kurvenerkennungseinrichtung (1300,1500,1600) umfasst, die ausgelegt ist, um das Nervenaktivitätsmuster in Form einer Mehrzahl von Signalen (1320,1322,1324;1520,1522,1524) parallel zu empfangen, und um die Signale (1320,1322,1324;1520,1522,1524) unterschiedlich schnell parallel durch eine Mehrzahl von hintereinandergeschalteten Stufen (1310,1312,1314;1530,1532,1524) weiterzuleiten, wobei mindestens eine vorbestimmte Stufe (1310,1312,1314;1530,1532,1534) eine Schwellwerterkennungseinrichtung (1560,1570,1562,1572,1564,1574) aufweist, die ausgelegt ist, um zu erkennen, wenn mindestens eine vorgegebene Anzahl an Signalen in der vorbestimmten Stufe gleichzeitig aktiv sind.

30. Verfahren zum Analysieren eines Audiosignals, um eine Analysedarstellung des Audiosignals zu erhalten, mit folgenden Schritten:

Berechnen eines Nervenaktivitätsmusters über der Zeit an Nervenfasern eines Ohrmodells, das sich aufgrund des Audiosignals ergibt; und

Verarbeiten des Nervenaktivitätsmusters, um als Analysedarstellung eine Folge von Zeitinformationen zu erhalten, die eine zeitliche Lage von aufeinanderfolgenden Trajektorien beschreiben, wobei eine Trajektorie Aktivitätsimpulse auf verschiedenen Nervenfasern aufgrund des gleichen Ereignisses in dem Audiosignal umfasst; **dadurch gekennzeichnet, dass** das Verarbeiten des Nervenaktivitätsmusters folgende Schritte umfasst:

schrittweises Verzerren einer zweidimensionalen Darstellung des Nervenaktivitätsmusters über der Zeit, um eine verzerrte zweidimensionale Darstellung des Nervenaktivitätsmusters über der Zeit zu erhalten, Erkennen, wenn in der verzerrten zweidimensionalen Darstellung des Nervenaktivitätsmusters über der Zeit eine näherungsweise gerade Linie enthalten ist, Erkennen der näherungsweise geraden Linie als eine Trajektorie,

Ermitteln der zeitlichen Lage der Trajektorie, und Liefern der zu der Trajektorie gehörigen Zeitinformation als Analysedarstellung des Audiosignals;

wobei das Verfahren unter Verwendung einer Hardware oder unter Verwendung eines Computers ausgeführt wird.

31. Verfahren zum Analysieren eines Audiosignals, um eine Analysedarstellung des Audiosignals zu erhalten, mit folgenden Schritten:

Berechnen eines Nervenaktivitätsmusters über der Zeit an Nervenfasern eines Ohrmodells, das sich aufgrund des Audiosignals ergibt; und

Verarbeiten des Nervenaktivitätsmusters, um als Analysedarstellung eine Folge von Zeitinformationen zu erhalten, die eine zeitliche Lage von aufeinanderfolgenden Trajektorien beschreiben, wobei eine Trajektorie Aktivitätsimpulse auf verschiedenen Nervenfasern aufgrund des gleichen Ereignisses in dem Audiosignal umfasst; **dadurch gekennzeichnet, dass** das Verarbeiten des Nervenaktivitätsmusters ein paralleles Empfangen des Nervenaktivitätsmusters in Form einer Mehrzahl von Signalen (1320,1322,1324;1520,1522,1524),

ein unterschiedlich schnelles Weiterleiten der Signale (1320,1322,1324;1520,1522,1524)durch eine Mehrzahl von hintereinandergeschalteten Stufen (1310,1312,1314;1530,1532,1524) und

ein Erkennen, wenn mindestens eine vorgegebene Anzahl an Signalen in einer vorbestimmten Stufe gleichzeitig aktiv sind, umfasst;

wobei das Verfahren unter Verwendung einer Hardware oder unter Verwendung eines Computers ausgeführt wird.

32. Computer-Programm mit einem Programmcode zur Durchführung des Verfahrens gemäß Anspruch 30 oder 31, wenn das Computer-Programm auf einem Computer abläuft.

**Claims**

1. A device (700) for analyzing an audio signal to obtain an analysis representation (760) of the audio signal, comprising:

means (720) for calculating a neural activity pattern over time resulting at nerve fibers of an ear model based on the audio signal (710); and

means (230) for processing the neural activity pattern in order to obtain a sequence of time information (t1, t2) as an analysis representation (760) describing a temporal position of consecutive trajectories (740, 750), wherein a trajectory (740, 750) includes activity impulses on different nerve fibers (NF1, NF2, NF3, NF4, NF5) based on the same event in the audio signal (710);

**characterized in that** the means (730) for processing the neural activity pattern is implemented to gradually distort a two-dimensional representation of the neural activity pattern over time in order to obtain a distorted two-dimensional representation of the neural activity pattern over time, and to recognize when an approximately straight line is contained in the distorted two-dimensional representation of the neural activity pattern over time in order to recognize the approximately straight line as a trajectory in order to determine the temporal position of the trajectory and to provide the time information belonging to the trajectory as an analysis representation of the audio signal.

2. The device according to claim 1, wherein a neural activity pattern (730) describes an activity of a group of nerve fibers of the ear model.

3. The device according to claim 1 or 2, wherein time information (t1, t2) describes a point in time of an occurrence of a trajectory (740, 750).

4. The device (700) according to one of claims 1 to 3, which is implemented to recognize a trajectory (740, 750) when activity impulses conditioned by like events in the audio signal are present on a number of nerve fibers which is greater than a predetermined minimum number.

5. The device according to one of claims 1 to 4, which is implemented in order to recognize the beginning of a vocal, a consonant or a tone in the audio signal as an acoustic event.

6. The device according to one of claims 1 to 5, which is implemented in order to recognize a trajectory in the neural activity pattern describing a traveling wave on a basilar membrane of the ear model.

7. The device according to one of claims 1 to 6, wherein a trajectory is defined as a neural activity pattern on a group of neighboring nerve fibers associated with a traveling wave on the basilar membrane of the ear model.

8. The device according to one of claims 1 to 7, wherein a trajectory is defined by an occurrence of activity impulses on a group of neighboring nerve fibers, wherein the activity impulses describe an excitation of neighboring nerve fibers by a traveling wave.

9. The device according to one of claims 1 to 8, wherein the time information describes starting points in time of the trajectories.

10. The device according to claim 9, wherein the starting point in time of a given trajectory is a point in time in which a first activity impulse associated with the given trajectory impinges upon a nerve fiber.

11. The device according to one of claims 1 to 10, wherein means (730) for processing the neural activity pattern (730) includes means for pattern recognition which is implemented to recognize, in a two-dimensional representation formed by the neural activity pattern over time, a straight or curved line-shaped pattern as a trajectory (740, 750), to determine the temporal position of the trajectory (740, 750) and provide time information (t1, t2) belonging to the trajectory (740, 750) as an analysis representation of the audio signal.

12. The device according to claim 11, wherein means for pattern recognition is further implemented to provide information about a shape of the trajectories as part of the analysis representation.

13. The device according to claim 12, wherein information about the shape of the trajectory includes information about a curvature of the trajectory.

14. The device according to one of claims 11 to 13, wherein means for pattern recognition is determined to recognize a straight or hyperbolically curved trajectory.

15. The device according to one of claims 11 to 14, wherein means for pattern recognition is further implemented to provide information about a length of the trajectory as part of the analysis representation.

16. The device according to one of claims 1 to 15, wherein means (730) for processing the neural activity pattern includes means for a pattern comparison which is implemented to compare a two-dimensional representation describing the neural activity pattern over time to at least one comparison pattern to recognize a trajectory and obtain time information describing a temporal position of the trajectory.

17. The device according to claim 16, wherein the comparison pattern is a straight or hyperbolically shaped curve.

18. The device according to one of claims 1 to 17, wherein means (730) for processing the neural activity pattern is implemented to gradually distort the two-dimensional representation of the neural activity pattern over time so that a curved trajectory in the neural activity pattern is gradually straightened by the gradual distortion, wherein a number of distortion steps required for a straightening of the curved trajectory are dependent on a curvature of the curved trajectory, and wherein the number of distortion steps required for the straightening of the curved trajectory includes a statement about the original form of the trajectory.

19. The device according to one of claims 1 to 18, wherein means (730) for processing the neural activity pattern includes curve recognition means (1300, 1500, 1600) which is implemented in order to receive the neural activity pattern in the form of a plurality of signals (1320, 1322, 1324; 1520, 1522, 1524) in parallel and to advance the signals (1320, 1322, 1324; 1520, 1522, 1524) at different speeds in parallel through a plurality of stages connected in series (1310, 1312, 1314; 1530, 1532, 1524), wherein at least one predetermined stage (1310, 1312, 1314; 1530, 1532, 1534) comprises threshold value recognition means (1560, 1570, 1562, 1572, 1564, 1574) which is implemented in order to recognize when at least a predetermined number of signals is simultaneously active in the predetermined stage.

20. The device according to claim 19, wherein at least one stage (1310, 1312, 1314; 1530, 1532, 1534) is implemented to delay several signals in a differently strong way when advancing the same through the stage.

21. The device according to claim 19 or 20, wherein the curve recognition means (1300; 1500; 1600) is a neural net.

22. The device according to one of claims 1 to 21, wherein means for processing the neural activity pattern is implemented in order to recognize a trajectory based on an evaluation of a Hough transformation.

23. The device according to one of claims 1 to 22, wherein means for processing the neural activity pattern includes means for performing a parallel Hough transformation.

24. The device according to one of claims 1 to 23, further including means (1800) for analyzing an audio signal content based on the time information (t1, t2).

25. The device according to claim 24, wherein means for analyzing the audio signal content includes comparison means (1810) which is implemented to compare time information (t1, t2) to reference time information (1840) present in a database (1820) in order to provide information (1850) about the audio signal content as a comparison result.

26. The device according to claim 24 or 25, wherein means for analyzing the audio signal content is implemented in order to recognize a vocal or a consonant based on the time information belonging to several consecutive trajectories.

27. The device according to one of claims 24 to 26, wherein means for analyzing the audio signal is further implemented to use information about a shape and/or curvature of the trajectory.

28. The device according to one of claims 24 to 27, wherein means for analyzing the audio signal is implemented to determine, based on the time information, information about a rhythm of the audio signal, wherein information about the rhythm of the audio signal represent a description for the content of the audio signal.

29. A device (700) for analyzing an audio signal to obtain an analysis representation (760) of the audio signal, comprising:

means (720) for calculating a neural activity pattern over time resulting at nerve fibers of an ear model based on the audio signal (710); and
means (230) for processing the neural activity pattern in order to obtain a sequence of time information (t1, t2) as an analysis representation (760) describing a temporal position of consecutive trajectories (740, 750), wherein a trajectory (740, 750) includes activity impulses on different nerve fibers (NF1, NF2, NF3, NF4, NF5) based on the same event in the audio signal (710);
**characterized in that** the means (730) for processing the neural activity pattern includes curve recognition means (1300, 1500, 1600) which is implemented in order to receive the neural activity pattern in the form of a plurality of signals (1320, 1322, 1324; 1520, 1522, 1524) in parallel and to advance the signals (1320, 1322, 1324; 1520, 1522, 1524) at different speeds in parallel through a plurality of stages connected in series (1310, 1312, 1314; 1530, 1532, 1524), wherein at least one predetermined stage (1310, 1312, 1314; 1530, 1532, 1534) comprises threshold value recognition means (1560, 1570, 1562, 1572, 1564, 1574) which is implemented in order to recognize when at least a predetermined number of signals is simultaneously active in the predetermined stage.

30. A method for analyzing an audio signal to obtain an analysis representation of the audio signal, comprising:

calculating a neural activity pattern over time resulting at nerve fibers of an ear model based on the audio signal; and
processing the neural activity pattern in order to obtain a sequence of time information as an analysis representation describing a temporal position of consecutive trajectories, wherein a trajectory includes activity impulses on different nerve fibers based on the same event in the audio signal;
**characterized in that** the processing of the neural activity pattern comprises:

gradually distorting a two-dimensional representation of the neural activity pattern over time in order to obtain a distorted two-dimensional representation of the neural activity pattern over time, recognizing when an approximately straight line is contained in the distorted two-dimensional representation of the neural activity pattern over time, recognizing the approximately straight line as a trajectory, determining the temporal position of the trajectory, and providing the time information belonging to the trajectory as an analysis representation of the audio signal.

31. A method for analyzing an audio signal to obtain an analysis representation of the audio signal, comprising:

calculating a neural activity pattern over time resulting at nerve fibers of an ear model based on the audio signal; and

processing the neural activity pattern in order to obtain a sequence of time information as an analysis representation describing a temporal position of consecutive trajectories, wherein a trajectory includes activity impulses on different nerve fibers based on the same event in the audio signal; characterized in that the processing of the neural activity pattern includes receiving the neural activity pattern in parallel in the form of a plurality of signals (1320, 1322, 1324; 1520, 1522, 1524), advancing the signals (1320, 1322, 1324; 1520, 1522, 1524) at different speeds through a plurality of stages connected in series (1310, 1312, 1314; 1530, 1532, 1524) and recognizing when at least a predetermined number of signals is simultaneously active in a predetermined stage; wherein the method is performed using a hardware or using a computer.

32. A computer program having a program code for performing the method of claim 30 or 31 when the computer program runs on a computer.

## Revendications

1. Dispositif (700) pour analyser un signal audio pour obtenir une représentation d'analyse (760) du signal audio, aux caractéristiques suivantes:

un moyen (720) destiné à calculer un modèle d'activité nerveuse dans le temps sur les fibres nerveuses d'un modèle d'ouïe qui se produit par suite du signal audio (710); et
un moyen (230) destiné à traiter le modèle d'activité nerveuse pour obtenir, comme représentation d'analyse (760), une séquence d'informations de temps (t1, t2) qui décrivent une position actuelle de trajectoires successives (740, 750), une trajectoire (740, 750) comportant des impulsions d'activité sur différentes fibres nerveuses (NF1, NF2, NF3, NF4, NF5) dues au même événement dans le signal audio 710;
caractérisé par le fait que le moyen (730) destiné à traiter le modèle d'activité nerveuse est conçu pour distorsionner pas à pas une représentation bidimensionnelle du modèle d'activité nerveuse dans le temps pour obtenir une représentation bidimensionnelle distorsionnée du modèle d'activité nerveuse dans le temps, et pour détecter le moment où est obtenue, dans la représentation bidimensionnelle distorsionnée du motif d'activité nerveuse dans le temps, une ligne environ droite, pour détecter la ligne environ droite comme trajectoire pour déterminer la position actuelle de la trajectoire et pour fournir l'information de temps appartenant à la trajectoire comme représentation d'analyse du signal audio.

2. Dispositif selon la revendication 1, dans lequel un modèle d'activité nerveuse (730) décrit une activité d'un groupe de fibres nerveuses du modèle d'ouïe.

3. Dispositif selon la revendication 1 ou 2, dans lequel une information de temps (t1, t2) décrit un moment d'une occurrence d'une trajectoire (740, 750).

4. Dispositif (700) selon l'une des revendications 1 à 3, qui est conçu pour détecter une trajectoire (740, 750) lorsque des impulsions d'activité qui sont dues à des mêmes événements dans le signal audio sont présentes sur un nombre de fibres nerveuses qui est supérieur à un nombre minimum prédéterminé.

5. Dispositif selon l'une des revendications 1 à 4, qui est conçu pour déterminer comme événement acoustique un début d'une voyelle, d'une consonne ou d'un son dans le signal audio.

**6.** Dispositif selon l'une des revendications 1 à 5, qui est conçu pour détecter une trajectoire dans le modèle d'activité nerveuse qui décrit une onde progressive sur une membrane basilaire du modèle d'ouïe.

**7.** Dispositif selon l'une des revendications 1 à 6, dans lequel une trajectoire est définie comme modèle d'activité nerveuse sur un groupe de fibres nerveuses adjacentes associé à une onde progressive sur la membrane basilaire du modèle d'ouïe.

**8.** Dispositif selon l'une des revendications 1 à 7, dans lequel une trajectoire est définie par une occurrence d'impulsions d'activité sur un groupe de fibres nerveuses adjacentes, les impulsions d'activité décrivant une stimulation des fibres nerveuses adjacentes par une onde progressive.

**9.** Dispositif selon l'une des revendications 1 à 8, dans lequel les informations de temps décrivent des moments de début des trajectoires.

**10.** Dispositif selon la revendication 9, dans lequel le moment de début d'une trajectoire prédéterminée est un moment auquel se produit sur une fibre nerveuse une première impulsion d'activité appartenant à la trajectoire prédéterminée.

**11.** Dispositif selon l'une des revendications 1 à 10, dans lequel le moyen (730) destiné à traiter le modèle d'activité nerveuse (730) comporte un moyen destiné à détecter le modèle qui est conçu pour détecter, dans une représentation bidimensionnelle qui est formée par le modèle d'activité nerveuse dans le temps, un modèle en forme de ligne droite ou courbe comme trajectoire (740, 750), la position actuelle de la trajectoire (740, 750) et pour fournir une information de temps (t1, t2) appartenant à la trajectoire (740, 750) comme représentation d'analyse du signal audio.

**12.** Dispositif selon la revendication 11, dans lequel le moyen destiné à détecter le modèle est par ailleurs conçu pour fournir une information sur une forme des trajectoires comme partie de la représentation d'analyse.

**13.** Dispositif selon la revendication 12, dans lequel l'information sur la forme de la trajectoire comporte une information sur la courbure de la trajectoire.

**14.** Dispositif selon l'une des revendications 11 à 13, dans lequel le moyen destiné à détecter le modèle est conçu pour détecter une trajectoire droite ou à courbure hyperbolique.

**15.** Dispositif selon l'une des revendications 11 à 14, dans lequel le moyen destiné à détecter le modèle est par ailleurs conçu pour fournir une information sur une longueur de la trajectoire comme partie de la représentation d'analyse.

**16.** Dispositif selon l'une des revendications 1 à 15, dans lequel le moyen (730) destiné à traiter le modèle d'activité nerveuse comporte un moyen destiné à comparer le modèle qui est conçu pour comparer une représentation bidimensionnelle qui décrit le modèle d'activité nerveuse dans le temps avec au moins un modèle de comparaison pour détecter une trajectoire et obtenir une information de temps qui décrit une position actuelle de la trajectoire.

**17.** Dispositif selon la revendication 16, dans lequel le modèle de comparaison est une droite ou une courbe hyperbolique.

**18.** Dispositif selon l'une des revendications 1 à 17, dans lequel le moyen (730) destiné à traiter le modèle d'activité nerveuse est conçu pour distorsionner pas à pas la représentation du modèle d'activité nerveuse bidimensionnelle dans le temps de sorte qu'une trajectoire courbe dans le modèle d'activité nerveuse soit redressée pas à pas en une ligne droite, un nombre de pas de distorsion qui sont nécessaires pour redresser la trajectoire courbe en une ligne droite dépendant d'une courbure de la trajectoire courbe, et le nombre de pas de distorsion qui sont nécessaires pour redresser la trajectoire courbe en une ligne droite comportant une indication sur une forme originale de la trajectoire.

**19.** Dispositif selon l'une des revendications 1 à 18, dans lequel le moyen (730) destiné à traiter le modèle d'activité nerveuse comporte un dispositif de détection de courbe (1300, 1500, 1600) qui est conçu pour recevoir le modèle d'activité nerveuse sous forme d'une pluralité de signaux (1320, 1322 1324; 1520, 1522, 1524) en parallèle, et pour transmettre les signaux (1320, 1322, 1324, 1520, 1522, 1524) à des vitesses différentes en parallèle par une pluralité d'étages connectés l'un derrière l'autre (1310, 1312, 1314, 1530, 1532, 1524), au moins un étage prédéterminé (1310, 1312, 1314, 1530, 1532, 1534) présentant un dispositif de détection de valeur de seuil (1560, 1570, 1562, 1572, 1564, 1574) qui est conçu pour détecter le moment où au moins un nombre prédéterminé de signaux sont actifs simultanément dans l'étage prédéterminé.

**20.** Dispositif selon la revendication 19, dans lequel au moins un étage (1310, 1312, 1314, 1530, 1532, 1534) est conçu pour retarder dans différentes mesures plusieurs signaux lors d'une transmission par l'étage.

**21.** Dispositif selon la revendication 19 ou 20, dans lequel le moyen de détection de courbe (1300; 1500; 1600) est un réseau neuronal.

**22.** Dispositif selon l'une des revendications 1 à 21, dans lequel le moyen destiné à traiter le modèle d'activité nerveuse est conçu pour détecter une trajectoire sur base d'une évaluation d'une transformation de Hough.

**23.** Dispositif selon l'une des revendications 1 à 22, dans lequel le moyen destiné à traiter le modèle d'activité nerveuse comporte un moyen destiné à réaliser une transformée de Hough parallèle.

**24.** Dispositif selon l'une des revendications 1 à 23, comportant par ailleurs un moyen (1800) destiné à analyser un contenu de signal audio sur base de l'information de temps (t1, t2).

**25.** Dispositif selon la revendication 24, dans lequel le moyen destiné à analyser le contenu du signal audio comporte un moyen de comparaison (1810) qui est conçu pour comparer l'information de temps (t1, t2) avec des informations de temps de référence (1840) présentes dans une base de données (1820) pour fournir comme résultat de comparaison une information (1850) sur le contenu de signal audio.

**26.** Dispositif selon la revendication 24 ou 25, dans lequel le moyen destiné à analyser le contenu de signal audio est conçu pour détecter une voyelle ou consonne sur base des informations de temps appartenant à plusieurs trajectoires successives.

**27.** Dispositif selon l'une des revendications 24 à 26, dans lequel le moyen destiné à analyser le signal audio est par ailleurs conçu pour utiliser une information sur une forme et/ou courbure de la trajectoire.

**28.** Dispositif selon l'une des revendications 24 à 27, dans lequel le moyen destiné à analyser le signal audio est conçu pour déterminer, sur base des informations de temps, une information sur un rythme du signal audio, l'information sur le rythme du signal audio représentant une description du contenu du signal audio.

**29.** Dispositif (700) pour analyser un signal audio pour obtenir une représentation d'analyse (760) du signal audio, aux caractéristiques suivantes:

un moyen (720) destiné à calculer un modèle d'activité nerveuse dans le temps sur les fibres nerveuses d'un modèle d'ouïe qui se produit par suite du signal audio (710); et
un moyen (230) destiné à traiter le modèle d'activité nerveuse pour obtenir, comme représentation d'analyse (760), une séquence d'informations de temps (t1, t2) qui décrivent une position actuelle de trajectoires successives (740, 750), une trajectoire (740, 750) comportant des impulsions d'activité sur différentes fibres nerveuses (NF1, NF2, NF3, NF4, NF5) par suite du même événement dans le signal audio 710;
**caractérisé par le fait que** le moyen (730) destiné à traiter le modèle d'activité nerveuse comporte un dispositif de détection de courbe (1300, 1500, 1600) qui est conçu pour recevoir le modèle d'activité nerveuse sous forme d'une pluralité de signaux (1320, 1322, 1324, 1520, 1522, 1524) en parallèle, et pour transmettre les signaux (1320, 1322, 1324, 1520, 1522, 1524) à des vitesses différentes en parallèle par une pluralité d'étages connectés l'un derrière l'autre (1310, 1312, 1314, 1530, 1532, 1524), au moins un étage prédéterminé (1310, 1312, 1314, 1530 1532 1534) présentant un dispositif de détection de valeur de seuil (1560, 1570, 1562, 1572, 1564, 1574) qui est conçu pour détecter le moment où au moins un nombre prédéterminé de signaux sont actifs simultanément dans l'étage prédéterminé.

**30.** Procécé pour analyser un signal audio pour obtenir une représentation d'analyse du signal audio, aux étapes suivantes consistant à:

calculer un modèle d'activité nerveuse dans le temps sur les fibres nerveuses d'un modèle d'ouïe qui se produit par suite du signal audio; et
traiter le modèle d'activité nerveuse pour obtenir, comme représentation d'analyse, une séquence d'informations de temps qui décrivent une position actuelle de trajectoires successives, une trajectoire comportant des impulsions d'activité sur différentes fibres nerveuses du fait du même événement dans le signal audio;
**caractérisé par le fait que** le traitement du modèle d'activité nerveuse comporte les étapes suivantes consistant à:

distorsionner pas à pas une représentation bidimensionnelle du motif d'activité nerveuse dans le temps pour obtenir une représentation bidimensionnelle distorsionnée du modèle d'activité nerveuse dans le temps,

détecter le moment où est obtenue une ligne environ droite dans la représentation bidimensionnelle distorsionnée du modèle d'activité nerveuse dans le temps,

détecter la ligne environ droite comme une trajectoire,

déterminer la position actuelle de la trajectoire, et

fournir l'information de temps appartenant à la trajectoire comme représentation d'analyse du signal audio;

dans lequel le procédé est réalisé à l'aide d'un matériel ou à l'aide d'un ordinateur.

31. Procédé pour analyser un signal audio pour obtenir une représentation d'analyse du signal audio, aux étapes suivantes consistant à:

calculer un modèle d'activité nerveuse dans le temps sur les fibres nerveuses d'un modèle d'ouïe qui se produit du fait du signal audio; et

traiter le modèle d'activité nerveuse pour obtenir, comme représentation d'analyse, une séquence d'informations de temps qui décrivent la position actuelle de trajectoires successives, une trajectoire comportant des impulsions d'activité sur différentes fibres nerveuses dues au même événement dans le signal audio;

**caractérisé par le fait que** le traitement du modèle d'activité nerveuse comporte une réception en parallèle du modèle d'activité nerveuse sous forme d'une pluralité de signaux (1320, 1322, 1324, 1520, 1522, 1524), une transmission à différente vitesse des signaux (1320, 1322, 1324, 1520, 1522, 1524) par une pluralité d'étages connectés l'un derrière l'autre (1310, 1312, 1314, 1530, 1532, 1524), et

une détection du moment où au moins un nombre prédéterminé de signaux dans un étage prédéterminé sont actifs simultanément;

dans lequel le procédé est réalisé à l'aide d'un matériel ou à l'aide d'un ordinateur.

32. Programme d'ordinateur avec un code de programme pour mettre en oeuvre le procédé selon la revendication 30 ou 31 lorsque le programme d'ordinateur est exécuté sur un ordinateur.

FIGUR 1

200

AUDIOSIGNAL

210

BERECHNEN EINER
BASILARMEMBRAN-
BEWEGUNG — 220

230
BASILARMEMBRAN-BEWEGUNG

BERECHNUNG EINER
AUSLENKUNG EINES
STEREOZILIUMS — 240

250
AUSLENKUNG EINES STEREOZILIUMS

BERECHNEN EINES
NEUROTRANSMITTER-
VESIKEL-AUFTRETENS — 260

NEUROTRANSMITTER-
VESIKEL-AUFTRETEN

FIGUR 2

36

300

AUDIOSIGNAL

—310

```
┌─────────────────────┐
│    MECHANISCHE      │
│  SCHALLWANDLUNG     │──320
│    IM INNENOHR      │
└─────────────────────┘
```

—324

ANREGUNG DES TROMMELFELLS

```
┌─────────────────────┐
│  BERECHNUNG DER     │
│  SCHALLÜBERTRAGUNG  │──330
│    ÜBER GEHÖR-      │
│    KNÖCHELCHEN      │
└─────────────────────┘
```

—334

ANREGUNG DES OVALEN FENSTERS
ZWISCHEN MITTELOHR UND COCHLEA

```
┌─────────────────────┐
│  BERECHNUNG DER     │
│  HYDROMECHANISCHEN  │──340
│   SCHWINGUNGSAN-    │
│  REGUNG DER COCHLEA │
└─────────────────────┘
```

—344

SCHWINGUNGSANREGUNG DER COCHLEA

```
┌─────────────────────┐
│  BERECHNUNG DER     │
│  BASILARMEMBRAN-    │──350
│     BEWEGUNG        │
└─────────────────────┘
```

—354

BASILARMEMBRAN-
BEWEGUNG

# FIGUR 3

400

BASILARMEMBRAN-BEWEGUNG
v(t)

STOCHASTISCHE
KRAFT $F_{stoch}(t)$

LÖSEN DER BEWEGUNGSGLEICHNUNG:
$$m\ddot{x} = -Dx - K\dot{x} + F_{ext}(t) + F_{stoch}(t)$$
$$F_{ext} = C_{bas}v(t)$$

AUSLENKUNG EINES
STEREOCILIUMS x(t)

m: EFFEKTIVE MASSE EINES STEREOCILIUMS
D: EFFEKTIVE FEDERKONSTANTE DES STEREOCILIUMS
K: KONSTANTE FÜR LAMINAREN STRÖMUNGSWIDERSTAND
   DES STEREOCILIUMS
$C_{bas}$: KONSTANTE FÜR ANREGUNG DES STEREOCILIUMS
   AUFGRUND BASILARMEMBRANBEWEGUNG

FIGUR 4

500

AUSLENKUNG EINES
STEREOZILIUMS u(t)

BERECHNEN EINES
SPITZEN-LEITWERKS

510

SPITZEN-LEITWERT Q(u)

BERECHNEN EINES INTRA-
ZELLULÄREN HAARZELLEN-
POTENTIALS

520

INTRAZELLULÄRES HAARZELLEN-POTENTIAL V(t)

BERECHNEN EINES
CALCIUMSTROMS

530

CALCIUMSTROM $I_{Ca}(t)$

BERECHNEN EINER CALCIUM-
KONZENTRATION

540

CALZIUM-KONZENTRATION $[Ca^{2+}](t)$

BERECHNEN EINER TRANS-
MITTER-FREISETZUNGS-
RATE

550

TRANSMITTER-FREISETZUNGS-RATE k(t)

BESTIMMEN EINES NEURO-
TRANSMITTER-VESIKEL-
AUFTRETENS

569

NEUROTRANSMITTER-VESIKEL-
AUFTRETEN

# FIGUR 5

600

NEUROTRANSMITTER-
VESIKEL-AUFTRETEN

610 — BERECHNEN
EINER
SPANNUNG IN
EINER NERVEN-
ZELLE

POSTSYNAPTISCHES
POTENTIAL

620 — TREFFEN EINER
ENTSCHEIDUNG
ÜBER EINE FREI-
SETZUNG EINES
AKTIONS-
POTENTIALS

AKTIONSPOTENTIAL

FIGUR 6

700

AUDIOSIGNAL

└─710

BERECHNEN EINES NERVEN-
AKTIVITÄTSMUSTERS

OHRMODELL

~720

NERVEN-
AKTIVITÄTS-
MUSTER

t

t₂

NF1     NF2  NF3     NF4  NF5      ~750
                                      ~730
                 AP9   AP10  ←
       AP6  AP7  AP8                 TRAJEKTORIE
                                     ~740
t₁     AP1 AP2  AP3  AP4  AP5

VERARBEITEN DES
NERVENAKTIVITÄTSMUSTERS

~754

760─┘ (t₂)
(t₁)

VERBESSERTE ANALYSEDARSTELLUNG

FIGUR 7

NEURONALE REPRÄSENTATION

AKTIVIERENDER STIMULUS TRANSFORMATION

2D TRAJEKTORIEN 850

TRAJEKTORIE 1   TRAJEKTORIE 2

834

862

870   872

APEX

x

BASILARMEMBRAN

NF5

NF4

810

NF3

820

NF2

IMPULSANTWORTEN

824

NF1

AKUSTISCHER IMPULS
(CLICK)

BASIS   830

t

892   860

890

x

APEX

x

BASILARMEMBRAN

F0
NF5

NF4

882

2 F0
NF3

NF2

4 F0
NF1

884

...

FOLGE VON
AKUSTISCHEN IMPULSEN
(CLICKS)
(T=1/F0)

BASIS

ÜBERLAGERUNG VON IMPULS-
ANTWORTEN AUF VER
SCHIEDENE IMPULSE

MODELLIERTE ANTWORTEN

t

FIGUR 8

EP 1 896 124 B1

# BERECHNUNG DER VERZÖGERUNG ALS FUNKTION DER FREQUENZ

DURCH EIN SINUSSIGNAL HERVOR-
GERUFENE LATENZZEIT DES HÖRNERVS
- Chinchilla (Ruggero & Rich, 1987)
—Cat (Goldstein et al, 1971)

$$d_a = (1000/f_i) + 2\,(ms)$$

LATENZZEIT DER AUDITORISCHEN NERVENFASER (ms)

CHARAKTERISTISCHE FREQUENZ (kHz)

BASILAR MEMBRAN

ANSTAND VON DER BASIS IN mm (SKAL. ZU EINER MENSCHLICHEN COCHLEA)

LATENZZEIT EINER ANREGUNG EINER EINZELNEN FASER DES HÖRNERVS
ALS FUNKTION DER SINUSFÖRMIGEN SIGNALFREQUENZ

FIGUR 9

EP 1 896 124 B1

COCHLEAGRAM DES VOKALS i

DETAILS DES CHOCHLEAGRAMS ZEIGEN DIE FEINE ZEITLICHE UND RÄUMLICHE STRUKTUR
IN DER MODELLIERTEN ANTWORT AUF EINEN VOKAL /i/ (LINKS) UND AUF EINEN
NICHT-HARMONISCHEN TONKOMPLEX VON 700 Hz, 900 Hz UND 1100 Hz (RECHTS)

FIGUR 10

EP 1 896 124 B1

VOKAL "A":

f
(n)

1140

f
(n)

1140

f
(n)

1140

CHOCHLEAGRAM
← 1110

FREISETZUNGS-
WAHRSCHEINLICHKEIT
← 1120

VESIKEL-FREISETZUNG
← 1130

t

FIGUR 11

EP 1 896 124 B1

DIE VERARBEITUNGSKETTE

1200

SCHALTUNGEN ZUR
DETEKTION DER ZEIT
ZWISCHEN ZWEI
SPITZEN

1220

1230

1260          1250

BASILARMEMBRAN

$F_0$
$F_1$
$F_2$
$F_3$

$F_0$
$F_1$
$F_2$
$F_3$

GEHIRN

AUDIOSIGNAL

1210

AKKUSTISCHER
KERN

1240

1260

FIGUR 12

EP 1 896 124 B1

FIGUR 13

FIGUR 14

# DAS SCHALTBILD DES HUBEL-WIESEL NETZES

FIGUR 15

VERZÖGERUNGSELEMENTE/ADDIERER

SCHWELLWERT-
REGISTER/
KOMPARATOREN

EP 1 896 124 B1

FIGUR 16

## DIE TRAININGSMUSTER

NEUN SINUSSE UNTERSCHIEDLICHER FREQUENZ

# FIGUR 17

1800

AUDIOSIGNAL-REPRÄSENTANT

~1830

1850

1810 ~ VERGLEICHS-
EINRICHTUNG

1840 ~ VERGLEICHS-
AUDIOSIGNAL-
REPRÄSENTANTEN

1820 ~ AUDIOSIGNAL
DATENBANK

FIGUR 18

1900

AUDIOSIGNAL
REPRÄSENTANT | MERKMALS-
EXTRAKTION | AUDIOSIGNAL
FINGERABDRUCK | DATEN-
BANK

1910

1920

1930

1940

FIGUR 19

RECESSUS EPITYMPANICUS  INCUS
MALLEUS
M. TENSOR TYMPANI
TUBA EUSTACHII
STAPES
M. LEVATOR
VELI PALATINI

ABBILDUNG 3.1: AUDITORISCHE PERIPHERIE

# FIGUR 20

ABBILDUNG 3.2:
AUSSENOHRÜBERTRAGUNGS-
FUNKTION - ZYLINDRISCHE
OBERFLÄCHENDARSTELLUNG
DER BETRAGSANTWORT ALS
FUNKTION DES AZIMUTHWINKEL
AUF RADIALER ACHSE

# FIGUR 21

ABBILDUNG 3.3: SCHEMA VON MITTELOHR UND AUFGEROLLTER COCHLEA

## FIGUR 22

ABBILDUNG 3.4: HÖRFLÄCHE

## FIGUR 23

TECTORIALMEMBRAN

INNERE HAARZELLEN

ÄUSSERE HAARZELLEN

[Na+]hoch
[K+]niedrig
0 mV

+85 mV
[K+]
[Na+]

[Na+]hoch
[K+]niedrig
0 mV

SCALA VESTIBULI

REISSNER MEMBRAN

SCALA MEDIA

HAARZELLEN

BASILARMEMBRAN

SCALA TYMPANI

HÖRNERVEN-
FASERN

KNOCHEN

ABBILDUNG 3.5: QUERSCHNITT DER COCHLEA

# FIGUR 24

SPITZENFADEN

SCHALLSIGNAL

ENDOLYMPHE
PERILYMPHE

STEREOZILIUM

DEFLEKTION

DEPOLARISATION

REPOLARISATION

ZYTOPLASMA

TRANSMITTER

AUDITORISCHER NERV

ABBILDUNG 3.6: SCHEMA DER HAARZELLE

# FIGUR 25

# ANATOMIE DER AUDITORISCHEN PERIPHERIE

EP 1 896 124 B1

TROMMELFELL

MITTELOHR

HÖRNERV

AUDITORISCHER
PFAD

GERÄUSCH

COCHLEA

a) SPEKTRALANALYSE
b) UMWANDLUNG VON VIBRATION
   IN NEURALE IMPULSE

## FIGUR 26

MECHANISMUS DER SIGNALÜBERTRAGUNG I

FIGUR 27

3210

EP 1 896 124 B1

# BASILARMEMBRAN

3310

4000    3000    0.5 mm
        APEX        3320
800
5000    600
        2000
1000    400
1500

7000    0.04 mm
    BASIS        3320

B
20000

CHARAKTERISTISCHE FREQUENZEN

3320

COCHLEA

OVALES FENSTER
3330
        3340

AUSBREITUNG

WANDERWELLE
3350

3360
BASILARMEMBRAN        HELICOTREMA

WANDERWELLE AUF DER BASILARMEMBRAN
DER COCHLEA

## FIGUR 28

EP 1 896 124 B1

# DAS ERWEITERTE ZWICKER-MODELL

a) HYDROMECHANIK DES INNENOHRS

b) GEDRUCKTE SCHALTUNG VON a)

c) NICHTLINEARE RÜCKKOPPLUNG DER ÄUSSEREN HAARZELLEN

FIGUR 29

## SCHEMA: CORTISCHES ORGAN

1  IHC
2  AHC
3  CORTISCHER TUNNEL
4  BASILARMEMBRAN
5  RETIKULARMEMBRAN
6  TEKTORIALMEMBRAN
7  DEITERSCHE ZELLEN
8  NUELSCHER RAUM
9  HENSEN-ZELLEN
10 INNERER SPIRALSULCUS

FIGUR 30

EP 1 896 124 B1

## AUFBAU DER HAARZELLEN

1 NUCLEUS
2 STEREOCILIA
3 CUTICULAR PLATE
4 RADIAL AFFERENT ENDING
(DENDRITE OF TYPE I NEURON)
5 LATERAL EFFERENT ENDING
6 MEDIAL EFFERENT ENDING
7 SPIRAL AFFERENT ENDING
(DENDRITE OF TYPE II NEURON)

NUR CA. 3,500 IHCS UND 12,000 OHCS
IN DER HUMANEN COCHLEA ANGELEGT!

EP 1 896 124 B1

FIGUR 31

FIGUR 32

FIGUR 33

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5381512 A

- US 3069654 A

**In der Beschreibung aufgeführte Nicht-Patentliteratur**

- *Elsevier-Journal for Speech Communication,* 2002, vol. 36, 181-203
- **C.J. SUMNER ; E.A. LOPEZ-POVEDA ; L.P. O'MARD ; R. MEDDIS.** A revised model of the inner hair cell and auditory nerve complex. *J. Acoust. Soc. Am.,* Mai 2002, vol. 111 (5

- **ERWIN NEHER ; TAKESHI SAKABA.** Estimating Transmitter Release Rates from Postsynaptic Current Fluctuations. *The Journal of Neuroscience,* 15. Dezember 2001, vol. 21 (24), 9638-9654
- **E. NEHER ; T. SAKABA.** Quantal release parameters estimated from noise. *J. Neurosience,* 15. Dezember 2001, vol. 21 (24), 9638-9654